(19) Europäisches Patentamt
European Patent Office
Office européen des brevets

(11) EP 1 103 563 A1

(12) # EUROPEAN PATENT APPLICATION

published in accordance with Art. 158(3) EPC

(43) Date of publication:
**30.05.2001 Bulletin 2001/22**

(21) Application number: **99931470.1**

(22) Date of filing: **22.07.1999**

(51) Int. Cl.[7]: **C07K 14/705**, C12N 15/12, C12P 21/02, C07K 16/28, G01N 33/50, A61K 38/17, C12Q 1/68

(86) International application number:
**PCT/JP99/03909**

(87) International publication number:
**WO 00/05264 (03.02.2000 Gazette 2000/05)**

(84) Designated Contracting States:
**AT BE CH CY DE DK ES FI FR GB GR IE IT LI LU MC NL PT SE**
Designated Extension States:
**AL LT LV MK RO SI**

(30) Priority: **23.07.1998 JP 20757998**
**07.08.1998 JP 22506098**
**06.10.1998 JP 28432898**

(71) Applicants:
- **Takeda Chemical Industries, Ltd.**
  **Osaka-shi, Osaka 541-0045 (JP)**
- **Kazusa Dna Research Institute**
  **Kisarazu-shi, Chiba 292-0812 (JP)**

(72) Inventors:
- **OHARA, Osamu**
  **Kisarazu-shi, Chiba 292-0801 (JP)**
- **NAGASE, Takahiro**
  **Kisarazu-shi, Chiba 292-0042 (JP)**
- **NOMURA, Nobuo**
  **Kisarazu-shi, Chiba 292-0804 (JP)**
- **HINUMA, Shuji**
  **Tsukuba-shi, Ibaraki 305-0821 (JP)**
- **FUJII, Ryo**
  **Tsukuba-shi, Ibaraki 305-0821 (JP)**
- **KITAHARA, Osamu**
  **Tsukuba-shi, Ibaraki 305-0821 (JP)**
- **MOGI, Shinichi**
  **Kitasoma-gun, Ibaraki 302-0121 (JP)**

(74) Representative:
**Keller, Günter, Dr. et al**
**Lederer, Keller & Riederer**
**Patentanwälte**
**Prinzregentenstrasse 16**
**80538 München (DE)**

(54) **NOVEL G PROTEIN-CONJUGATED RECEPTOR PROTEIN AND DNA THEREOF**

(57) The present invention relates to a human brain-derived protein, a partial peptide thereof or a salt thereof, a DNA encoding the receptor protein, a process for producing the protein, a method for determining a ligand for the G protein-coupled receptor protein, a method for screening/a kit for screening a compound which alters property of a ligand binding with the protein, a compound or a salt thereof obtainable by the screening, an antibody to the G protein-coupled receptor protein and the like.

The human brain-derived G protein-coupled receptor protein or the DNA encoding the protein of the present invention are useful as or in (1) determination of a ligand, (2) obtaining of an antibody and antiserum, (3) construction of the expression system for a recombinant receptor protein, (4) development of receptor binding assay using the same expression system and screening of drug candidates, (5) implementation of drug design nucleotide on comparison with a ligand receptor having the structural similarity, (6) reagents for preparing a probe or a PCR primer in gene therapy, (7) production of a transgenic animal, or (8) a drug for gene prophylaxis or therapy.

EP 1 103 563 A1

**Description**

Technical Field

The present invention relates to a human brain-derived novel protein (G protein-coupled receptor protein) or a salt thereof and a DNA encoding it.

Background Technique

Many hormones and neurotransmitters regulate the functions of the living body via specific receptor proteins present in a cell membrane. Many of these receptor proteins perform intracellular signal transmittance through activation of conjugated guanine nucleotide-binding protein (hereinafter abbreviated as G protein in some cases) and, since they have the common structure having seven transmembrane regions, they are collectively called G protein-coupled receptor proteins or seven transmembranes receptor protein.

G protein-coupled receptor protein is present at various functional cell surfaces of cells or organs of the living body and carries an important role as a target for molecules which regulate the functions of cells or organs of the living body, for example, hormones, neurotransmitters and physiologically active substances.

Revelation of the relationship between substances which regulate the complex functions within cells and organs of the various living bodies and specific receptor proteins therefore, in particular, G protein-coupled receptors elucidates the functions of cells and organs of various living bodies and provides a very important mean for development of medicals related to the functions.

For example, in central nervous system organs, regulation of the physiological functions of the brain is performed under the regulation by many hormones, neurotransmitters or physiologically active substances. In particular, neurotransmitters are present in various sites in the brain and perform the regulation of the physiological functions through each corresponding receptor protein. There are many unknown transmitters in the brain and it is thought that many of the structures of cDNA's encoding receptor proteins have not been reported yet. Further, whether a subtype of the known receptor protein is present or not has not been elucidated.

Revelation of the relationship between substances which regulate the complicated functions within cells and organs of the various living bodies and specific receptor proteins therefore is a very important mean for developing medicals related to the functions. In addition, in order to effectively screen agonists and antagonists for a receptor protein and develop medicals, it is necessary to elucidate the functions of genes for receptor proteins which are expressed in the brain and allow the genes to be expressed in the suitable expressing system.

Recently, as means for analyzing genes expressed in the living body, random analysis of the sequences of cDNA's have been actively studied and the sequences of fragments of cDNA's thus obtained are registered in database as Expressed Sequence Tag (EST) and published. However, many EST's are only information in the sequence and it is difficult to presume the functions.

Summary of the Invention

The present invention provides a human brain-derived novel protein (G protein-coupled receptor protein), its partial peptide or a salt thereof, a DNA containing a DNA encoding the protein or its partial peptide, a recombinant vector containing the DNA, a transformant transformed with the recombinant vector, a process for producing the protein, an antibody to the protein, its partial peptide or the salt thereof, a method for determining a ligand for the protein (G protein-coupled receptor protein), a method of screening a compound for altering property of a ligand binding with the protein (G protein-coupled receptor protein) or a salt thereof, a kit for the screening, a compound for altering binding of a ligand obtainable by the screening method or the screening kit-with the protein (G protein-coupled receptor protein) or a salt thereof, and a pharmaceutical containing a compound for altering property of a ligand binding with the protein (G protein-coupled receptor protein) or a salt thereof.

The present inventors studies intensively and, as a result, we successfully isolated a cDNA encoding a human brain-derived novel protein (G protein-coupled receptor protein) and analyzed the entire nucleotide sequence. And when this nucleotide sequence was translated into an amino acid sequence, the first to the seventh transmembrane regions were confirmed on a hydrophobic plot and a protein encoded by the cDNA was confirmed to be the seventh membrane type G protein-coupled receptor protein. The present inventors further continued to study based on these findings and, as a result, completed the present invention.

That is, the present invention provides:

(1) A protein comprises the same or substantially the same amino acid sequence represented by SEQ ID No:1, SEQ ID No:3 or SEQ ID No:5, or a salt thereof,

(2) A partial peptide of the protein according to the above item (1), or a salt thereof,

(3) A DNA comprises a DNA having a nucleotide sequence encoding the protein according to the above item (1),

(4) The DNA according to the above item (3), which comprising a nucleotide sequence represented by SEQ ID No:2, SEQ ID No:4 or SEQ ID No:6,

(5) A recombinant vector comprising the DNA according to the above item (3).

(6) A transformant transformed with the recombinant vector according to the above item (5),

(7) A process for producing the protein or a salt thereof according to the above item (1), which comprises culturing the transformant according to the above item (6), and producing and accumulating the protein according to the above item (1),

(8) An antibody to the protein according to the above item (1), the partial peptide according to the above item (2) or a salt thereof,

(9) A method for determining a ligand for the protein according to the above item (1) or a salt thereof, which comprises using the protein according to the above item (1), the partial peptide according to the above item (2) or a salt thereof.

(10) A method for screening a compound or a salt thereof which alters property of a ligand binding with the protein according to the above item (1) or a salt thereof, which comprises using the protein to the above item (1), the partial peptide according to the above item (2) or a salt thereof,

(11) A kit for screening a compound or a salt thereof which alters property of a ligand binding with the protein according to the above item (1) or a salt thereof, which comprises the protein according to the above item (1), the partial peptide according to the above item (2) or a salt thereof,

(12) A compound or a salt thereof which alters property of a ligand binding with the protein according to the above item (1), or a salt thereof, which is obtainable by the method for screening according to the above item (10) or the kit for screening according to the above item (11).

(13) A pharmaceutical which comprises a compound or a salt thereof which alters property of a ligand binding with the protein according to the above item (1) or a salt thereof, which is obtainable by the method for screening according to the above item (10) or the kit for screening according to the above item (11),

(14) A DNA which hybridizes with the DNA according to the above item (3) under stringent conditions,

(15) A nucleotide containing a nucleotide sequence encoding the protein according to the above item (1), and

(16) A nucleotide containing a part of a nucleotide sequence which is complementary to a nucleotide sequence encoding the protein according to the above item (1).

More particularly, the present invention provides:

(17) The protein or a salt thereof according to the above item (1), wherein the protein is a protein containing:

① an amino acid sequence in which 1 or 2 or more (preferably around 1 - 30, more preferably around 1-9, most preferably a few) amino acids in an amino acid sequence of SEQ ID No.:1, SEQ ID No.:3 or SEQ ID No. :5 are deleted, ② an amino acid sequence in which 1 or 2 or more (preferably around 1 - 30, more preferably around 1 - 10, most preferably a few) amino acids are added to an amino acid sequence of SEQ ID No.:1, SEQ ID No.:3 or SEQ ID No.:5, ③ an amino acid sequence in which 1 or 2 or more (preferably around 1 - 30, more preferably around 1-10, most preferably a few) amino acids in an amino acid sequence of SEQ ID No. :1, SEQ ID No.:3 or SEQ ID No.:5 are substituted with other amino acids, or ④ an amino acid sequence in combination thereof,

(18) A method for determining the ligand according to the above item (9), which comprises contacting the protein or a salt thereof according to the above item (1) or the partial peptide or a salt thereof according to the above item (2) with a test compound,

(19) A method for determining the ligand according to the above item (9), wherein the ligand is angiotensin, bombesin, canabinoid, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES and the like), endothelin, enterogastrin, histamine, neurotensin, TRH, pancreatic polypeptide, galanin, α-latrotoxin, neurexophilin, subtype thereof, or analogue thereof,

(20) The method for determining a ligand according to the above item (9), wherein the ligand is α-latrotoxin, neurexophilin, subtype thereof, or analogue thereof,

(21) The screening method according to the above item (10), wherein (i) the case where the protein or a salt thereof according to the above item (1) or the partial peptide or a salt thereof according to the above item (2) is contacted with a ligand, and (ii) the case where the protein or a salt thereof according to the above item (1) or the partial peptide or a salt thereof according to the above item (2) is contacted with a ligand and a test compound are compared,

(22) A method for screening a compound or a salt thereof which alters property of a ligand binding with the protein or a salt thereof according to the above item (1), which comprises measuring and comparing an amount of a labeled ligand binding to the protein or a salt thereof according to the above item (1) or the partial peptide or a salt thereof according to the above item (2) in (i) the case where a labeled ligand is contacted with the protein or a salt thereof according to the above item (1) or the partial peptide or a salt thereof according to the above item (2) and the amount in (ii) the case where a labeled ligand and a test compound are contacted with the protein or a salt thereof according to the above item (1) or the partial peptide or a salt thereof according to the above item (3),

(23) A method for screening a compound or a salt thereof which alters property of a ligand binding with the protein or a salt thereof according to the above item (1), which comprises measuring and comparing an amount of a labeled ligand binding with a cell having the protein according to the above item (1) in (i) the case where a labeled ligand is contacted with the cell, and the amount in (ii) the case where a labeled ligand and a test compound are contacted with the cell,

(24) A method for screening a compound or a salt thereof which alters property of a ligand binding with the protein or a salt thereof according to the above item (1) which comprises measuring and comparing an amount of a labeled ligand binding with a membrane fraction of a cell having the protein according to the above item (1) in (i) the case where a labeled ligand is contacted with the cell, and the amount in (ii) the case where a labeled ligand and a test compound are contacted with the fraction,

(25) A method for screening a compound or a salt thereof which alters property of a ligand binding with the protein or a salt thereof according to the above item (1) which comprises measuring and comparing an amount of a labeled ligand binding with a protein expressed on a cell membrane of the transformant according to the above item (6) in (i) the case where a labeled ligand is contacted with the protein by culturing the transformant, and the amount in (ii) the case where a labeled ligand and a test compound are contacted with the protein by culturing the transformant,

(26) A method for screening a compound or a salt thereof which alters property of a ligand binding with the protein or a salt thereof according to the above item (1), which comprises measuring and comparing the cell stimulating activity via a protein in (i) the case where a compound which activates the protein or a salt thereof according to the above item (1) is contacted with a cell containing the protein according to the above item (1), and the activity in (ii) the case where the protein or a salt thereof according to the above item (1) and a test compound are contacted with a cell containing the protein according to the above item (1),

(27) A method for screening a compound or a salt thereof which alters property of a ligand binding with the protein or a salt thereof according to the above item (1), which comprises measuring and comparing the cell stimulating activity via a protein expressed in the transformant according to the above item (6) in (i) the case where a compound or a salt thereof which activates the protein or a salt thereof according to the above item (1) is contacted with the protein by culturing the transformant according. to the above item (6), and the activity in (ii) the case where a compound which activates the protein or a salt thereof according to the above item (1) and a test compound are contacted with the protein by culturing the transformant according to the above item (6),

(28) The screening method according to the above item (26) or (27), wherein the compound which activates the protein according to the above item (1) is angiotensin, bombesin, canabinoid, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES and the like), endothelin, enterogastrin, histamine, neurotensin, TRH, pancreatic polypeptide, galanin, α-latrotoxin, neurexophilin, subtype thereof, or analogue thereof,

(29) The screening method according to the above item (26) or (27), wherein the compound which activates the protein according to the above item (1) is α-latrotoxin, neurexophilin, a subtype thereof, or an analogue thereof,

(30) A compound which alters property of a ligand binding with the protein or a salt thereof, which is obtainable by the screening method according to the above item (21) to (28), or a salt thereof,

(31) A pharmaceutical which comprises a compound which alters property of a ligand binding with the protein or a salt thereof according to the above item (1), which is obtainable by the screening method according to the above item (21) to (28), or a salt thereof,

(32) A kit for the screening according to the above item (11), which comprises a cell containing the protein according to the above item (1),

(33) A kit for the screening according to the above item (11), which comprises a membrane fraction of a cell containing the protein according to the above item (1),

(34) A kit for the screening according to the above item (11), which comprises a protein expressed on a cell membrane of the transformant according to the above item (6) by culturing the transformant,

(35) A compound which alters property of a ligand binding with the protein or a salt thereof, which is obtainable by the kit for screening according to the above item (32) to (34), or a salt thereof,

(36) A pharmaceutical which comprises a compound which alters property of a ligand binding with the protein or a salt thereof according to the above item (1), which is obtainable by the kit for screening according to the above item (32) to (34), or a salt thereof,

(37) A method for quantitating the protein according to the above item (1), the partial peptide according to the above item (2) or a salt thereof, which comprises contacting the antibody according to the above item (8) with the protein according to the above item (1), the partial peptide according to the above item (2) or a salt thereof,

(38) A method for quantitating the protein according to the above item (1), the partial peptide according to the above item (2) or a salt thereof in a specimen solution, which comprises competitively reacting the antibody according to the above item (8) with the test solution and the labeled protein according to the above item (1), the partial peptide according to the above item (2) or a salt thereof, and determining a ratio of the protein according to the above item (1), the partial peptide according to the above item (2) or a salt thereof which is bound to the antibody,

(39) A method for quantitating the protein according to the above item (1), the partial peptide according to the above item (2) or a salt thereof in a specimen solution, which comprises reacting the specimen solution with the antibody according to the above item (8) and the labeled antibody according to the above item (8) which is unsolubilized on a carrier simultaneously or successively, and determining the activity of a labeling agent on the unsolubilized carrier.

Brief Description of the Drawings

Fig. 1 shows a nucleotide sequence encoding a human brain-derived protein of the present invention obtained in Example 1 and an amino acid sequence predicted therefrom (continued to Fig. 2).

Fig. 2 shows a nucleotide sequence encoding a human brain-derived protein of the present invention obtained in Example 1 and an amino acid sequence predicted therefrom (continued from Fig. 1).

Fig. 3 shows a hydrophobic plot of a human brain-derived protein of the present invention, which was made based on the amino acid sequences shown in Fig. 1 and Fig. 2. Parts designated by 1 - 7 show a hydrophobic domain.

Fig. 4 shows an amino acid sequence predicted from a nucleotide sequence encoding a human brain-derived protein of the present invention obtained in Example 1 (a sequence represented by HK05006 in the figure) and an amino acid sequence predicted from a nucleotide sequence encoding a human brain-derived protein of the present invention obtained in Example 2 (a sequence represented by HK05490 in the figure)(continued to Fig. 5).

Fig. 5 shows an amino acid sequence predicted from a nucleotide sequence encoding a human brain-derived protein of the present invention obtained in Example 1 (a sequence represented by HK05006 in the figure) and an amino acid sequence predicted from a nucleotide sequence encoding a human brain-derived protein of the present invention obtained in Example 2 (a sequence represented by HK05490 in the figure)(continued from Fig. 4).

Fig. 6 shows a hydrophobic plot of a human brain-derived protein of the present invention, which was made based on the amino acid sequence predicted based on a nucleotide sequence of a DNA encoding a human brain-derived protein of the present invention obtained in Example 2. Part designated by 1 - 7 show a hydrophobic domain.

Fig. 7 shows a nucleotide sequence of a DNA encoding a human brain-derived protein of the present invention obtained in Example 2, and an amino acid sequence predicted therefrom (continued to Fig. 8).

Fig. 8 shows a nucleotide sequence of a DNA encoding a human brain-derived protein of the present invention obtained in Example 2, and an amino acid sequence predicted therefrom (continued from Fig. 7 and continued to Fig. 9).

Fig. 9 shows a nucleotide sequence of a DNA encoding a human brain-derived protein of the present invention obtained in Example 2, and an amino acid sequence predicted therefrom (continued from Fig. 8 and continued to Fig. 10).

Fig. 10 shows a nucleotide sequence of a DNA encoding a human brain-derived protein of the present invention obtained in Example 2, and an amino acid sequence predicted therefrom (continued from Fig. 9 and continued to Fig. 11).

Fig. 11 shows a nucleotide sequence of a DNA encoding a human brain-derived protein of the present invention obtained in Example 2, and an amino acid sequence predicted therefrom (continued from Fig. 10 and continued to Fig. 12).

Fig. 12 shows a nucleotide sequence of a DNA encoding a human brain-derived protein of the present invention obtained in Example 2, and an amino acid sequence predicted therefrom (continued from Fig. 11 and continued to Fig. 13).

Fig. 13 shows a nucleotide sequence of a DNA encoding a human brain-derived protein of the present invention obtained in Example 2, and an amino acid sequence predicted therefrom (continued from Fig. 12 and continued to Fig. 14).

Fig. 14 shows a nucleotide sequence of a DNA encoding a human brain-derived protein of the present invention obtained in Example 2, and an amino acid sequence predicted therefrom (continued from Fig. 13 and continued to Fig. 15).

Fig. 15 shows a nucleotide sequence of a DNA encoding a human brain-derived protein of the present invention obtained in Example 2, and an amino acid sequence predicted therefrom (continued from Fig. 14).

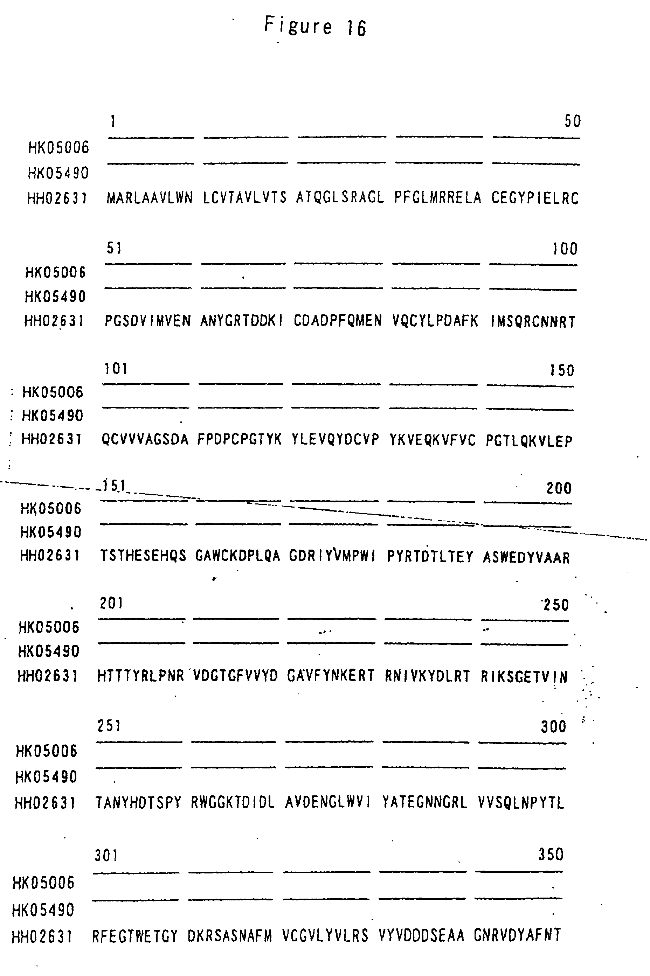

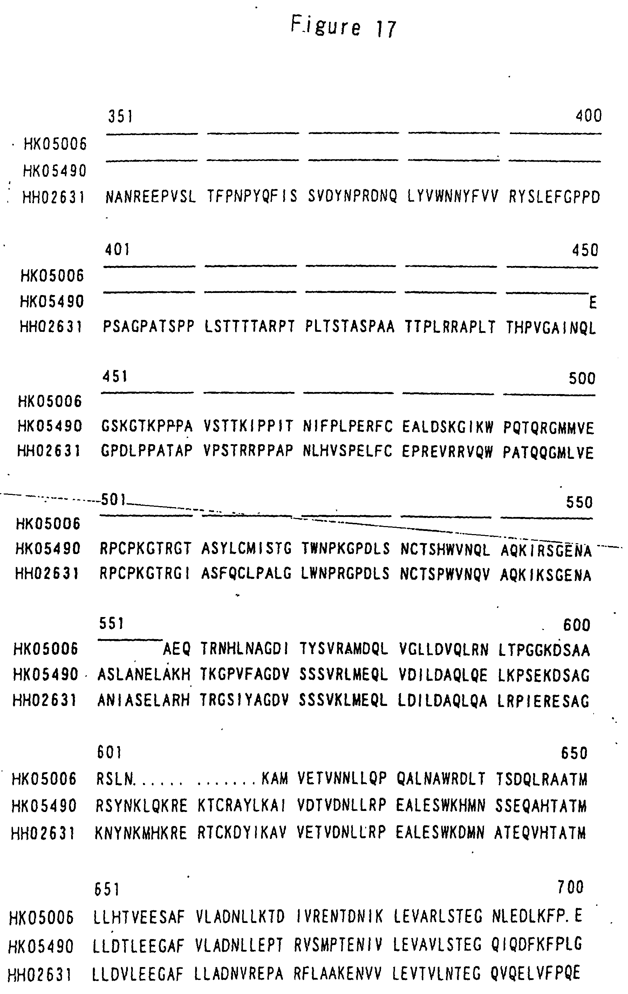

Fig. 16 shows an amino acid sequence predicted from a nucleotide sequence of a DNA encoding a human brain-derived protein of the present invention obtained in Example 1 (a sequence represented by HK05006 in the figure), an amino acid sequence predicted from a nucleotide sequence of a DNA encoding a human brain-derived protein of the present invention obtained in Example 2 (a sequence represented by HK05490 in the figure) and an amino acid sequence predicted from a nucleotide sequence of a DNA encoding a human brain-derived protein of the present invention obtained in Example 3 (a sequence represented by HH02631 in figure)(continued. to Fig.17).

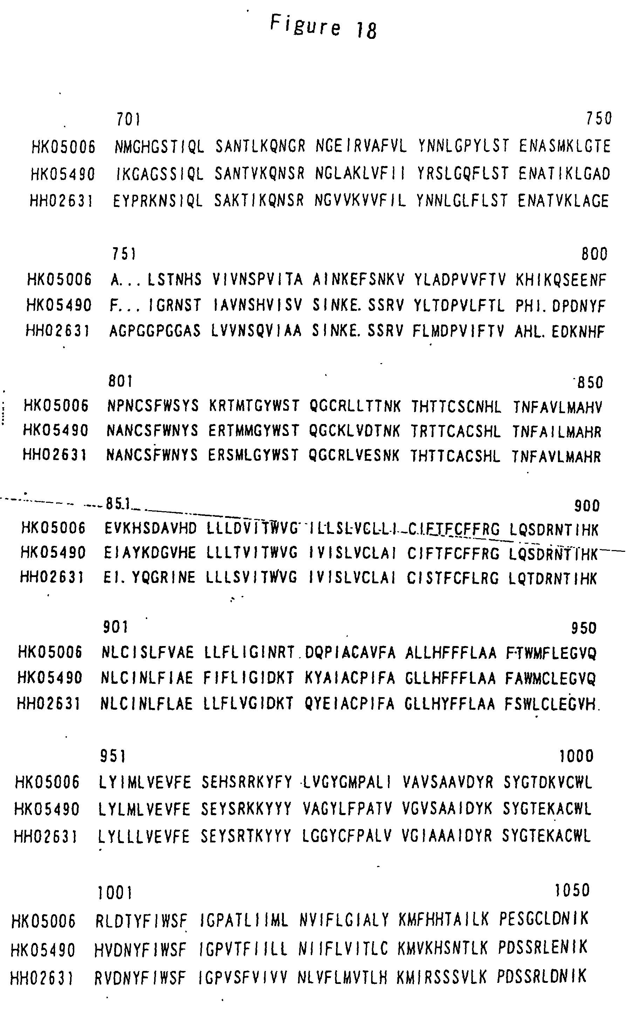

Fig. 17 shows an amino acid sequence predicted from a nucleotide sequence of a DNA encoding a human brain-derived protein of the present invention obtained in Example 1 (a sequence represented by HK05006 in the figure), an amino acid sequence predicted from a nucleotide sequence of a DNA encoding a human brain-derived protein of the present invention obtained in Example 2 (a sequence represented by HK05490 in the figure) and an amino acid sequence predicted from a nucleotide sequence of a DNA encoding a human brain-derived protein of the present invention obtained in Example 3 (a sequence represented by HH02631 in the figure)(continued to Fig.18).

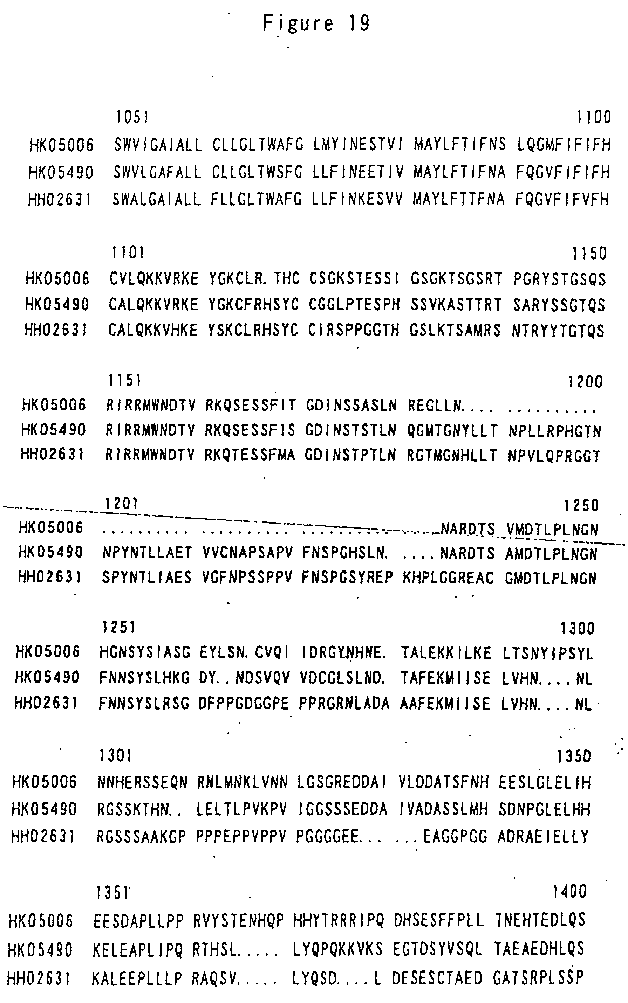

Fig. 18 shows an amino acid sequence predicted from a nucleotide sequence of a DNA encoding a human brain-derived protein of the present invention obtained in Example 1 (a sequence represented by HK05006 in the figure), an amino acid sequence predicted from a nucleotide sequence of a DNA encoding a human brain-derived protein of the present invention obtained in Example 2 (a sequence represented by HK05490 in the figure) and an amino acid sequence predicted from a nucleotide sequence of a DNA encoding a human brain-derived protein of the present invention obtained in Example 3 (a sequence represented by HH02631 in the figure)(continued to Fig.19).

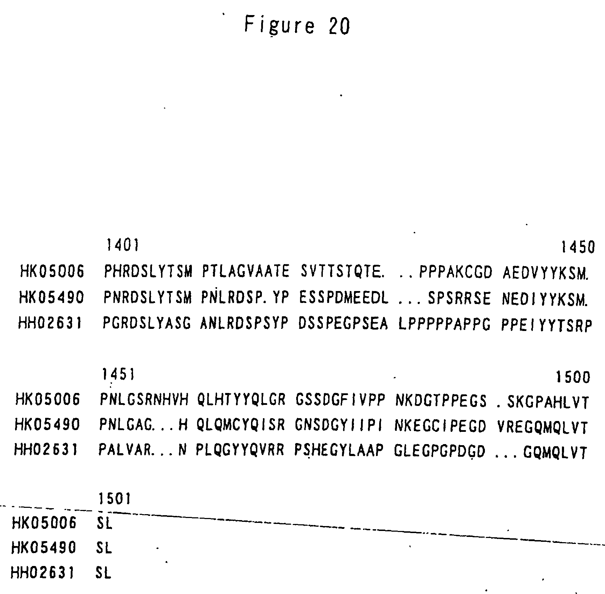

Fig. 19 shows an amino acid sequence predicted from a nucleotide sequence of a DNA encoding a human brain-derived protein of the present invention obtained in Example 1 (a sequence represented by HK05006 in the figure), an amino acid sequence predicted from a nucleotide sequence of a DNA encoding a human brain-derived protein of the present invention obtained in Example 2 (a sequence represented by HK05490 in the figure) and an amino acid sequence predicted from a nucleotide sequence of a DNA encoding a human brain-derived protein of the present invention obtained in Example 3 (a sequence represented by HH02631 in the figure)(continued to Fig.20).

Fig. 20 shows an amino acid sequence predicted from a nucleotide sequence of a DNA encoding a human brain-derived protein of the present invention obtained in Example 1 (a sequence represented by HK05006 in the figure), an amino acid sequence predicted from a nucleotide sequence of a DNA encoding a human brain-derived protein of the present invention obtained in Example 2 (a sequence represented by HK05490 in the figure) and an amino acid sequence predicted from a nucleotide sequence of a DNA encoding a human brain-derived protein of the present invention obtained in Example 3 (a sequence represented by HH02631 in the figure)(continued from Fig.19).

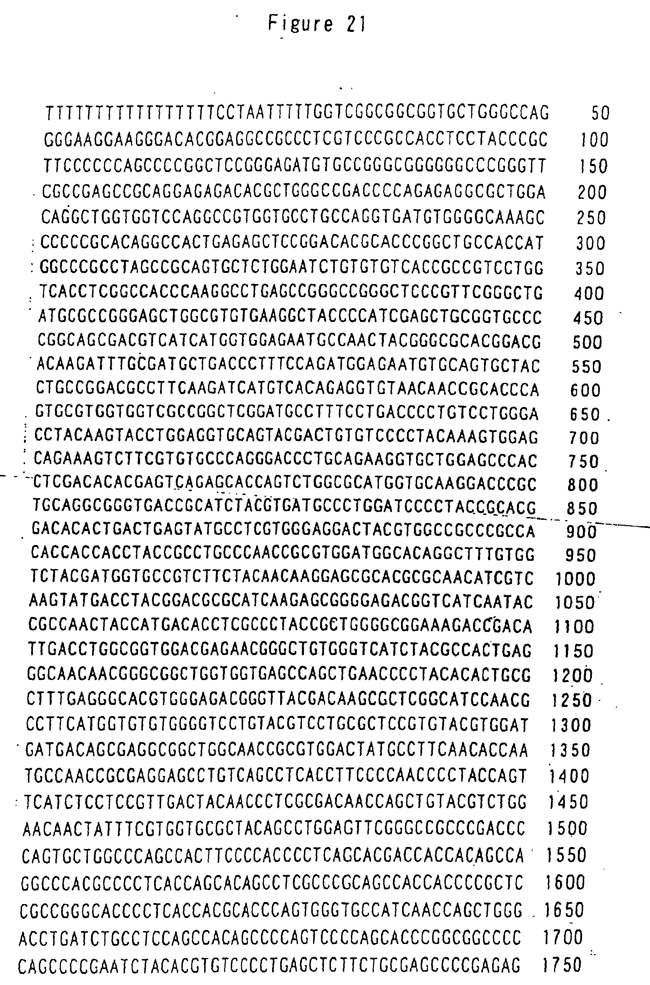

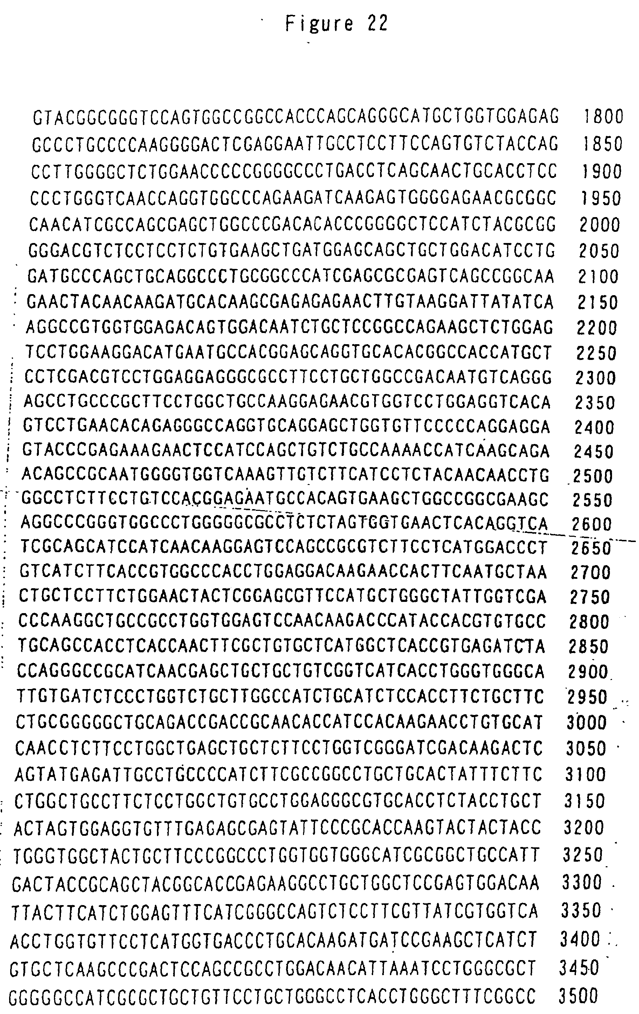

Fig. 21 shows a nucleotide sequence contained in the plasmid pHH02631 obtained in Example 3 (continued to Fig. 22).

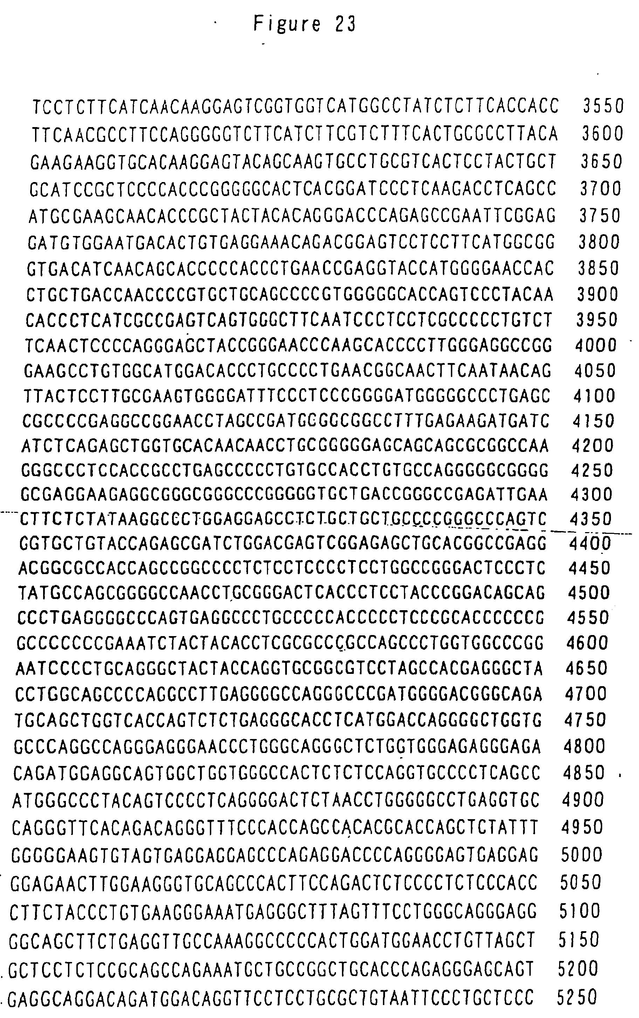

Fig. 22 shows a nucleotide sequence contained in the plasmid pHH02631 obtained in Example 3 (continued to Fig. 23).

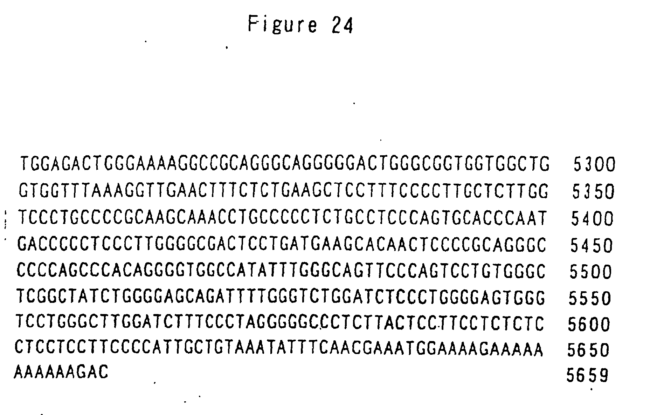

Fig. 23 shows a nucleotide sequence contained in the plasmid pHH02631 obtained in Example 3 (continued to Fig. 24).

Fig. 24 shows a nucleotide sequence contained in the plasmid pHH02631 obtained in Example 3 (continued from Fig. 23).

Fig. 25 shows a hydrophobic plot of a human brain-derived protein of the present invention, which was made based on an amino acid sequence predicted from a nucleotide sequence of a DNA encoding a human brain-derived protein of the present invention obtained in Example 3. Parts designated by 1 - 7 show a hydrophobic domain.

Best Mode for Carrying Out the Invention

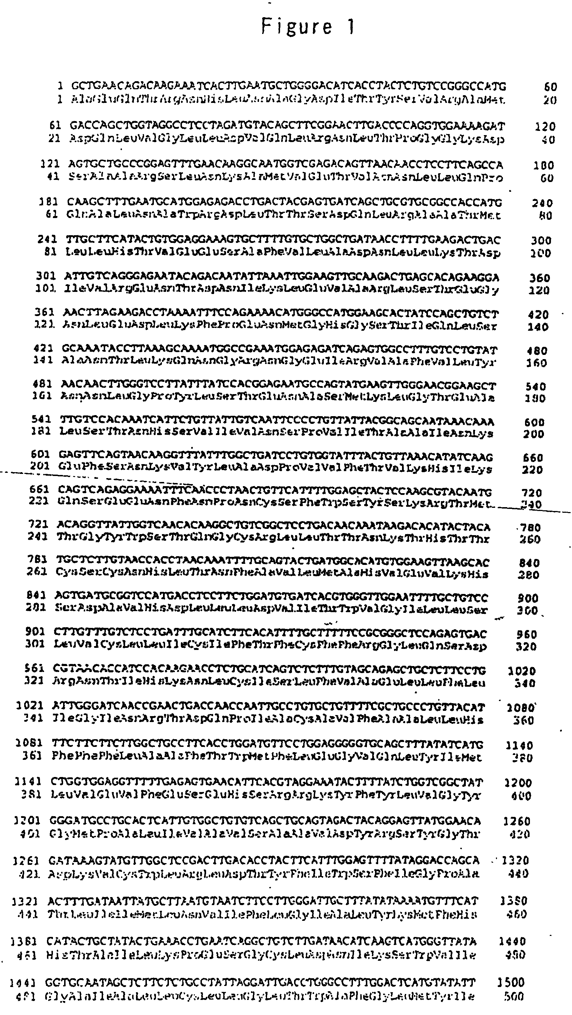

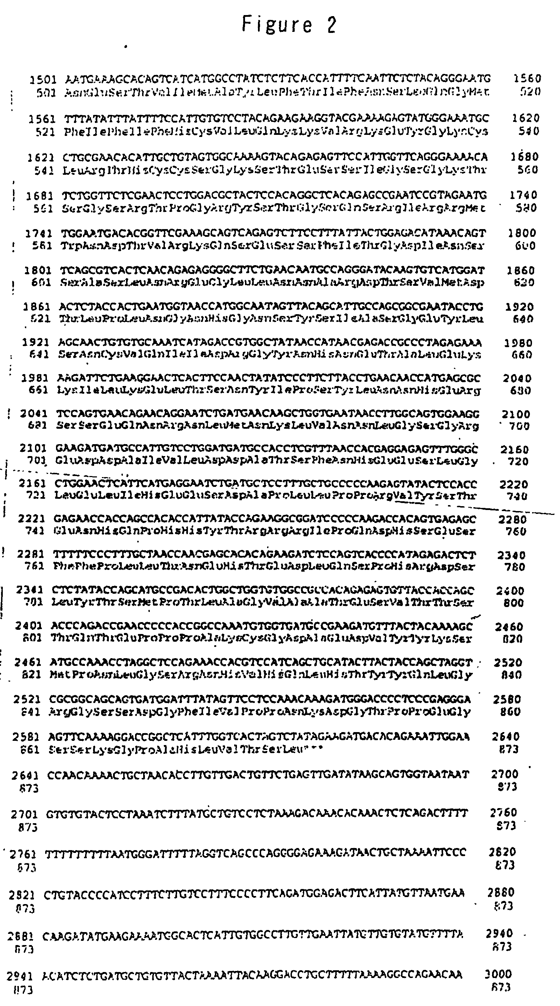

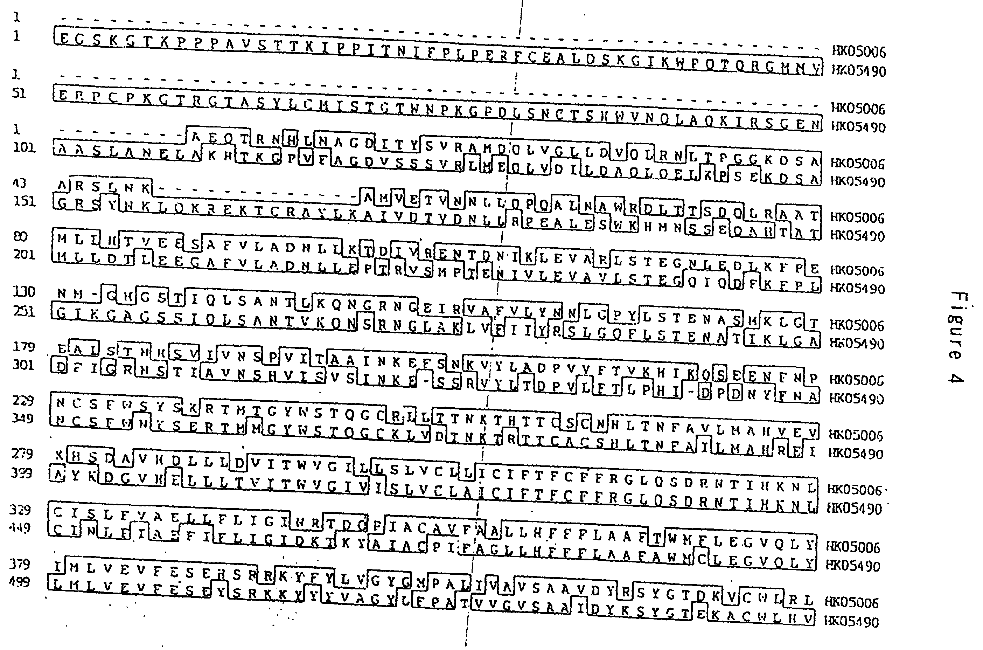

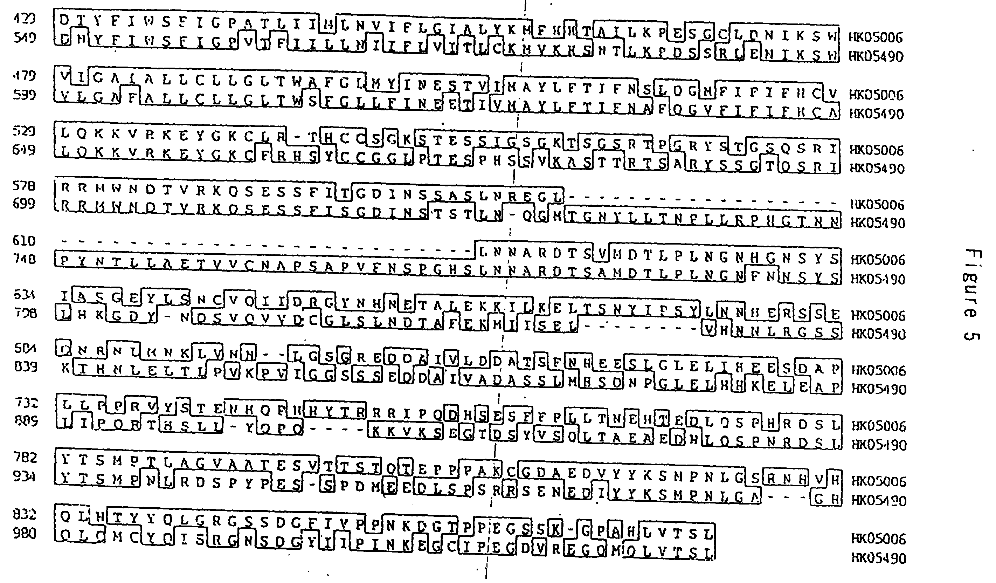

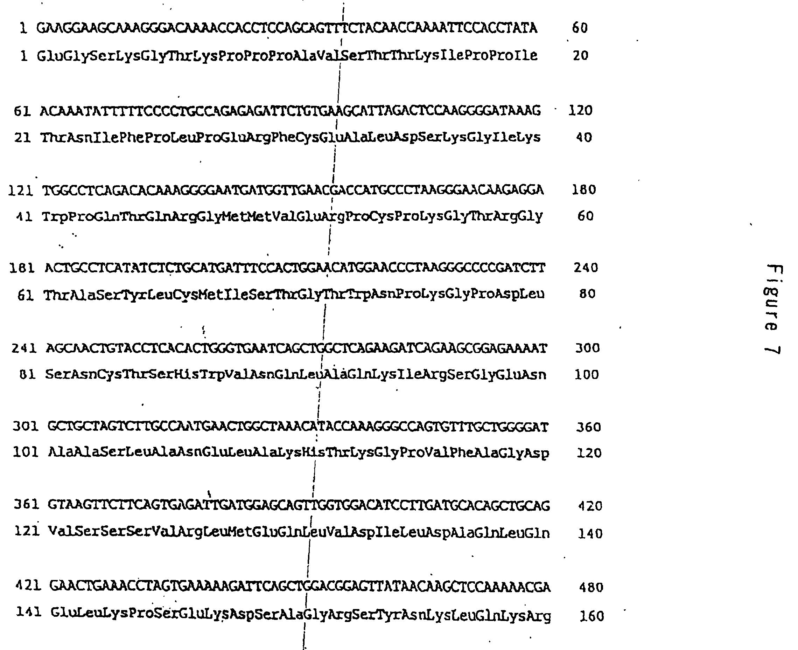

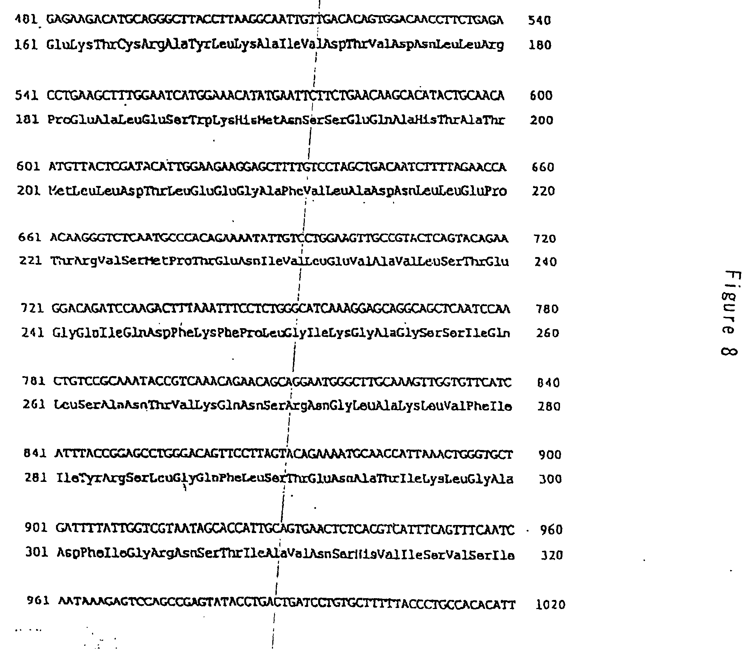

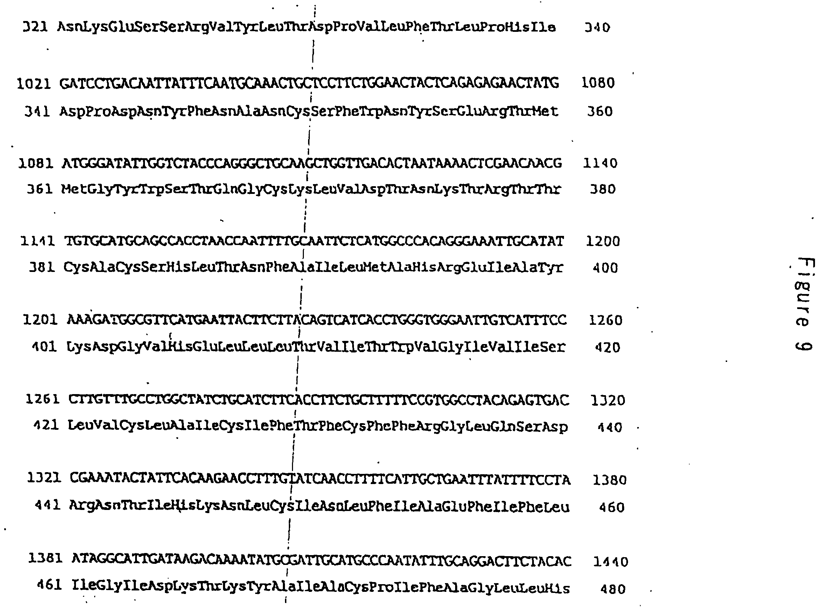

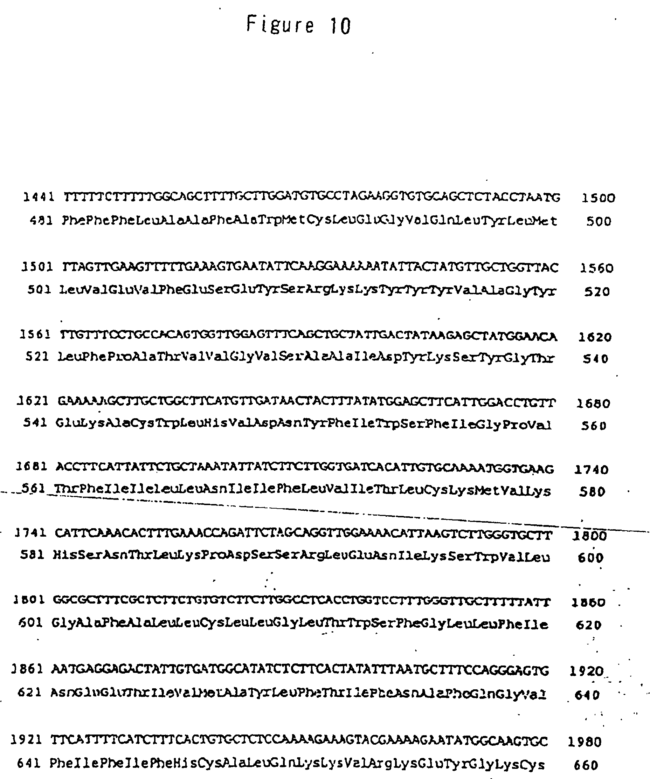

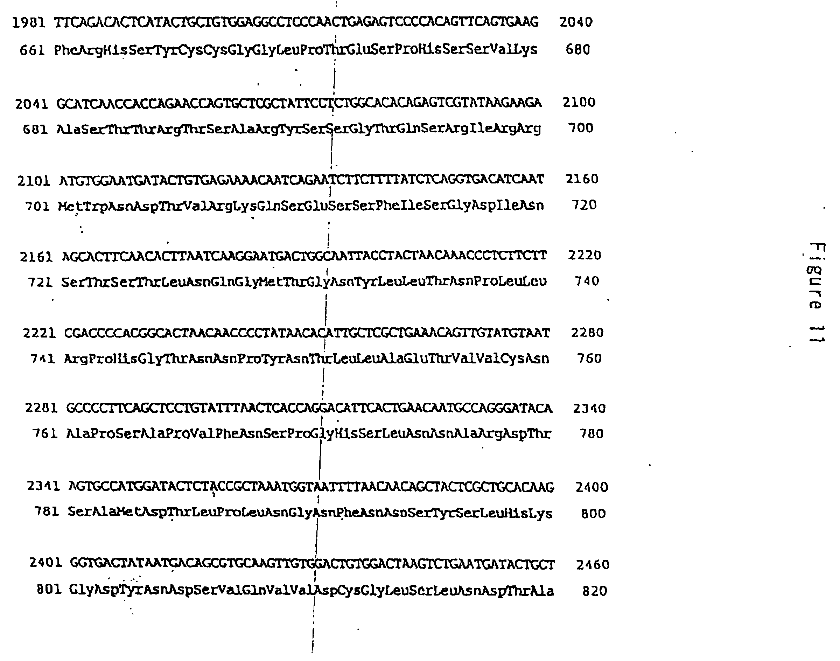

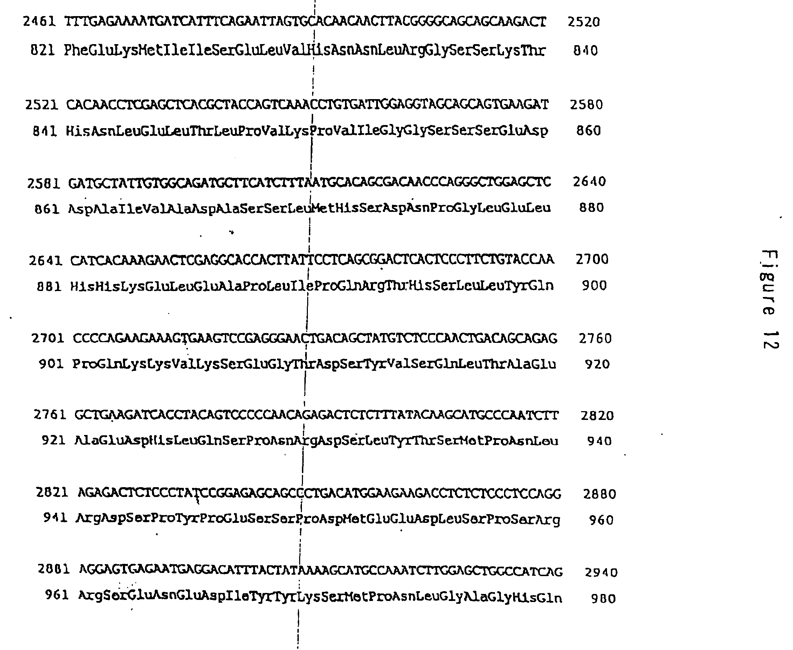

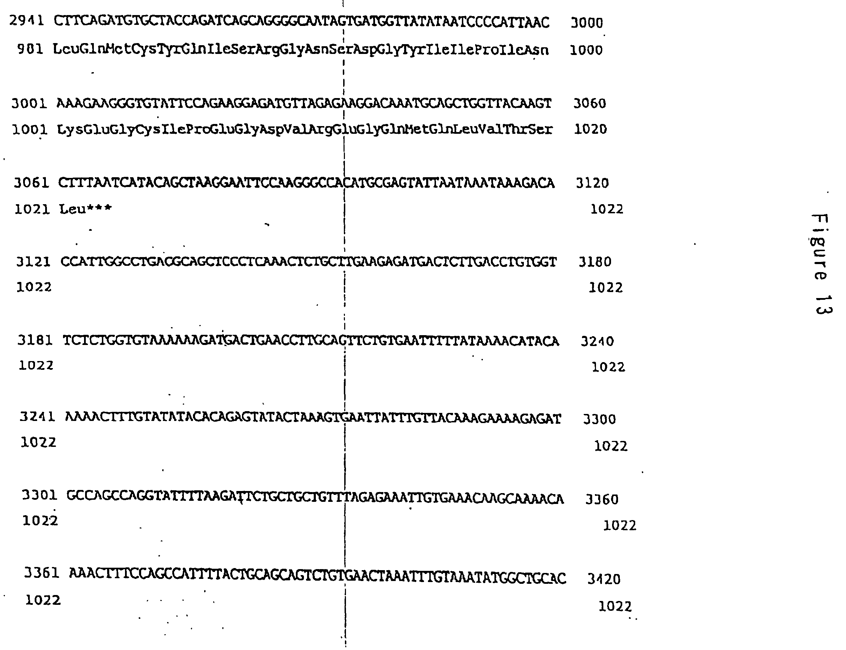

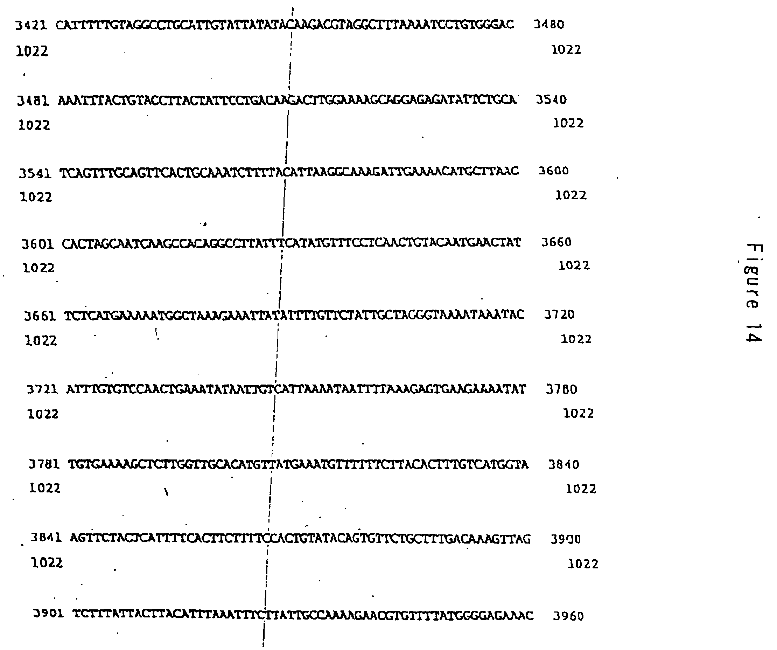

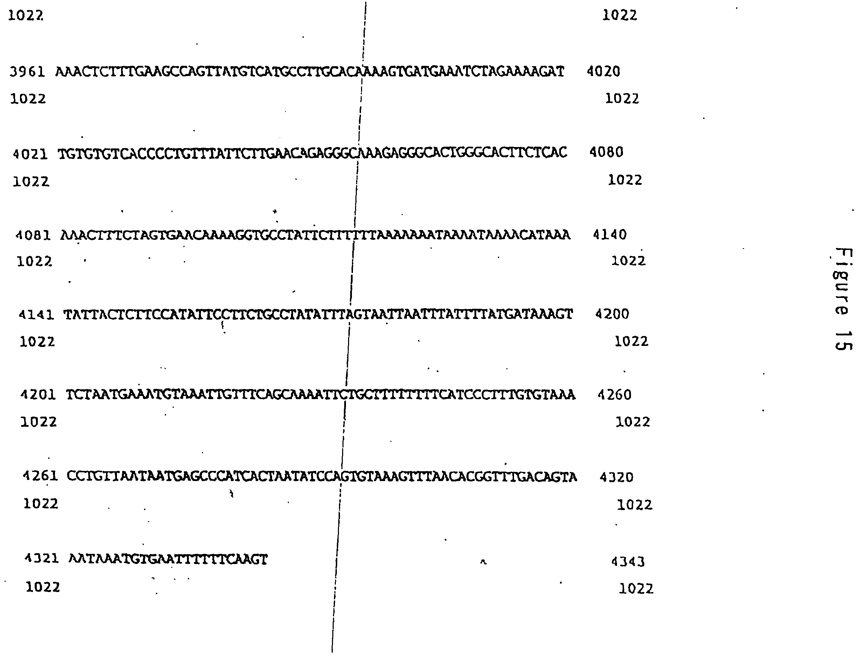

A protein of the present invention (G protein-coupled receptor protein) is a receptor protein containing the same or substantially the same amino acid sequence as amino acid sequence represented by SEQ ID No.:1 [amino acid sequence of Fig. 1 and Fig. 2], an amino acid sequence represented by SEQ ID No.:3 [amino acid sequence represented by HK05490 in Fig.4 and Fig. 5;an amino acid sequence in. Fig.7 to Fig. 15] or an amino acid sequence represented by SEQ ID No.:5 [amino acid sequence represented by HH02631 Fig.16 to Fig.20] (hereinafter a protein of the present invention (G protein-coupled receptor protein) or a salt thereof is abbreviated as present protein in some cases)))).

Present protein (G protein conjugated type protein) may be derived from, for example, in cases of human being and mammal (for example, guinea pig, rat, mouse, rabbit, pig, sheep, cow, monkey and the like), any cell (for example, spleen cell, nerve cell, glia cell, pancreatic β cell, marrow cell, mesangial cell, Langerhans's cell, epidermic cell, epithelial cell, endothelial cell, fibroblast, fibrocyte, myocyte, fat cell, immunocyte (for example, macrophage, T cell, B cell, natural killer cell, mast cell, neutrophil, basophil, eosinophil, monocyte), megakaryocyte, synovial cell, chondrocyte, bone cell, osteoblast, osteoclast, mammary cell, hepatic cell or interstitial cell, or precursor cell, stem cell or cancer cell thereof) and blood cell (for example, MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01 and the like), or any tissue in which the above cells are present, for example, brain, each site of brain (bulb olfactorius, tonsil nucleus, cerebral basal bulb, hippocampus, thalamus, hypothalamus, subthalamic nucleus, cerebral cortex, meddula oblongata, cerebellum, occipital lobe, frontal lobe, temporal lobe, putamen, caudatum, corpus callosum, nigra), spinal cord, pituitary gland, stomach, pancreas, kidney, liver, gonad, thyroid gland, cholecystis, bone marrow, drenal gland, skin, muscle, lung, alimentary tract (for example, large intestine, small intestine), blood vessel, cardiac thymus, spleen, submadibular gland, peripheral blood, peripheral blood cell, prostate, testis, testicle, ovary, placenta, uterus, bone, joint, skeletal muscle (in particular, brain and respective sites of brain), or a synthetic protein.

An amino acid sequence which is the same or substantially the same as an amino acid sequence represented by SEQ ID No.:1, SEQ ID No.:3 or SEQ ID No.: 5, there are an amino acid sequence having about 50% or more, preferably about 70% or more, more preferably about 80% or more, further preferably about 90% or more, most preferably about 95% or more homology with an amino acid sequence represented by SEQ ID No.:1, SEQ ID No.:3 or SEQ ID No.: 5.

As a protein having an amino acid sequence which is the same or substantially the same as an amino acid sequence represented by SEQ ID No.:1, SEQ ID No.:3 or SEQ ID No.: 5, a protein having the substantially same amino acid sequence as an amino acid sequence represented by SEQ ID No.:1, SEQ ID No.:3 or SEQ ID No.: 5 and having substantially the same nature of activity as that of an amino acid sequence represented by SEQ ID No.:1, SEQ ID No.:3 or SEQ ID No.: 5.

As the activity of substantially the same nature, for example, there is a ligand binding property, signal information transmitting action and the like. "Substantially the same" denotes that their activities are the same in nature. Therefore, it is preferable that the activities such as ligand binding activity and signal information transmitting action are equivalent (for example, about 0.5-2-fold) but quantitative elements such as an extent of these activities and a molecular weight of a protein may be different.

Measurement of the activities such as ligand binding and signal transmitting action can be performed according to the method known per se but, for example, measurement can be performed according to a method for determining a ligand or a method for screening described below.

In addition, as present protein, proteins having ① an amino acid sequence in which 1 or 2 or more (preferably around 1 - 30, more preferably around 1 -10, further preferably a few (1 or 2)) amino acids in an amino acid sequence represented by SEQ ID No.:1, SEQ ID No.:3 or SEQ ID No.: 5 are deleted, ② an amino acid sequence in which 1 or 2 or more (preferably around 1 - 30, more preferably around 1 - 10, further preferably a few (1 or 2)) amino acids are added to an amino acid sequence represented by SEQ ID No.:1, SEQ ID No.:3 or SEQ ID No.: 5, ③ an amino acid sequence in which 1 or 2 or more (preferably around 1 - 30, more preferably around 1 - 10, further preferably a few (1 or 2)) amino acids in an amino acid sequence represented by SEQ ID No.:1, SEQ ID No.:3 or SEQ ID No.: 5 are substituted with an another amino acid, or ④ amino acid sequence in combination thereof may be used.

In present protein, a left end is a N-terminal (amino terminal) and a right end is a C-terminal (carboxyl terminal) according to the usual convention. In present protein including a protein having an amino acid sequence represented by SEQ ID No.:1, SEQ ID No.:3 or SEQ ID No.:5, a C-terminal is usually a carboxyl group (-COOH) or carboxylate ($-COO^-$) but a C-terminal may be amide ($-CONH_2$) or ester (-COOR).

As R in an ester, $C_{1-6}$ alkyl group such as methyl, ethyl, n-propyl, isopropyl and n-butyl, $C_{3-8}$ cycloalkyl group such as cyclopentyl and cyclohexyl, $C_{6-12}$ aryl group such as phenyl and α-naphthyl, phenyl-$C_{1-2}$ alkyl group such as benzyl and phenethyl, or $C_{7-14}$ aralkyl group such as α-naphthyl-$C_{1-2}$ alkyl group such as α-naphthylmethyl as well as pivaloyloxymethyl group which is used for an oral ester may be used.

When present protein has a carboxyl group (carboxylate) at a site other than a terminal, a protein in which a carboxyl group is amidated or esterified is contained in present protein. As an ester of this case, for example, the aforementioned C-terminal ester and the like are used.

Further, the aforementioned protein in which an amino group of a methionine residue at a N-terminal is protected with a protecting group (for example, $C_{1-6}$ acyl group such as formyl group, acetyl group), in which a N-terminal side is cut in the living body and the produced glutamyl group is pyroglutamine-oxidized, or in which a substituent (for example, -OH, -COOH, amino group, imidazole group, indole group, guianidino group) on a side chain of an intramolecular amino acid is protected with a suitable protecting group (for example, $C_{1-6}$ acyl group such as formyl group, acetyl group), and a glucoprotein in which a sugar chain is bound are included in present protein.

As an embodiment of present protein, for example, a human-derived (more preferably human brain-derived) protein having an amino acid sequence represented by SEQ ID No.:1, SEQ ID No.:3 or SEQ ID No. :5 and the like are used.

As a partial peptide of present protein (hereinafter referred to as partial peptide in some cases), any partial peptide may be used as long as it is a partial peptide of the aforementioned present protein, for example, a site which is protruding outside a cell membrane and has a receptor activity within present protein is used.

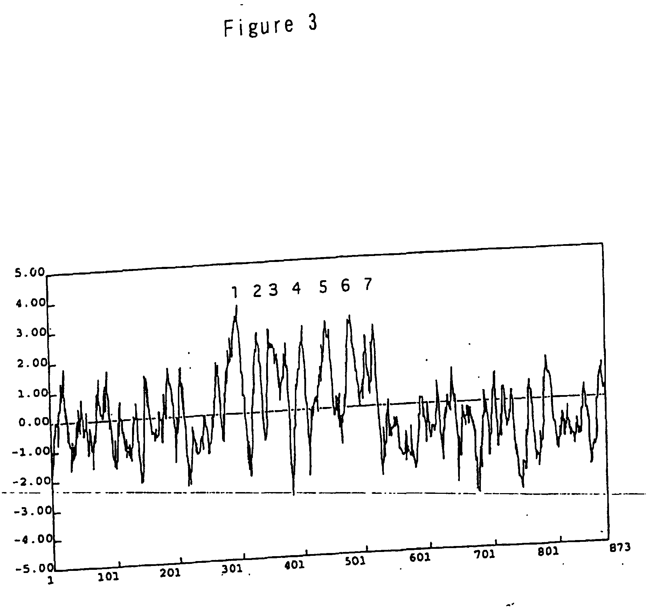

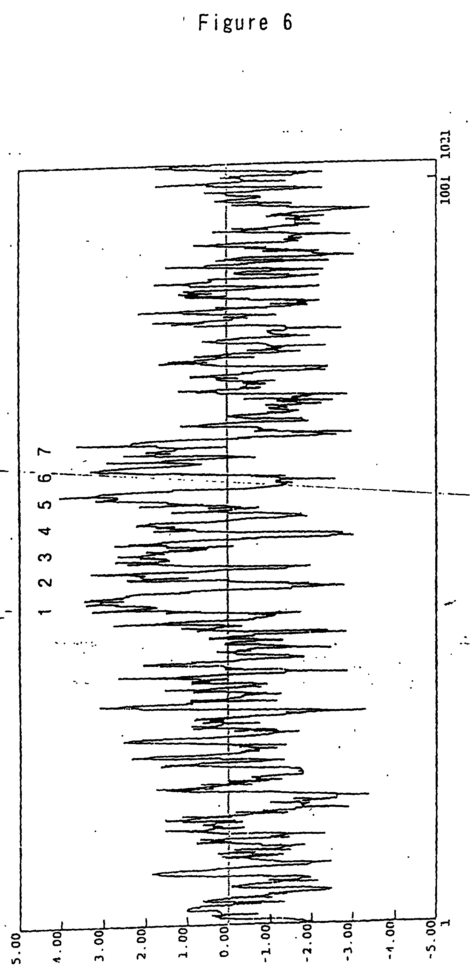

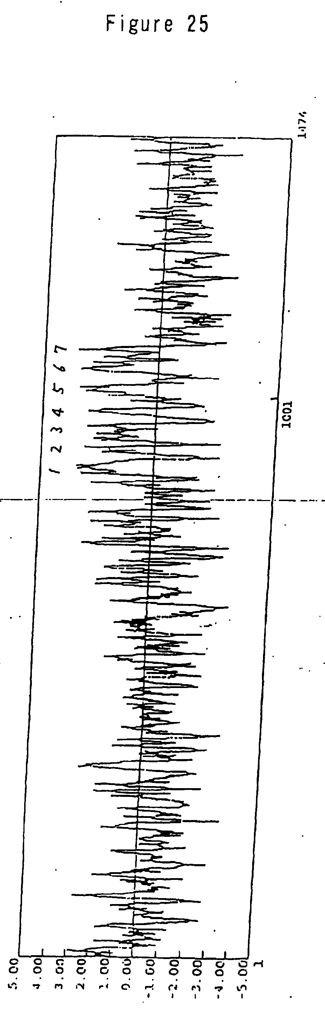

More particularly, as a partial peptide of a protein having an amino acid sequence represented by SEQ ID No.:1, SEQ ID No. :3 or SEQ ID No.:5, it is a peptide containing a part which is analyzed to be an extracellular region (hydrophilic site) in hydrophobic plot analysis shown by [Fig.3], [Fig.6] or [Fig. 25]. In addition, a peptide containing a hydrophobic region in its part may be also used. A peptide containing separately an individual domain may be used, a peptide of a part containing a plurality of domains at the same time may be used.

Regarding the number of amino acids of a partial peptide of the present invention, a peptide having at least 20 or more, preferably 50 or more, more preferably 100 or more amino acid sequences among component amino acid sequences of the aforementioned present protein is preferable.

"Substantially the same amino acid sequence" denotes an amino acid sequence having about 50% or more, preferably about 70% or more, more preferably about 80% or more, further preferably about 90% or more, most preferably about 95% or more homology with these amino, acid sequences.

Here, "substantially the same nature of activity" is as defined above. Measurement of "substantially the same nature of activity" can be performed as described above.

In addition, in a partial peptide of the present invention, 1 or 2 or more (preferably around 1 - 10, more preferably a few (1 or 2) amino acids may be deleted from the aforementioned amino acid sequence, 1 or 2 or more (preferably around 1 - 20, more preferably around 1 - 10, further preferably a few (1 or 2) amino acids may be added to the aforementioned amino acid sequence, or 1 or 2 or more (preferably around 1 - 10, more preferably around 1 - 5, further preferably a few (1 or 2) amino acids in the aforementioned amino acid sequence may be substituted with an another amino acid.

In addition, in a partial peptide of the present invention, a C-terminal is usually a carboxyl group (-COOH) or carboxylate ($-COO^-$) but, as in the aforementioned present protein, a C-terminal may be amide ($-CONH_2$) or ester (-COOR).

Further, as in the aforementioned present protein, a partial peptide in which an amino group of a methionine residue of a N-terminal is protected with a protecting group, a partial peptide in which a N-terminal side is cut in the living body and the produced Gln is pyroglutamine-oxidized, a partial peptide in which a substituent on a side chain of an intramolecular amino acid is protected with a suitable protecting group, and a composite peptide such as a glucopeptide in which a sugar chain is bound thereto are included in a partial peptide of the present invention.

In addition, in a partial peptide of the present invention, a C-terminal is usually a carboxyl group (-COOH) or carboxylate ($-COO^-$) but, as in the aforementioned present protein, a C-terminal maybe amide ($-CONH_2$) or ester (-COOR).

As salts of present protein or a partial peptide thereof, inter alia, physiologically acceptable acid addition salts are preferable. As such the salt, for example, salts with inorganic acids (such as hydrocloric acid, phosphoric acid, hydrobromic acid, sulfuric acid), salts with organic acids (such as acetic acid, formic acid, propioic acid, fumaric acid, maleic acid, succinic acid, tartaric acid, citric acid, malic acid, oxalic acid, benzoic acid, methanesulfonic acid, benzenesulfonic acid) and the like are used.

Present protein or a salt thereof can be prepared from the aforementioned cells or tissues derived from human or a mammal by the method of purifying a protein which is known per se, or may be prepared by culturing a transformant containing a DNA encoding present protein described below. Alternatively, the protein may be prepared by a method for synthesizing a protein described below or a similar method.

When prepared from tissues or cells of human being or a mammal, tissues or cells of human being or a mammal are homogenized and, thereafter, extraction is performed with an acid or the like, and the extract may be purified and isolated by combining chromatography such as reverse phase chromatography, ion-exchange chromatography and the like.

In order to synthesize present protein, or a partial peptide thereof or a salt thereof or an amide thereof, a commercially available resin for synthesizing a protein may be usually used. As such the resin, for example, there are chloromethyl resin, hydroxymethyl resin, benzhdrylamine resin, aminomethyl resin, 4-benzyloxybenzylalcohol resin, 4-methylbenzhydrylamine resin, PAM resin, 4-hydroxymethylmethylphenylacetamidemethyl resin, polyacrylamide resin, 4-(2',4'-dimethoxyphenyl-hydroxymethyl)phenoxy resin, 4-(2',4'-dimethoxyphenyl-Fmoc aminoethyl)phenoxy resin and the like. By using such the resin, α-amino acid and an amino acid having a side functional group suitably protected are condensed on a resin as a sequence of a protein of interest according to various condensing methods which are known per se. At the end of a reaction, a protein is cut from a resin and at the same time various protecting groups are removed and, further, an intramolecular disulfide linkage forming reaction is performed and a protein of interest or an amide thereof is obtained.

As the aforementioned condensation of a protected amino acid, although various activating reagents which can be used for synthesizing a protein may be used, in particular, carbodiimides are better. As carbodiimides, DCC, N',N'-diisopropylcarbodiimide, N-ethyl-N'-(3-dimethylaminoprolyl)carbodiimide and the like are used. For activation by them, a protected amino acid is directly added to a resin together with a racemization inhibiting agent (for example, HOBt, HOOBt), or after a protected amino acid is activated in advance as a symmetrical anhydride or HOBt ester or HOOBt ester, it may be added to a resin.

A solvent used for activating a protected amino acid or condensing with a resin may be appropriately selected from solvents which are known to be used for a protein synthesizing reaction. For example, acid amides such as N,N-dimethylformamide, N,N-dimethylacetamide, N-methylpyrrolidone and the like, halogenated hydrocarbons such as methyl chloride, chlorofom and the like, alcohols such as trifluoroethanol and the like, sulfoxides such as dimethyl sulfoxide and the like, ethers such as pyridine, dioxane, tetrahydrofuran and the like, nitriles such as acetonitrile, propionitrile and the like, esters such as methyl acetate, ethyl acetate and the like or an appropriate mixture thereof may be used. A reaction temperature is appropriately selected from a range which is known to be used for a protein binding forming reaction and is usually selected from a range of about -20°C to 50°C. An activated amino acid derivative is usually used at 1.5-4 fold excess amount. When condensation is insufficient as a result of a test using a ninhidrin reaction, sufficient condensation can be performed by repeating a condensation reaction without leaving a protecting group. When sufficient condensation is not obtained even by repeating a reaction, an unreacted amino acid may be acetylated using acetic anhydride or acetylimidazole.

As a protecting group for a raw material amino acid, for example, Z, Boc, tertiary pentylcarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfonyl, diphenylphosphinothioyl, Fmoc and the like are used.

A carboxyl group can be protected by, for example, alkylesterification (straight, branched or cyclic alkylesterification such as methyl, ethyl, propyl, butyl, tertiary-butyl, cyclopentyl, cyclohexyl, cyclopentyl, cyclooctyl, 2-adamantyl and the like), aralkylesterification (for example, benzylester, 4-nitrobenzylester, 4-methoxybenzylester, 4-chlorobenzylester, benzhydrylesterification), phenacylesterification, benzyloxycarbonylhydrizidation, tertiary-butoxycarbonylhydrizidation, tritylhydrizidation and the like.

A hydroxy group of serine can be protected by, for example, esterification or etherification. As a group suitable for this esterification, for example, lower alkanoyl group such as acetyl group, aroyl group such as benzoyl group, a group derived from carbonic acid such as benzyloxycarbonyl group, ethoxycarbonyl group and the like are used. In addition, a group suitable for etherification, for example, there are benzyl group, tetrhydropyranyl group, t-butyl group and the like.

As a protecting group for a phenolic hydroxy group of thyrosine, for example, Bzl, $Cl_2$-Bzl, 2-nitobenzyl, Br-Z, tertiary-butyl and the like are used.

As a protecting group for imidazole of histidine, for example, Tos, 4-methoxy-2,3,6-trimethylbenzenesulfonyl, DNP, benzyloxymethyl, Bum, Boc, Trt, Fmoc and the like are used.

As activated carboxyl group of a raw material, for example, corresponding anhydride, azide, active ester [ester with alcohol (for example, pentachlorophenol, 2,4,5-trichlorophenol, 2,4-dinitrophenol, cyanomethylalcohol, paranitrophenol, HONB, N-hydroxysuccinimide, N-hydroxyphthalimide, HOBt)] and the like are used. As an activated amino group of a raw material, for example, corresponding phosphoric amide is used.

As a method of removing (eliminating) a protecting group, for example, catalytic reduction in a hydrogen stream in the presence of a catalyst such as Pd-black or Pd-carbon, treatment with an acid such as anhydrous hydrofluoric acid, methanesulfonic acid, trifluoromethanesulfonic acid, trifluoroacetic acid or a mixture thereof, treatment with a base such as diisopropylethylamine, triethylamine, piperidine and the like, and reduction with sodium in liquid ammonia are used. In the acid treatment, for example, the addition of a cation capturing agent such as anisole, phenol, thioanisole, methacresol, paracresol, dimethyl sulfide, 1,4-butanediol, 1,2-ethanedithiol and the like is effective. In addition, 2,4-dinitrophenyl group used as a protecting group for imidazole of histidine is removed by treatment with thiophenol, and formyl group used as a protecting group for indole of tryptophan is also removed by alkali treatment with an aqueous dilute solution of sodium hydroxide, dilute ammonia and the like, in addition to deprotection by the aforementioned acid treatment in the presence of 1,2-ethanedithiol, 1,4-butanedithiol and the like.

Protection of functional groups which should not be involved in a reaction of a raw material and protecting group, elimination of the protecting groups; and activation of functional groups which should be involved in a reaction can be appropriately selected from the known groups and the known means.

As an another method for obtaining an amide of a protein, for example, an α-carboxyl group of a carboxyl terminal amino acid is first amidated for protection and, thereafter, a peptide (protein) chain is extended to a desired chain length on an amino group side, a protein in which only a protecting group for a N-terminal α-amino acid of the peptide chain is removed and a protein in which only a protecting group for a C-terminal carboxyl group is removed are prepared, and both proteins are condensed in the aforementioned mixed solvent. The details of a condensing reaction are as defined above. After a protected protein obtained by condensation is purified, all the protecting groups can be removed by the aforementioned method to obtain a crude desired protein. The crude protein can be purified by the known various purifying means and main fractions can be lyophylized to obtain an amide of a desired protein.

In order to obtain an ester, for example, after a α-carboxyl group of a carboxy terminal amino acid is condensed with a desired alcohols to make an amino acid ester, an ester of a desired protein can be obtained as in an amide of a protein.

Present protein and a salt thereof can be prepared according to the method of synthesizing a peptide which is known per se, or by cutting present protein with a suitable peptidase. A method of synthesizing a peptide may be by a solid phase synthesizing method or a solution phase synthesizing method. That is, a partial peptide or amino acids which can constitute present protein can be condensed with the remaining part and, when a product has a protecting group, a protecting group can be eliminated to prepare a peptide of interest. As the known condensing method and elimination of a protecting group, for example, there are methods described in the following ① -⑤ .

① M. Bondanszky and M.A. Ondetti, Peptide Synthesis, Interscience Publishers, New York (1966),
② Schroeder and Luebke, The Peptide, Academic Press, New York (1965),
③ Nobuo Izumiya and others, Fundament and Experiment of Peptide Synthesis, Maruzen (K.K.)(1975)
④ Haruaki Yajima and Shunpei Sakakibara, Biochemistry Experiment 1, Chemistry for Proteins IV, 205, (1977),
⑤ Supervised by Haruaki Yajima, Development of Drugs, Second Series, vol.14, Peptide Synthesis, Hirokawa Shoten.

In addition, after a reaction, a partial peptide of the present invention can be purified and isolated by combining, for example, solvent extraction, distillation, column chromatography, liquid chromatography, recrystallization and the like. When a partial peptide obtained by the aforementioned method is a free compound, it can be converted into a suitable salt by the known method and, conversely, when it is obtained as a salt, it can be converted into a free compound by the known method.

As a DNA encoding present protein, it may be any DNA as long as it contains a nucleotide sequence encoding the aforementioned present protein. In addition, it may be any of a genomic DNA, a genomic DNA library, a cDNA derived from the aforementioned cells or tissues, a cDNA library derived from the aforementioned cells or tissues, and a synthetic DNA. A vector used in a library may be any of bacteriophage, plasmid, cosmid, phagemide and the like. In addition, a total RNA or a mRNA fraction is prepared from the aforementioned cells or tissues, which may be used to directly amplify by Reverse Transcriptase Polymerase Chain Reaction (hereinafter, abbreviated as RT-PCR method).

More particularly, a DNA encoding present protein may be, for example, a DNA having a nucleotide sequence which hybridizes with a nucleotide sequence represented by SEQ ID No.:2, SEQ ID No.:4 or SEQ ID No.:6, or any DNA having a nucleotide sequence which hybridizes with a nucleotide sequence represented by SEQ ID No.:2, SEQ ID No.:4 or SEQ ID No.:6 under the stringent conditions and encoding a protein having substantially the same nature of activity (for example, ligand binding activity, signal information transmitting action and the like) as that of present protein.

As a DNA which can hybridize with a nucleotide sequence represented by SEQ ID No.:2, SEQ ID No.:4 or SEQ ID No.:6, a DNA having a nucleotide sequence having about 70% or more, preferably about 80% or more, more preferably about 90% or more, most preferably about 95% or more homology with a nucleotide sequence represented by SEQ ID No.:2, SEQ ID No.:4 or SEQ ID No.:6 is used.

Hybridization can be performed according to the method known per se or a similar method, for example, a method described in Molecular Cloning, 2nd (J. Sambrook et al., Cold Spring Harbor Lab. Press, 1989). In addition, when a commercially available library is used, hybridization can be performed by a method described in the attached specification. More preferably, hybridization can be performed according to the high stringent conditions.

High stringent conditions show the conditions in which the sodium concentration is about 19-40 mM, preferably about 19-20 mM, and a temperature is about 50-70°C, preferably about 60-65°C. In particular, the case where the sodium concentration is about 19 mM and a temperature is about 65°C is the most preferable.

More particularly, as a DNA encoding a protein having an amino acid sequence represented by SEQ ID No.:1, SEQ ID No.:3 or SEQ ID No.:5, a DNA having a nucleotide sequence represented by SEQ ID No.:2, SEQ ID No.:4 or SEQ ID No.:6 is used.

A nucleotide (oligonucleotide) a nucleotide sequence having a nucleotide sequence encoding present protein, or having a part of a nucleotide sequence complementary to the aforementioned nucleotide sequence is meant to include not only a DNA but also a RNA encoding present protein or a partial peptide thereof.

According to the present invention, an antisense • (oligo)nuclotide (nucleic acid) can be designed and synthesized based on a nucleotide sequence information of a nucleotide sequence encoding a cloned or sequenced protein. Such the (oligo)nuclotide (nucleic acid) can hybridize with a RNA of a G protein coupled receptor protein gene. Synthesis or function of the RNA can be inhibited, or expression of a G protein coupled receptor protein gene can be regulated and controlled via interaction with a G protein coupled receptor protein related RNA. An (oligo)nuclotide complementary to a selected sequence of a G protein coupled receptor protein related RNA, and an (oligo)nuclotide which can specifically hybridize with a G protein coupled receptor protein related RNA are useful for regulating and controlling expression of a G protein coupled receptor protein gene in or outside the living body and also useful for treating or diagnosing diseases.

Term "corresponding" means to have homology with or complementary to a particular sequence of a nucleotide, a nucleotide sequence or a nucleic acid including a gene. "Corresponding" between a nucleotide, a nucleotide sequence or a nucleic acid and a peptide (protein) denotes an amino acid of a peptide (protein) under direction derived from a nucleotide (nucleic acid) sequence or a complementary sequence thereof. Although 5' terminal hairpin loop, 5' terminal 6 base pair repeat, 5' terminal untranslated region, polypeptide translation initiation codon, protein coding region, ORF translation initiation codon, 3'terminal untranslated region, 3'terminal palindrome region, and 3'terminal hairpin loop can be selected as a preferable target region, any region within a G protein conjugated type protein gene can be selected as a target.

Relationship between a nucleic acid of interest and an (oligo)nucleotide which is complementary to at least a part of a target region, and relationship between a subject and an (oligo)nucleotide which can hybridize with the subject can be said to be "antisense". As an antisense (oligo)nucleotide, there are a polydeoxynucleotide containing 2-deoxy-D-ribose, a polydeoxynucleotide containing D-ribose, an another type of polynucleotide which is N-glycoside of pyrine or pyrimidine base, or an another polymer having a non-nucleotide skeleton (for example, a commercially available protein nucleic acid and a synthetic sequence-specific nucleic acid polymer) or an another polymer containing a special linkage (provided that the polymer contains a nucleotide having arrangement permitting a phase pairing or base attachment found in a DNA or a RNA). They can be a double-stranded DNA, a single-stranded DNA, a double-stranded RNA, a single-stranded RNA, or a DNA:RNA hybrid, further may be an unmodified polynucleotide or an unmodified oligonucleotide, or further may have the known modification added, for example, may have a label which is known in the art, may have a cap added, may be methylated, may have 1 or more natural nucleotides substituted with an analogue, may have an intramolecular nucleotide modified, for example, may have non-charged linkage (for example, methylphosphonate, phosphotriester, phosphoramidate, carbamate and the like), may have a linkage having a charge or a sulfur-containing linkage (for example, phosphorothioate, phosphorodithioate and the like), for example, may have a side group such as a protein (nuclease, nuclease inhibitor, toxin, antibody, signal peptide, poly-L-lysine and the like) or a sugar (for example, monosaccharide and the like), may have an interchalant compound (for example, acridine, psoralen and the like), may have a chelating compound (for example, a metal, a metal having radioactivity, boron, an oxidative metal and the like), may have an alkylating agent, may have a modified linkage (for example, $\alpha$ anomer type nucleic acid). Here, "nucleoside", "nucleotide" and "nucleic acid" may not only contain a pyrine and a pyrimidine base but also an another heterocyclic base. Such the modification may contain a methylated purine and pyrimidine, an acylated purine and pyrimidine, or an another heterocycle. In a modified nucleoside and a modified nucleotide, a sugar part may be modified and, for example, 1 or more hydroxy groups may be substituted with a halogen or an aliphatic group, or may be converted into a functional group such as ether and amine.

An antisense nucleic acid of the present invention is a RNA, a DNA, or a modified nucleic acid. Embodiments of a modified nucleic acid are not limited to but include a sulfur derivative or a thiophosphate derivative of a nucleic acid, and a nucleic acid having resistance to degradation of polynucleosideamide or oligonucleosideamide. An antisense nucleic acid of the present invention can be preferably designed according to the following guideline. That is, an antisense nucleic acid is made stable in a cell, cell permeability of an antisense nucleic acid is enhanced, affinity for a target sense chain is made greater and, if it has toxicity, toxicity is made smaller.

A number of such the modifications are known in the art and are disclosed in J. Kawakami et al., Pharm Tech Japan, Vol. 8, pp.247 1992; Vol. 8, pp.395, 1992; S.T. Crooke et al. ed., Antisense Research and Applications, CRC Press, 1993.

An antisense nucleic acid of the present invention may be altered or may have a modified sugar, base or linkage, and can be supplied as a special form such as a microsphere, or may be applied to gene therapy, or may be given as an added form. As an antisense nucleic acid which is used as such an added form, there are a polycation such as polylysine which exerts so as to neutralize a charge of a phosphate skeleton, and a hydrophobic lipid (for example, phospholipid, cholesterol and the like) which increases uptake of a nucleic acid. As a preferable lipid to be added, there are cholesterol and its derivatives (for example, cholesteryl chloroformate, cholic acid and the like). They can be attached to a 3'terminal or a 5'terminal of a nucleic acid, can be attached via a base, a sugar or an intramolecular nucleoside linkage. As the other group, there is a group for capping which is specifically arranged at a 3'terminal or a 5'terminal of a nucleic acid and is for inhibiting degradation by nuclease such as exonuclease, RNase and the like. Examples of such the group for capping are not limited to but include protecting groups known in the art including glycols such as polyethylene glycol, tetraethylene glycol and the like.

The inhibitory activity of an antisense nucleic acid of the present invention can be examined using a transformant of the present invention, gene expressing system in and outside the living body of the present invention, or translation system for a protein in and outside the living body. The nucleic acid can be applied to cells by various method known per se.

A DNA encoding a partial peptide of the present invention may be any one as long as it has a nucleotide sequence encoding a partial peptide of the present invention. Further, the DNA may be any of a genomic DNA, a genomic DNA library, a cDNA derived from the aforementioned cells or tissues, a cDNA library derived from the aforementioned cells or tissues, or a synthetic DNA. A vector used for a library may be any of bacteriophage, plasmid, cosmid, and phagemide. In addition, a mRNA fraction is prepared from the aforementioned cells or tissues, which may be used to directly amplify by Reverse Transcriptase Polymerase Chain Reaction (hereinafter, abbreviated as RT-PCR method).

More particularly, as a DNA encoding a partial peptide of the present invention may be, for example, a DNA having a partial nucleotide sequence of a DNA which has a nucleotide sequence represented by SEQ ID No.:2, SEQ ID No.:4 or SEQ ID No.:6, or any DNA having a nucleotide sequence which hybridizes with a nucleotide sequence represented by SEQ ID No.:2, SEQ ID No.:4 or SEQ ID No.:6 under the high stringent conditions and having a partial nucleotide sequence of a DNA encoding a protein having substantially the same nature of activity (for example, ligand binding activity, signal information transmitting action and the like) as that of a protein peptide of the present invention is used.

As a DNA which can hybridize with a nucleotide sequence represented by SEQ ID No.:2, SEQ ID No.:4 or SEQ ID No.:6, a DNA having a nucleotide sequence having about 70% or more, preferably about 80% or more, more preferably about 90% or more, most preferably about 95% or more homology with a nucleotide sequence represented by SEQ ID No.:2, SEQ ID No.:4 or SEQ ID No.:6 is used.

For cloning a DNA fully encoding present protein or a partial peptide thereof (hereinafter, abbreviated as present protein), the DNA can be amplified by a PCR method using a synthetic DNA primer having a partial nucleotide sequence of present protein, or can be selected by hybridization of a DNA incorporated into a suitable vector with a labeled DNA fragment or synthetic DNA encoding a part of or an entire region of present protein. A hybridization method may be performed by a method described in, for example, Molecular Cloning 2nd (J. Sambrook et al., Cold Spring Harbor Lab. Press, 1989). In addition, when a commercially available library is used, it can be performed according to a method described in the attached specification.

Conversion of a nucleotide sequence of a DNA can be performed by the method known per se such as a Gupped duplex method or a Kunkel method or a similar method using Mutant™-G (Takara shuzo Co., Ltd.), Mutant™-K(Takara shuzo Co., Ltd.) or the like.

A DNA encoding a cloned protein can be used as it is, or used optionally by digesting with a restriction enzyme, or adding a linker, depending upon the purposes. The DNA may have ATG as a translation initiation codon at its 5'terminal, or TAA, TGA or TAG as a translation termination codon at its 3'terminal. These termination initiation codon and translation termination codon can be added using a suitable synthetic DNA adapter.

An expression vector for present protein can be prepared by, for example, (a) excising a DNA fragment of interest from a DNA encoding present protein, (b) ligating the DNA fragment to downstream of a promoter in a suitable expression vector.

As a vector, a plasmid derived from Escherichia coli (for example, pBR322, pBR325, pUC12, pUC13), a plasmid derived from Bacillus subtilis (for example, pUB110, pTP5, pC194), a plasmid derived from yeast (for example, pSH19, pSH15), bacteriophage such as γ phase, animal virus such as retrovirus, vaccinia virus, baculovirus, as well as pA1-11, pXT1, pRc/CMV, pRc/RSV, pcDNAI/Neo and the like are used.

As a promoter used in the present invention, any promoter which is suitable for a host used for expressing a gene may be used. For example, when an animal cell is used as a host, there are SRα-promoter, SV40 promoter, LTR promoter, CMV promoter, HSV-TK promoter and the like.

Among them, the use of CMV promoter and SRα promoter is preferable. When a host is a bacterium belonging to genus Escherichia, trp promoter, lac promoter, recA promoter, λPL promoter, lpp promoter and the like are preferable and, when a host is a bacterium belonging to genus Bacillus, SPO1 promoter, SPO2 promoter, penP promoter are preferable and, when a host is yeast, PHO5 promoter, PGK promoter, GAP promoter, ADH promoter and the like are preferable. When a host is an insect cell, polyhedron, P10 promoter and the like are preferable.

As an expression vector, a vector optionally containing enhancer, splicing signal, polyA addition signal, selectable marker, SV40 replication origin (hereinafter abbreviated as SV40ori in some cases) or the like may be used. As a selectable marker, for example, there are dihydrofolate reductase (hereinafter, abbreviated as dhfr in some cases) gene [methotrexate (MTX) resistant], ampicillim resistant gene (hereinafter, abbreviated as $Amp^r$ in some cases), neomycin resistant gene (hereinafter, abbreviated as Neo in some cases, G418 resistant) and the like. In particular, when dhfr gene is used as a selectable marker using CHO ($dhfr^-$) cell, a gene of interest can be selected also on a medium containing no thymidine.

In addition, as necessary, a signal sequence compatible for a host is added to a N-terminal of present protein. When a host is a bacterium belonging to genus Escherichia, PhoA • signal sequence, Omp A • signal sequence and the like can be utilized and, when a host is a bacterium belonging to genus Bacillus, α-amylase • signal sequence, subtilisin • signal sequence and the like can be utilized and, when a host is yeast, MFα • signal sequence, SUC2 • signal sequence and the like can be utilized and, when a host is an animal cell, insulin • signal sequence, α-interferon • signal sequence, antibody • signal sequence and the like can be utilized.

A vector containing a DNA encoding present protein thus constructed can be used to produce a transformant.

As a host, for example, a bacterium belonging to genus Escherichia, a bacterium belonging to genus Bacillus, yeast, insect cell, insect, animal cell and the like are used.

As an example of a bacterium belonging to genus Escherichia, Escherichia coli K12 • DH1 [Proc. Natl. Acad. Sci. USA), vol.60, 160 (1968), JM103 [Nucleic Acids Research), Vol.9, 309(1981), JA211 [Journal of Molecular Biology], vol.120, 517 (1978), HB101[Journal of Molecular Biology, vol.41, 459(1969)], C600 [Genetics, vol.39, 440(1954)] and the like are used.

As an example of a bacterium belonging to genus Bacillus, for example, Bacillus subtilis MI114 [Gene, vol.24, 255 (1983)], 207-21 [Journal of Biochemistry, vol.95, 87(1984)] and the like are used. As yeast, for example, Saccharomyces cerevisiae) AH22, $AH22R^-$, NA87-11A, DKD-5D, 20B-12, Schizosaccharomyces pombe) NCYC1913, NCYC2036, Pichia pastoris and the like are used.

As an insect cell, established cell derived from Barathra larva (Spodoptera frugiperda cell;Sf cell), MG1 cell derived from midgut of Trichoplusia ni, High Five™ cell derived from egg of Trichophusia ni, cell derived from Mamestra brassicae, cell derived from Estigmena acrea and the like are used. When a virus is BmNPV, established cell derived from silkworm (Bombyx mori N;BmN cell) and the like are used. As the Sf cell, for example, Sf9 cell (ATCC CRL1711), Sf21 cell (Vaughn, J.L. et al., In Vivo, 13, 213-217 (1977)) and the like are used.

As an insect, for example, silkworm larva and the like are used [Maeda et al., Nature, vol.315, 592(1985)].

As an animal cell, for example, monkey COS-7, V ero, Chinese hamster cell CHO (hereinafter, abbreviated as CHO cell), dhfr gene-deficient Chinese hamster cell CHO (hereinafter, abbreviated as CHO ($dhfr^-$) cell), mouse L cell, mouse AtT-20, mouse myeloma cell, rat GH3, human FL cell and the like are used.

In order to transform a bacterium belonging to genus Escherichia, for example, transformation can be performed according to a method described in Proc. Natl. Acad. Sci. USA, vol.69, 2110(1972) and Gene, vol.17, 107(1982). In order to transform a bacterium belonging to genus Bacillus, for example, transformation can be performed according to a method described in Molecular & General Genetics, vol.168, 111(1979).

In order to transform yeast, transformation can be performed according to a method described, for example, in Methods in Enzymology, vol.194, 182-187 (1991), Proc. Natl. Acad. Sci. USA, vol.75, 1929(1978) and the like.

In order to transform an insect cell or an insect, transformation can be performed according to a method described in Bio/Technology, 6, 47-55(1988) and the like.

In order to transform an animal cell, transformation can be performed according to a method described, for example, in Cell Technology, separate column 8, New Cell Technology Experiment Protocol, 263-267 (1995) (published by Shujunsha), Virology, vol.52, 456(1973) and the like.

Like this, a transformant transformed with an expression vector containing a DNA encoding a G protein conjugated protein can be obtained.

When a transformant, a host of which is a bacterium belonging to genus Escherichia or a bacterium belonging to genus Bacillus, is cultured, as a medium used for culturing, a liquid medium is suitable and carbon source, nitrogen source, inorganic substances and other necessary for growth of the transformant are contained therein. As carbon source, for example, there are glucose, dextrin, soluble starch, sucrose and the like. As nitrogen source, for example, there are inorganic and organic substances such as ammonium salts, nitrate salts, corn steep liquior, peptone, casein, broth extract, soy bean bran, potato extract solution and the like. As an inorganic substance, for example, there are calcium chloride, sodium dihydrogen phosphate, magnesium chloride and the like. In addition, yeast, vitamines, growth promoting factor and the like may be added. pH of a medium is desirably about 5-8.

As a medium when a bacterium belonging to Escherichia is cultured, for example, the M9 medium containing glucose and casamino acid [Miller, Journal of Experiments in Molecular Genetics, 431-433, Cold Spring Harbor Laboratory, New York, 1972] is preferable. In-order to allow a promoter to exert effectively as necessary, for example, a chemical such as 3β-indolyl acrylic acid can be added. When a host is a bacterium belonging to genus Escherichia, culturing is usually performed at about 15-43°C for about 3-24 hours and, if needed, airation and stirring may be added.

When a host is a bacterium belonging to genus Bacillus, culturing is usually carried out at about 30-40°C for about 6-24 hours and airation and stirring may be added as necessary.

When a transformant, a host of which is yeast, is cultured, as a medium, there are Burkholder minimum medium [Bostian, K.L.et al. (Proc. Natl. Acad. Sci. USA, vol.77, 4505(1980)] and the SD medium containing 0.5% casamino acid [Bitter, G.A. et al. (Proc. Natl. Acad. Sci. USA), vol.81, 5330(1984)]. pH of a medium is preferably adjusted to about 5-8. Culturing is usually performed at about 20-35°C for about 24-72 hours and airation and stirring are added as necessary.

When a transformant, a host of which is an insect cell or an insect, is cultured, as a medium, Grace's Insect Medium (Grace T.C.C., Nature, 195, 788 (1962)) to which an additive such as immobilized 10% bovine serum is appropriately added and the like are used. pH of a medium is preferably adjusted to about 6.2-6.4. Culturing is usually performed at about 27°C for 3-5 days and airation and stirring are added as necessary.

When a transformant, a host of which is an animal cell, is cultured, as a medium, MEM medium containing about 5-20% fetal bovine serum [Science, vol.122, 501 (1952)], DMEM medium [Virology, vol.8, 396(1959)], RPMI 1640 medium [The Journal of the American Medical Association, vol.199, 519(1967)], 199 medium [Proceedings of the Society for the Biological Medicine, vol.73, 1(1950)] and the like are used. pH is preferably about 6-8. Culturing is performed at about 30-40°C for 15-60 hours and airation and stirring are added as necessary.

Like this, a G protein coupled receptor protein of the present invention can be produced in a cell membrane of a transformant.

In order to purify and isolate present protein from the aforementioned culture, for example, purification and isolation can be performed according to the following method.

Upon extraction of present protein from cultured bacterium or cells, a method of collecting the bacterium or cell by the known method after culturing, suspending this in a suitable buffer, disrupting the bacterium or cell by lysozyme and/or freeze-melting, and obtaining a crude extract of a protein by centrifugation or filtration or the like is appropriately used. A protein denaturing agent such as urea and guanidine hydrochloride, or a surfactant such as TritonX-100™ may be contained in the buffer. When a protein is secreted in the culturing solution, after culturing is complete, the bacterium or cell and the supernatant are separated by the method known per se and the supernatant is collected.

Purification of the culturing supernatant thus obtained or a protein contained in the extract may be performed by combining the separating and purifying methods known per se. As the known separating and purifying methods, a method utilizing the solubility such as salting out and a solvent precipitation method, a method utilizing mainly a difference in a molecular weight such as dialysis, ultrafiltration, gel filtration and SDS-polyacrylamide electrophoresis, a method utilizing a difference in charge such as ion-exchange chromatography, a method utilizing the specific affinity such as affinity chromatography, a method utilizing a difference in hydrophobicity such as reverse phase high performance liquid chromatography, a method utilizing a difference in isoelectric point such as electrofocusing and the like are used.

When the protein thus obtained is obtained as a free material, it can be converted into a salt by the method known per se or a similar method and, conversely, when the protein is obtained as a salt, it can be converted into a free material or other salt by the method known per se or a similar method.

By acting a suitable protein modifying enzyme before or after purification, a protein produced by a transformant may be modified or a polypeptide may be partially removed therefrom. As a protein modifying enzyme, for example, trypsin, chymotrypsin, aluginineendopeptidase, proteinkinase, glucosidase and the like are used.

The activity of present protein or a salt thereof thus obtained can be measured by an experiment for binding with a labeled ligand, enzyme immunoassay and the like.

An antibody to present protein, a partial peptide thereof or a salt thereof may be any of polyclonal antibody and monoclonal antibody as long as it is an antibody which can recognize present protein, a partial peptide thereof or a salt thereof.

An antibody to present protein, a partial peptide thereof or a salt thereof (hereinafter, abbreviated as present protein or the like) can be prepared using the present protein or the like as an antigen according to the method for preparing an antibody or antiserum known per se.

[Preparation of a monoclonal antibody]

(a) Preparation of a monoclonal antibody producing cell

The present protein or the like is administered to a site of a mammal being capable of producing an antibody as it is or together with a carrier or a diluent. In order to enhance the antibody producing ability upon administration, Freund's complete adjuvant or Freund's incomplete adjuvant may be administered. Administration is usually conducted once per 2-6 weeks at a total of around 2-10 times. As a mammal to be used, although there are, for example, monkey, rabbit, dog, guinea pig, mouse, rat, sheep and goat, mouse and rat are preferably used.

Upon preparation of a monoclonal antibody producing cell, a monoclonal producing hybridoma can be prepared by selecting a warm blood animal immunized with an antigen, for example, an individual having recognized titer from mice, taking spleen or lymph node 2-5 days after final immunization, and fusing an antibody producing cell contained therein with a myeloma cell. Measurement of the antibody titer in antiserum can be performed, for example, by measuring the activity of a labeling agent bound to an antibody after the labeled present protein or the like described below is reacted with antiserum. The fusing operation can be performed, for example, by a method of Khler and Milstein [Nature, vol.256, pp.495 (1975)]. As a fusion promoting agent, for example, although there are polyethylene glycol (PEG), Sendai virus and the like, PEG is preferably used.

As a myeloma cell, although there are, for example, NS-1, P3U1, SP2/0 and the like, P3U1 is preferably used. A preferable ratio of the number of antibody producing cells (spleen cells) used and that of myeloma cells used is around 1:1-20:1. PEG (preferably PEG 1000-PEG 6000) is added at the concentration of around 10-80%. Cell fusion can be performed effectively by incubation at about 20-40°C, preferably about 30-37°C for about 1-10 minutes.

For screening a monoclonal antibody producing hybridoma, a variety of methods can be used. For example, there are a method of adding the hybridoma culturing supernatant to a solid phase on which the present protein or the like is adsorbed directly or together with a carrier (for example, microplate) and, then, adding an anti-immunoglobulin antibody (when a cell used in cell fusion is mouse, an anti-mouse immunoglubulin antibody is used) labeled with a radioisotope or an enzyme or protein A to detect a monoclonal antibody bound to a solid phase, a method of adding the hybridoma culturing supernatant to a solid phase on which anti-immunoglobulin antibody or a protein A is adsorbed and adding the present protein or the like labeled with a radioisotope or an enzyme to detect a monoclonal antibody bound to a solid phase and the like.

Although selection of a monoclonal antibody can be performed according to the method known per se or a similar method, it can be usually performed with a medium for an animal cell to which HAT (hypoxanthine, aminopterin, thymidine) is added. As a medium for selection and growth, any medium may be used as long as a hybridoma can be grown therein. For example, RPMI 1640 medium containing 1-20%, preferably 10-20% fetal bovine serum, GIT medium containing 1-10% fetal bovine serum (Wako Pure Chemical Industries, Ltd.) or serem-free medium for growing a hybridoma (SFM-101, Nissui Pharmaceutical Co., Ltd.) and the like can be used. A culturing temperature is usually 20-40°C, preferably about 37°C. A culturing time is usually 5 days to 3 weeks, preferably 1 to 2 weeks. Culturing can be usually performed under 5% carbonic acid gas. The antibody titer of the hybridoma culturing supernatant can be measured as in the measurement of the antibody titer of antiserum described above.

(b) Purification of a monoclonal antibody

Separation and purification of a monoclonal antibody can be usually performed according to a separation and purification method of an immunoglobulin [for example, salting out method, alcohol precipitation method, isoelectric focusing method, electrophoresis method, adsorption and desorption method by ion exchanging material (for example, DEAE), ultracentrifugation method, gel filtration method, a specific purifying method of taking only antibody with an active adsorbing agent such as antigen binding solid phase, protein A or protein G].

[Preparation of a polyclonal antibody]

The polyclonal antibody of the present invention can be prepared according to the method known per se or a similar method. For example, it can be prepared by making a complex of an immunological antigen (receptor protein and the like antigen) and a carrier protein, which is used to immunize a mammal as in the case of the aforementioned preparation of a monoclonal antibody, and taking a material containing an antibody to the present protein or the like from the immunized mammal to perform separation and purification of an antibody.

As a complex of an immunoantigen and a carrier protein which is used for immunizing a mammal, any of a kind of a carrier protein and a mixturing ratio of a carrier and a hapten may be used for cross-linking as long as an antibody can be effective to a hapten immunized by cross-linking with a carrier. For example, a method for coupling bovine serum alubmin, bovine thyroglobulin, keyhole limpet hemocyanin and the like with a hapten at a rate by weight of about 0.1-20, preferably about 1-5 relative to 1 of hapten is used.

In addition, for coupling hapten with a carrier, a variety of condensing agents can be used, and glutaraldehyde and carbodiimide, maleimide-active ester, active ester reagent containing a thiol group or a dithiobyridyl group can be used.

A condensation product can be administered to a site of a mammal which can produce an antibody as it is or together with a carrier or a diluent. In order to enhance the antibody producing ability upon administration, Freund's complete adjuvant or Freund's incomplete adjuvant can be administered. Administration can be usually performed once per about 2-6 weeks, at a total about 3-10 times.

A polyclonal antibody can be taken from blood, ascites and the like, preferably blood of a mammal immunized according to the above method.

The polyclonal titer in antiserum can be measured as in the aforementioned measurement of the antibody titer in serum. Separation and purification of a polyclonal antibody can be carried out according to separation and purification of immunogloblin as in the aforementioned separation and purification of a monoclonal antibody.

Present protein, a partial peptide thereof or a salt thereof can be used as (1) a method of determining a ligand for present protein, (2) obtaining of an antibody and antiserum, (3) construction of an expression system for a recombinant protein, (4) development of a receptor binding assay using the same expression system, and screening of medicine candidates, (5) implementation of drug design based on comparison with a ligand receptor having the structural similarity, (6) a reagent for making a probe and a PCR primer in gene therapy, (7) production of a transgenic animal, or (8) a drug for gene prophylaxis and therapy.

In particular, a compound which alters property of a ligand binding with a G protein-coupled receptor specific for human being or a mammal (for example, agonist, antagonist and the like) can be screened by using a receptor binding assay system using an expression system for a recombinant protein of the present invention, and the agonist and the antagonist can be used as an agent for preventing or treating various diseases.

The use of present protein, a partial peptide or a salt thereof (hereinafter, abbreviated as present protein or the like in some cases), a DNA encoding present protein or a partial peptide thereof (hereinafter, abbreviated as present DNA in some cases) and an antibody to the present invention or the like (hereinafter, abbreviated as present antibody in some cases) is explained more particularly.

(1) A method of determining a ligand (agonist) for present protein

Present protein or a salt thereof or a partial peptide of the present invention or a salt thereof are useful as a reagent for searching or determining a ligand (agonist) for present protein or a salt.

That is, the present invention provides a method for determining a ligand for present protein, which comprises contacting a test compound with present protein or a salt thereof or a partial peptide of the present invention or a salt thereof.

As a test compound, there are the known ligand (for example, angiotensin, bombesin, canabinoid, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES and the like), endothelin, enterogastrin, histamine, neurotensin, TRH, pancreatic polypeptide, galanin), subtype thereof or analogue thereof, known α-latrotoxin, neurexophilin, subtype thereof, or analogue thereof, and for example, tissues extract, and cell culture supernatant of human being or a mammal (for example, mouse, rat, pig, cow, sheep, monkey and the like) are used. For example, the tissue extract, cell culture supernatant or the like is added to present protein, fractionated while measuring the cell stimulating activity and, finally, a single ligand can be obtained. Inter alia, α-latrotoxicin, neurophilin, subtype thereof or an analogue thereof are a preferable ligand.

More particularly, a method for determining a ligand of the present invention is a method for determining a compound (for example, peptide, protein, non-peptide compound, synthetic compound, fermented product and the like) or a salt thereof having the cell stimulating activity (for example, activity of promoting or inhibiting arachidonic acid release, acetylcholine release, intracellular $Ca^{2+}$ release, intracellular cAMP production, intracellular cGMP production, inositol phosphate production, intracellular potential variation, phosphorylation of an intracellular protein, c-fos activation, decrease in pH and the like) by binding to present protein using present protein, a partial peptide thereof or a salt thereof, or using the receptor binding assay system using a constructed expression system for a recombinant protein.

A method for determining a ligand of the present invention is characterized in that an amount of a test compound bound to present protein or a partial peptide thereof when a test compound is contacted with the protein or the partial peptide.

More particularly, the present invention provides:

(1) a method for determining the ligand for present protein or a salt thereof, which comprises measuring an amount of a labeled test compound bound to present protein or a salt thereof or a partial peptide of the present invention or a salt thereof when a labeled compound is contacted with the protein or the salt or the partial peptide or the salt,
(2) a method for determining a ligand for present protein or a salt thereof, which comprises measuring an amount of a labeled test compound bound to a cell containing present protein or a membrane fraction of a cell when a labeled test compound is contacted with the cell or the membrane fraction,
(3) a method for determining a ligand for present protein, which comprises measuring an amount of a labeled test compound bound to a protein expressed on a cell membrane by culturing a transformant containing a DNA encoding present protein when a labeled test compound is contacted with the protein or a salt thereof,
(4) a method for determining a ligand for present protein or a salt thereof, which comprises measuring the cell stimulation activity (for example, the activity of promoting or inhibiting arachidonic acid release, acetylcholine release, intracellular $Ca^{2+}$ release, intracellular cAMP production, intracellular cGMP production, inositol phosphate production, cell membrane potential variation, phosphorylation of intracellular protein, activation of c-fos, decrease in pH and the like) via a protein when a test compound is contacted with a cell containing present protein,

(5) a method for determining a ligand for present protein or a salt thereof, which comprises measuring the cell stimulation activity (for example, the activity of promoting or inhibiting arachidonic acid release, acetylcholine release, intracellular $Ca^{2+}$ release, intracellular cAMP production, intracellular cGMP production, inositol phosphate production, cell membrane potential variation, phosphorylation of intracellular protein, activation of c-fos, decrease in pH and the like) via a protein when a test compound is contacted with a protein expressed on a cell membrane by culturing a transformant containing a DNA encoding present protein.

In particular, it is preferable that, after the aforementioned tests, (1)-(3) are conducted and the binding of a test compound with present protein is confirmed, the aforementioned (4)-(5) are conducted.

First, although a protein used for a method for determining a ligand may be any protein as long as it contains the aforementioned protein of the present invention or the partial peptide of the present invention, a protein which is expressed using an animal cell is suitable.

In order to prepare present protein, the aforementioned expression method is used and it is preferable that preparation is conducted by expressing a DNA encoding the protein in a mammal cell or an insect cell. As a DNA fragment encoding a protein part of interest, a complementary DNA is usually used and the fragment is not necessarily limited to it. For example, a gene fragment and a synthetic DNA may be used. In order to introduce a DNA fragment encoding present protein into a host animal cell and express it effectively, it is preferable that the DNA fragment is incorporated downstream of polyhedron promoter of nuclear polyhedrosis virus (NPV) belonging to genus baculovirus, promoter derived from SV40, promoter of retrovirus, metallothionein promoter, human heat shock promoter, cytomegalovirus promoter, SRα promoter and the like. Examination of an amount and quality of expressed receptor can be conducted by the method known per se. For example, examination can be conducted according to a method described in the literature [Nambi, P. et al., The Journal of Biological Chemistry, J. Biol. Chem., vol.267, 19555-19559, 1992).

Therefore, as one containing present protein, a partial peptide thereof or a salt thereof, it may be a protein purified according to the method known per se, a partial peptide thereof or a salt thereof, or a cell containing the protein or its cell membrane fraction may be used.

In a method for determining a ligand, when a cell containing present protein is used, the cell may be fixed by glutaraldehyde, formalin or the like. A fixing method can be conducted according to the method known per se.

A cell containing present protein refers to a host cell which expressed present protein. As the host cell, Escherichia coli, Bacillus subtilis, yeast, insect cell, animal cell and the like are used.

A cell membrane fraction refers to a fraction in which a cell membrane obtained by the method known per se after a cell is ruptured is contained at a large amount. As a method for repturing a cell, there are a method of squeezing a cell with a Potter-Elvehjem type homogenizer, rupture with Waring blender or Polytron (manufactured by Kinematica), rupture by ultrasound, rupture by ejecting a cell through a thin nozzle while pressing with a French press and the like. For fractionating a cell membrane, a fractionating method by centrifugation such as a fractionation centrifugation method and a density gradient centrifugation method are mainly used. For example, a ruptured cell solution is centrifuged at a low speed (500 rpm-3000 rpm) for a shorter period of time (usually, about 1 minute to 10 minutes), a supernatant is further centrifuged at a higher speed (15000 rpm-30000 rpm) usually for 30 minutes to 2 hours and the resulting precipitates are used as a membrane fraction. An expressed protein and a membrane component such as phospholipid and membrane protein derived from a cell are contained in the membrane fraction at a large amount.

An amount of a cell containing the protein or a protein in the membrane fraction is preferably $10^3$-$10^8$ molecules per 1 cell, suitably $10^5$-$10^7$ molecules. As an expression amount grows larger, the ligand binding activity (specific activity) is increased and, thereby, not only construction of high sensitive screening system becomes possible but also a large amount of samples can be measured at the same lot.

In order to perform the aforementioned methods (1)-(3) for determining a ligand for present protein or a salt thereof, a suitable protein fraction and a labeled test compound are necessary.

As a protein fraction, a natural receptor protein fraction or a recombinant receptor fraction having the equivalent activity thereto are desirable. Here, equivalent activity denotes equivalent ligand binding activity, signal information transmitting action and the like.

As a labeled test compound, angiotensin, bombesin, canabinoid, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene related peptide), leukotriene, pancreastatin, prostaglandin, thromboxane, adenosine, adrenaline, α and β-chemokine (for example, IL8, GROα, GROβ, GROγ, NAP-2, ENA-78, PF4, IP10, GCP-2, MCP-1, HC14, MCP-3, I-309, MIP1α, MIP-1β, RANTES and the like), endothelin, enterogastrin, histamine, neurotensin, TRH, pancreatic polypeptide, galanin, α-latrotoxin, neurexophilin, subtype thereof, or analogue thereof labeled with [$^3$H], [$^{125}$I], [$^{14}$C], [$^{35}$S] or the like are suitable. Among them, α-latrotoxin, neuroxophilin, subtype thereof or an analogue thereof are preferably used.

More particularly, in order to perform a method for determining a ligand for present protein or a salt thereof, an authentic receptor is first prepared by suspending a cell containing present protein or a membrane fraction of the cell in a buffer suitable for determination. As a buffer, any buffer such as phosphate buffer having pH 4-10 (desirably, pH6-8) and Tris-HCl buffer may be used as long as it dose not inhibit binding of a ligand with present protein. In addition, in order to reduce the non-specific binding, surfactants such as CHAPS, Tween-80™ (Kao-Atlas), digitonin and deoxycholate, and proteins such as serum albumin and gelatin may be added to a buffer. Further, in order to suppress degradation of lipase or a ligand by protease, protease inhibiting agents such as PMSF, leupeptin, E-64 (manufactured by Peptide Laboratory) and pepstatin may be added thereto. A test compound labeled with [$^3$H], [$^{125}$I], [$^{14}$C], [$^{35}$S] or the like at a constant amount (5000 cpm-500000 cpm) is present in 0.01 ml-10 ml of a solution of the receptor. In order to know an amount of non-specific binding (NBS), a reaction tube to which a large excess unlabeled test compound is added is prepared. A reaction is performed at 0°C to 50°C, desirably at about 4°C to 37°C for about 20 minutes to 24 hours, desirably for about 30 minutes to 3 hours. After reaction, the reaction is filtered with a glass fiber filter or the like, washed with a suitable amount of a buffer, and the radioactivity remaining on a glass fiber filter is measured with a liquid scintillation counter or a γ-counter. A compound having the count (non-specific binding amount (NSB) is subtracted from whole binding amount (B)) (B-NSB) over 0 cpm can be a ligand (an agonist) for present protein or a salt thereof.

In order to perform the aforementioned methods (4)-(5) for determining a ligand for present protein or a salt thereof, the cell stimulating activity (for example, activity of promoting or inhibiting arachidonic acid release, acetylcholine release, intracellular $Ca^{2+}$ release, intracellular cAMP production, intracellular cGMP production, inositol phosphate production, intracellular potential variation, phosphorylation of an intracellular protein, c-fos activation, decrease in pH and the like) via the protein can be measured using the known protein or a commercially available measuring kit. More particularly, first, a cell containing present protein is cultured on a multiwell-plate or the like. Upon implementation of ligand determination, a test compound and the like are added, incubated for a certain time, the cell is extracted or the supernatant is recovered to quantitate the resulting products according to respective methods. When the production of a substance [for example, arachidonic acid and the like] which is an index for the cell stimulating activity is difficult to be detected due to degradation of a degrading enzyme contained in the cell, an inhibiting agent for the degrading enzyme may be added to perform an assay. In addition, regarding the activity of cAMP production suppression, it can be detected as production inhibiting action for a cell for which fundamental producing amount has been increased with forscholin.

A kit for determining a ligand for present protein or a salt thereof contains present protein or a salt thereof, a partial peptide of the present invention or a salt thereof, a cell containing present protein, or a membrane fraction of a cell containing present protein.

As an example of a kit for determining a ligand of the present invention, there are the following ones:

1. A reagent for determining a ligand

(1) A buffer for measurement and a buffer for washing

0.05% bovine serum albumin (manufactured by Sigma) is added to Hank's Balanced Salt Solution (manufactured by Gibco)

Filter-sterilized with a filter having a pore size 0.45 μm, which may be stored at 4°C or prepared upon use.

(2) An authentic G protein-coupled receptor protein

CHO cell in which present protein is expressed is subcultured on a 12-well plate at $5\times10^5$ cells/well, and cultured at 37°C, in 5% $CO_2$ and 95% air.

(3) A labeled test compound

A compound labeled with commercially available [$^3$H], [$^{125}$I], [$^{14}$C], [$^{35}$S] or the like, or labeled by a suitable method.

An aqueous solution is stored at 4°C or -20°C and diluted with a measuring buffer upon use. A test compound which shows the poor water-solubility is dissolved in dimethylformamide, DMSO, methanol or the like.

(4) Non-labeled test compound

Prepared to 100-1000 fold higher concentration as in a labeled compound.

2. A detecting method

(1) CHO cell, expressing present protein, which was cultured on a 12-well plate for tissue culturing is washed twice with a 1 ml of a measuring buffer, and a 490 μl of a measuring buffer is added to each well.
(2) 5 μl of a labeled test compound is added, which is reacted at room temperature for 1 hour. In order to known an amount of non-specific binding, 5 μl of a non-labeled test compound is added.
(3) The reaction solution is removed and washed with 1 ml of a washing buffer three times. A labeled test compound bound to a cell is dissolved with 0.2N NaOH-1% SDS, and mixed with 4 ml of liquid scintillator A (manufactured by Wako Pure Chemical Industries, Ltd.).
(4) The radioactivity is measured using a liquid scintillation counter (manufactured by Beckmann).

As a ligand which can bind to present protein or a salt thereof, there are substances which are specifically present, for example, in brain, pituitary gland, pancreas or the like. More particularly, angiotensin, bombesin, canabinoid, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene related peptide), leukotriene, pancreastatin, prostaglandin, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES and the like), endothelin, enterogastrin, histamine, neurotensin, TRH, pancreatic polypeptide, galanin, α-latrotoxin, neurexophilin, subtype thereof, or analogue thereof are used. Inter alia, α-latrotoxin, neurexophilin, subtype thereof, or analogue thereof are preferably used.

(2) An agent for preventing or treating present protein deficient-disease

In the aforementioned method (1), if a ligand for present protein is revealed, the present protein or a DNA encoding the present protein can be used as a medicine such as an agent for preventing or treating present protein deficient-disease.

For example, regarding a patient in which physiological action of a ligand is not expected due to decrease in present protein in the living body, an amount of a protein in the living body of a patient can be increased and an action of a ligand can be sufficiently exerted by (1) administering present protein to a patient to supplement an amount of the protein, or (2) (a) administering a DNA encoding present protein to the patient to express it, or (b) inserting a DNA encoding present protein to express it, which is transplanted into the patient. Therefore, a DNA encoding present protein is useful as a safe and low-toxic medicine for preventing or treating present protein deficient-disease.

When a DNA encoding present protein (hereinafter, abbreviated as present DNA in some cases) is used as the aforementioned preventing or treating agent, present DNA can be used according to the conventional method as it is or by inserting into a suitable vector such as retrovirus vector, adenovirus vector, adenovirus associated virus vector or the like. Present DNA can be administrated as it is or together with an adjuvant for promoting uptake by a gene gun or a catheter such as a hydrocatheter.

For example, present DNA can be orally administrated as a tablet optionally coated with a sugar coating, capsulate, elixir or microcapsule and the like, or parenterally administrated in the form of a sterile solution with water or other pharmaceutically acceptable solution or an injectable such as suspension. For example, pharmaceutical preparations can be make by mixing present DNA with a carrier, a flavor, an excipient, a vehicle, a preservative, a stabilizer or a binding agent into an unit dosage form required for art-recognized preparations. An amount of an ingredient in these preparations is adjusted such that a suitable dose can be obtained in a designated range.

As an additive which can be mixed into a tablet, a capsulate or the like, for example, a binding agent such as gelatin, corn starch, tragacanth and gum arabic, an excipient such as crystalline cellulose, a swelling agent such as corn starch, gelatin and alginic acid, a lubricating agen such as magnesium stearate, a sweetening agent such as sucrose, lactose and saccharin, a flavor such as peppermint, an alamono oil and cherry are used. When a preparation unit form is a capsule, a liquid carrier such as a lipid can be contained in the aforementioned type material. A sterile composition for injection can be formulated according to the conventional pharmacy by such as dissolving or suspending an active substance in a vehicle such as water for injection, a natural vegetable oil such as a sesami oil and a palm oil. As an aqueous solution for injection, for example, an isotonic (for example, D-sorbitol, D-mannitol, sodium chloride or the like) containing, for example, a physiological saline, glucose or other adjuvant and the like are used, and may be used together with a suitable solubilizer, for example, an alcohol (for example, ethanol), a polyalcohol (for example, propylene glycol, polyethylene glycol), nonionic surfactant (for example, polysorbate 80 (TM), HCO-50) or the like. As an oily solution, for example, a sesame oil and a soybean oil are used, and may be used together with benzyl benzoate, benzylalcohol or the like.

In addition, the aforementioned preventing or treating agent may be incorporated with, for example, a buffer (for example, a phosphate buffer, a sodium acetate buffer), a soothing agent (for example, benzalkonium chloride, procaine hydrochloride and the like), a stabilizing agent (for example, human serum alubmin, polyethylene glycol and the like), a preservative (for example, benzylalcohol, phenol and the like), and an antioxidant. A prepared injectable is usually filled into a suitable ampul.

Since pharmaceutical preparations thus obtained are safe and low toxic, and can be administered to, for example, human being or a mammal (for example, rat, rabbit, sheep, pig, cow, cat, dog, monkey).

A dose of present protein or DNA is different depending upon an administration subject, a subject organ, symptom and an administration method and, in the case of oral administration, the dose is generally about 0.1 mg-100 mg, preferably about 1.0-50 mg, more preferably about 1.0-20 mg per day, in an adult (60 kg). When parentally administrated, one time dose is different depending upon an administration subject, a subject organ, symptom and an administration method and, in the form of injectable, it is usually advantageous to administer about 0.01-30 mg, preferably about 0.1-20 mg, more preferably about 0.1-10 mg by intravenous injection per day, in an adult (60 kg). In other animals, an amount calculated per 60 kg can be administrated.

(3) An agent for diagnosing a gene

Since abnormality (gene abnormality) of a DNA or a mRNA encoding present protein in human being or a mammal (for example, rat, rabbit, sheep, pig, cow, cat, dog, monkey and the like) can be detected by using present DNA as a probe, for example, the present DNA is useful as an agent for diagnosing a gene such as damage of the DNA or the mRNA, mutation or decrease in expression, increase in excess expression of the DNA or the mRNA.

The aforementioned gene diagnosis using present DNA can be conducted by, for example, northern hybridization and a PCR-SSCP method which are known per se (Genomics, vol. 5, 874-879 (1989), Proceedings of the National Academy of Sciences of the United States of America), vol.86, pp.2766-2770 (1989)).

(4) A method for quantitating a ligand for present protein

Since present protein or the like has the binding property to a ligand, the concentration of a ligand in the living body can be quantitated with high sensitivity.

A quantitating method of the present invention can be used by combining with, for example, a competition method. That is, the concentration of a ligand in a specimen by contacting a specimen with present protein or the like. More particularly, for example, it can be used according to a method described in the following (i) or (ii) or a similar method.

(i) "Radioimmuno assay" edited by Hiroshi Irie (Kodansha, published in 1973)
(ii) "A Sequel to the Radioimmuno assay" edited by Hiroshi Irie (Kodansha, published in 1978)

(5) A method for screening a compound which alters property of ligand binding with present protein

By using the receptor binding assay by using present protein or the like, or by constructing an expression system for a recombinant protein and using the expression system, a compound which alter property of a ligand binding with present protein or the like (for example, peptide, protein, non-peptide compound, synthetic compound, fermented product and the like) or a salt thereof can be effectively screened.

(a) a compound having the cell stimulating activity (for example, activity of promoting or suppressing arachidonic acid release, acetylcholin release, intracellular $Ca^{2+}$ release, intracellular cAMP production, intracellular cGMP production, inositol phosphate production, cell membrane potential variation, phosphorylation of intracellular protein, activation of c-fos and decrease in pH) via a G protein-coupled receptor (so called agonist to present protein), (b) a compound having no such the cell stimulating activity (so called antagonist to present protein), (c) a compound which enhances binding force of a ligand with present protein, and (d) a compound which decreases binding force of a ligand with present protein are contained in such the compound (it is preferred that the above (a) compound is screened by the aforementioned method for determining a ligand).

That is, the present invention provides a method for screening a compound which alters property of a ligand with present protein, a partial peptide thereof or a salt thereof, which comprises comparing (i) the case where present protein, a partial peptide thereof or a salt thereof and a ligand are contacted, and (ii) the case where present protein, a partial peptide thereof or a salt thereof and a ligand and a test compound are contacted.

In a method for screening of the present invention is characterized in that, for example, an amount of a ligand to bind with the protein or the like and cell stimulating activity in the cases (i) and (ii) are measured and compared.

More particularly, the present invention provides:

(1) a method for screening a compound which alters property of a ligand binding with present protein or the like or a salt thereof, which comprises measuring and comparing an amount of a labeled ligand to bind to present protein or the like in the case where a labeled ligand is contacted with present protein or the like, and the case where a labeled ligand and a test compound are contacted with present protein or the like,

(2) a method for screening a compound which alters property of a ligand binding with present protein or the like or a salt thereof, which comprises measuring and comparing an amount of a labeled ligand to bind to a cell containing present protein or the like or a membrane fraction of the cell in the case where a labeled ligand is contacted with the cell or the membrane fraction, and the case where a labeled ligand and a test compound are contacted with the cell or the membrane fraction,

(3) a method for screening a compound which alters property of a ligand binding with present protein or the like or a salt thereof, which comprises measuring and comparing an amount of a labeled ligand to bind to protein or the like expressed on a cell membrane by culturing a transformant containing present DNA in the case where a labeled ligand is contacted with the protein or the like, and the case where a labeled ligand and a test compound are contacted with the protein or the like,

(4) a method for screening a compound which alters property of a ligand binding with present protein or the like, which comprises measuring and comparing cell stimulating activity (for example, activity of promoting or suppressing arachidonic acid release, acetylcholin release, intracellular $Ca^{2+}$ release, intracellular cAMP production, intracellular cGMP production, inositol phosphate production, cell membrane potential variation, phosphorylation of intracellular protein, activation of c-fos and decrease in pH) via a receptor in the case where a compound which activates present protein or the like (for example, ligand for present protein or the like) is contacted with a cell containing present protein or the like, and the case where a compound which activates present protein or the like and a test compound are contacted with a cell containing present protein or the like, and

(5) a method for screening a compound which alters property of a ligand binding with present protein or the like, which comprises measuring and comparing cell stimulating activity (for example, activity of promoting or suppressing arachidonic acid release, acetylcholin release, intracellular $Ca^{2+}$ release, intracellular cAMP production, intracellular cGMP production, inositol phosphate production, cell membrane potential variation, phosphorylation of intracellular protein, activation of c-fos and decrease in pH) via a receptor in the case where a compound which activates present protein or the like (for example, ligand for present protein or the like) is contacted with present protein or the like expressed on a cell membrane by culturing a transformant containing present DNA, and the case where a compound which activates present protein or the like and a test compound are contacted with present protein or the like expressed on a cell membrane by culturing a transformant containing present DNA.

Before present protein or the like is obtained, when a G protein-coupled receptor agonist or antagonist is screened, a test was necessary in which a cell, a tissue or its cell membrane fraction which contains a G protein-coupled receptor agonist or antagonist is first used to obtain candidate compounds (first screening) and, thereafter, whether candidate compounds actually inhibit binding of a human G protein-coupled receptor protein and a ligand is confirmed (secondary screening). When a cell, a tissue or a cell membrane fraction is used as it is, it was difficult to actually screen an agonist or an antagonist for a receptor protein of interest due to the presence of other receptors.

However, for example, the use of a human-derived protein of the present invention excludes the necessity of first screening and a compound which inhibits binding of a ligand and a G protein-coupled receptor protein can be effectively screened. Further, whether a screened compound is an agonist or an antagonist can be simply evaluated.

A method for screening of the present invention is explained more particularly.

First, as present protein or the like used in a method for screening of the present invention, any protein can be used as long as it contains the aforementioned present protein or the like, although a cell membrane fraction of an organ of a mammal containing present protein or the like is suitable. However, since a human-derived organ is obtained with very difficulty, a human-derived receptor protein or the like which was expressed at a large amount using a recombinant is suitable for use in screening.

For preparing present protein or the like, the aforementioned method is used, although it is preferred that present DNA is expressed in a mammal cell or an insect cell. As a DNA fragment coding a protein portion of interest, a complementary DNA is used, being not limiting. For example, a gene fragment and a synthetic DNA may be used. In order to introduce a DNA fragment encoding present protein and express it effectively, the DNA fragment is preferably incorporated downstream of polyhedron promoter of nuclear polyhedrosis virus (NPV) belonging to genus Bacurovirus, a host of which is an insect, SV40-derived promoter, promoter of retrovirus, metallothionein promoter, human heat shock promoter, cytomegalovirus promoter and SRα promoter. Examination of an amount and quality of expressed receptor can be performed by the method known per se. For example, it can be performed according to a method described in the literature [Nambi, P. et al., The Journal of Biological Chemistry, J. Biol. Chem., vol.267, pp.19555-19559, 1992].

Therefore, in a method for screening of the present invention, as one containing present protein or the like, protein or the like which was purified by the method known per se may be used, or a cell containing the protein or the like may be used, or a membrane fraction of a cell containing the protein or the like may be used.

In a method for screening of the present invention, when a cell containing present protein or the like is used, the cell may be fixed with glutaraldehyde, formalin or the like. Fixation can be carried out according to the method known per se.

A cell containing present protein or the like refers to a host cell which expressed the protein or the like and, as a host cell, Escherichia coli, Bacillus subtilis, yeast, insect cell and animal cell are preferable.

A cell membrane fraction refers to a fraction containing a cell membrane obtained according to a method known per se at a large amount after a cell is ruptured. As a method for repturing a cell, there are a method of squeezing a cell with a Potter-Elvehjem type homogenizer, rupture with Waring blender or Polytron (manufactured by Kinematica), rupture by ultrasound, rupture by ejecting a cell through a thin nozzle while pressing with a French press and the like. For fractionating a cell membrane, a fractionating method by centrifugation such as a fractionation centrifugation method and a density gradient centrifugation method are mainly used. For example, a ruptured cell solution is centrifuged at a low speed (500 rpm-3000 rpm) for a shorter period of time (usually, about 1 minute to 10 minutes), a supernatant is further centrifuged at a higher speed (15000 rpm-30000 rpm) usually for 30 minutes to 2 hours and the resulting precipitates are used as a membrane fraction. An expressed protein and a membrane component such as phospholipid and membrane protein derived from a cell are contained in the membrane fraction at a large amount.

An amount of a cell containing the protein or the protein in the membrane fraction is preferably $10^3$-$10^8$ molecules per 1 cell, suitably $10^5$-$10^7$ molecules. As an expression amount grows larger, the ligand binding activity (specific activity) is increased and, thereby, not only construction of high sensitive screening system becomes possible but also a large amount of samples can be measured at the same lot.

In order to perform the aforementioned methods (1)-(3) for screening a compound which alters property of a ligand binding with present protein or the like, for example, a suitable protein fraction and a labeled test compound are necessary.

As a protein fraction, a natural receptor protein fraction or a recombinant receptor fraction having the equivalent activity thereto are desirable. Here, equivalent activity denotes equivalent ligand binding activity, signal information transmitting action and the like.

As a labeled test compound, a labeled ligand and a labeled ligand analogue compound are used. For example, a ligand labeled with [$^3$H], [$^{125}$I], [$^{14}$C] or [$^{35}$S] is used.

More particularly, in order to perform a method for screening a compound which alters property of a ligand binding with present protein or the like, an authentic protein is first prepared by suspending a cell containing present protein or the like or a membrane fraction of the cell in a buffer suitable for screening. As a buffer, any buffer such as phosphate buffer having pH 4-10 (desirably, pH 6-8) and Tris-HCl buffer may be used as long as it dose not inhibit binding of a ligand with a protein. In addition, in order to reduce the non-specific binding, surfactants such as CHAPS, Tween-80™ (Kao-Atlas), digitonin and deoxycholate may be added to a buffer. Further, in order to suppress degradation of a receptor or a ligand by protease, protease inhibiting agents such as PMSF, leupeptin, E-64 (manufactured by Peptide Laboratory) and pepstatin may be added thereto. A labeled ligand is added to 0.01 ml-10 ml of a solution of the receptor at a certain amount (5000 cpm-500000 cpm) and, at the same time, a test compound is present at $10^{-4}$M-$10^{-10}$M. In order to know an amount of non-specific binding (NBS), a reaction tube to which a large excess unlabeled ligand is added is prepared. A reaction is performed at 0°C to 50°C, desirably at about 4°C to 37°C for about 20 minutes to 24 hours, desirably for about 30 minutes to 3 hours. After reaction, the reaction is filtered with a glass fiber filter or the like, washed with a suitable amount of a buffer, and the radioactivity remaining on a glass fiber filter is measured with a liquid scintillation counter or a γ-counter. A test compound having nonspecific binding amount (B-NSB) (wherein a count (B0-NSB) obtained by subtracting non-specific binding amount (NSB) from a count (B0) of no competing substance) can be selected as a candidate compound having the competition inhibiting ability.

In order to perform the aforementioned methods (4)-(5) for screening a compound which alters property of a ligand binding with present protein or the like, for example, the cell stimulating activity (for example, activity of promoting or inhibiting arachidonic acid release, acetylcholine release, intracellular Ca release, intracellular cAMP production, intracellular cGMP production, inositol phosphate production, intracellular potential variation, phosphorylation of an intracellular protein, c-fos activation, decrease in pH and the like) via a protein can be measured using the known method or a commercially available measuring kit.

More particularly, first, a cell containing present protein or the like is cultured on a multiwell-plate or the like.

Upon implementation of screening medium is freshly prepared or buffer is changed to the proper one which does not denote toxicity for cell beforehand, a test compound and the like are added, incubated for a certain time, the cell is extracted or the supernatant is recovered to quantitate the resulting products according to respective methods. When the production of a substance (for example, arachidonic acid and the like) which is an index for the cell stimulating activity is difficult to be detected due to a degrading enzyme contained in the cell, an inhibiting agent for the degrading enzyme may be added to perform an assay. In addition, regarding the activity of cAMP production suppression, it can be detected as production inhibiting action for a cell for which fundamental producing amount has been increased with forscholin.

In order to perform screening by measuring the cell stimulating activity, a cell which expressed a suitable protein is necessary. As a cell which expressed present protein or the like, a cell strain containing natural type present protein or the like, and a cell strain which expressed the aforementioned recombinant protein or the like are desirable.

As a test compound, for example, a peptide, a protein, a non-peptide compound, a synthetic compound, a fermented product, a cell extract solution, a plant extract solution, and an animal tissue extract solution are used, and these compounds may be a novel compound or the known compound.

A kit for screening a compound which alters property of a ligand binding with present protein or the like contains present protein or the like, a cell containing present protein or the like, or a membrane fraction of a cell containing present protein or the like.

As an example of a kit for screening of the present invention, there are the following ones:

1. A reagent for screening

(1) A buffer for measurement and a buffer for washing

0.05% bovine serum albumin (manufactured by Sigma) is added to Hanks' Balanced Salt Solution (manufactured by Gibco).

Filter-sterilized with a filter having a pore size of 0.45 μm, which may be stored at 4°C or prepared upon use.

(2) An authentic G protein-coupled receptor

CHO cell in which a protein of the present invention is expressed is subcultured on a 12-well plate at $5 \times 10^5$ cells/well, and cultured at 37°C, in 5% $CO_2$ and 95% air for two days.

(3) A labeled ligand

A ligand labeled with commercially available [$^3$H], [$^{125}$I], [$^{14}$C], [$^{35}$S] or the like. An aqueous solution is stored at 4°C or -20°C and diluted with a measuring buffer upon use.

(4) A standard solution of ligand

A ligand is dissolved to be 1 Mm with PBS which obtained 0.1 % bovine serum albumin (manufactured by Sigma) and stored at -20°C

2. A measuring method

(1) CHO cell, expressing present protein, which was cultured on a 12-well plate for tissue culturing is washed twice with a 1 ml of a measuring buffer, and a 490 μl of a measuring buffer is added to each well.

(2) After 5 μl of a $10^{-3}$-$10^{-10}$M test compound is added, 5 μl of a labeled ligand is added, which is reacted at room temperature for 1 hour. In order to know an amount of non-specific binding, 5 μl of a $10^{-3}$M ligand is added instead of a test compound.

(3) The reaction solution is removed and washed with 1 ml of a washing buffer three times. A labeled ligand bound to a cell is dissolved with 0.2N NaOH-1% SDS, and mixed with 4 ml of liquid scintillator A (manufactured by Wakojunyaku).

(4) The radioactivity is measured using a liquid scintillation counter (manufactured by Beckmann) and Percent Maximum Binding (PMB) is obtained according to the following equation [Mathematic 1]

$$PMB=[(B-NSB)/(B0-NSB)]\times100 \qquad \text{[Mathematic 1]}$$

PMB: Percent Maximum Binding
B: value when a specimen is added
NSB: Nonspecific Binding (non-specific binding amount)
B0: Maximum binding amount

A compound obtained by using a method for screening or a kit for screening of the present invention or a salt thereof is a compound which alters property of a ligand binding with present protein or the like. More particularly, there are (a) a compound having the cell stimulating activity (for example, activity of promoting or suppressing arachidonic-acid release, acetylcholin release, intracellular $Ca^{2+}$ release, intracellular cAMP production, intracellular cGMP production, inositol phosphate production, cell membrane potential variation, phosphorylation of intracellular protein, activation of c-fos and decrease in pH) via a G protein-coupled receptor (so called agonist to present protein), (b) a compound having no such a cell stimulating activity (so called antagonist to present protein), (c) a compound which enhances binding force of a ligand with present G protein-coupled receptor, and (d) a compound which decreases binding force of a ligand with present G-protein conjugated type protein.
As the compound, there are a peptide, a protein, a non-peptide compound, a synthetic compound and a fermented product, and these compounds may be a novel compound or the known compound.
Since an agonist to present protein or the like has the similar physiological activity to that of a ligand for present protein or the like, it is useful as a safe and low-toxic drug depending upon the ligand activity.
Since an antagonist to present protein or the like has the similar physiological activity to that of a ligand for present protein or the like, it is useful as a safe and low-toxic drug which inhibits the ligand activity.
A compound which enhances the binding force of a ligand binding with present protein is useful as a safe and low-toxic drug for enhancing the physiological activity harbored by a ligand for present protein or the like.
A compound which decreases the binding force of a ligand binding with present protein is useful as a safe and low-toxic drug for decreasing the physiological activity harbored by a ligand for present protein or the like.
A compound obtained by a screening method or a kit for screening of the present invention or a salt thereof can be used as the aforementioned pharmaceutical composition according to the conventional method. For example, it can be formulated into a tablet, a capsulate, an elixir, a microcapsulate, a sterile solution or a suspension solution as in the aforementioned drug containing present DNA.
Since pharmaceutical preparations thus obtained are safe and low toxic, they can be administered to, for example, human being or a mammal (for example, rat, rabbit, sheep, pig, cow, cat, dog, monkey).
A dose of the compound or a salt thereof is different depending upon an administration subject, a subject organ, symptom and an administration method and, in the case of oral administration, the dose is generally about 0.1 mg-100 mg, preferably about 1.0-50 mg, more preferably about 1.0-20 mg per day, in an adult (60 kg). When parentally administrated, one time dose is different depending upon an administration subject, a subject organ, symptom and an administration method and, in the form of injectable, it is usually advantageous to administer about 0.01-30 mg, preferably about 0.1-20 mg, more preferably about 0.1-10 mg by intravenous injection per day, in an adult (60 kg). In other animals, an amount calculated per 60 kg can be administrated.

(6) Quantitation of present protein , or a partial peptide thereof or a salt thereof

Since an antibody of the present invention can specifically recognize present protein or the like, it can be used for quantitation of present protein or the like in a specimen solution, in particular, quantitation by a sandwich immunodetection method. That is, the present invention provides, for example, (i) a method for quantitating present protein or the like in a specimen solution, which comprises reacting competitively an antibody of the present invention with a specimen solution and a labeled protein or the like, and measuring a ratio of a labeled protein or the like bound to the antibody, (ii) a method for quantitating present protein or the like in a specimen solution, which comprises reacting a specimen solution with an antibody of the present invention which is insolubilized on a carrier and a labeled antibody of the present invention simultaneously or successively, and measuring the activity of a labeling agent on an insolubilized carrier.
In the above (ii), it is preferred that one antibody is an antibody which recognizes a N-terminal of present protein or the like and an another antibody is an antibody which reacts with a C-terminal of present protein or the like.
By using a monoclonal antibody to present protein or the like (hereinafter, referred to as present monoclonal antibody in some cases), detection by tissue staining or the like can be conducted in addition to measurement of present protein or the like. For these purposes, an antibody molecule may be used or F(ab')2, Fab' or Fab fraction of an antibody may be used. A method for measurement using an antibody to present protein or the like is not particularly limited, but any measuring method may be used as long as it is a method for chemically or-physically detecting an amount of an antibody, an antigen or an antibody-antigen complex corresponding to an amount of antigen in a specimen solution (for example, an amount of a protein), and calculating this from a standard curve made using a standard solution containing the known amounts of an antigen. For example, nephrometry, competitive method, immunometric method and sandwich method are suitably used, although the use of a sandwich method described below is particularly preferable in a viewpoint of sensitivity and specificity.

As a labeling agent used in a measuring method using a labeling substance, for example, a radioisotope, an enzyme, a fluorescent substance and emitting substance are used. As a radioisotope, for example, [$^{125}$I], [$^{131}$I], [$^{3}$H] and [$^{14}$C] are used. As the above enzyme, enzymes which are safe and have the great specific activity are preferable. For example, β-glactosidase, β-glycosidase, alkaline phosphatase, peroxidase and malate dehydrogenase are used. As a fluorescent substance, for example, fluorescamine and fluorescein isothiocyanate are used. As an emitting substance, for example, luminol, luminol derivative, luciferin and lucigenin are used. Further, biotin-avidin system may be used for binding an antibody or an antigen and a labeling agent.

Upon insolubization of an antigen or an antibody, physical adsorption may be used and, a method using chemical binding usually used for insolubilizing or immobilizing a protein or an enzyme may be used. As a carrier, for example, insoluble polysaccharides such as agarose, dextran and cellulose, synthetic resins such as polystyrene, polyacrylamide, silicone and the like, or glasses are used.

In a sandwich method, a specimen solution is reacted with an insolubilized monoclonal antibody of the present invention (primary reaction) and a labeled monoclonal antibody of the present invention is further reacted therewith (secondary reaction) and, thereafter, the activity of a labeling agent on an insolubilized carrier can be measured to quantitate an amount of present protein in a specimen solution. The primary reaction and the secondary reaction may be performed in the reverse order, or simultaneously, or at a different time. A labeling agent and an insolubilizing method can be according to the aforementioned methods.

In addition, in an immunological detecting method by a sandwich method, an antibody used for a solid phase antibody or a labeling antibody is not necessarily one kind and a mixture of two or more kinds of antibodies may be used for improving the measuring sensitivity.

In a method for measuring present protein or the like, as monoclonal antibodies of the present invention used in the primary reaction and the secondary reaction, antibodies having a different site to which present protein or the like is bound are preferably used. That is, regarding antibodies used in the primary reaction and the secondary reaction, an antibody used in the primary reaction is preferably an antibody which recognizes a site other than a C-terminal, for example, a N-terminal when an antibody used in the secondary reaction recognizes a C-terminal of present protein.

A monoclonal antibody of the present invention can be used a measuring system other than a sandwich method, for example, competitive method, immunometric method or nephrometry. In a competitive method, after an antigen in a specimen solution and a labeled antigen are reacted competitively with an antibody, an unreacted labeled antigen (F) and a labeled antigen (B) bound to an antibody are separated (B/F separation), and a labeled amount of either of B or F is measured to quantitate an amount of an antigen in a specimen solution. In the present reaction, there are used a solution method in which a soluble antibody is used as an antibody, B/F separation is performed using polyethylene glycol and the second antibody to the aforementioned antibody, and a solid phase method in which a soluble antibody is used as the first antibody and a solid phased antibody is used as the second antibody.

In an immunometric method, after an antigen in a specimen solution and a solid phased antigen are reacted competitively with a constant amount of labeled antibody, the solid phase and the solution phase are separated, or after an antigen in a specimen solution and an excessive amount of labeled antibody are reacted and, then, a solid phased antigen is added to bind unreacted labeled antibody to a solid phase, the solid phase and the solution phase are separated. Next, a labeled amount of either phase is measured to quantitate an amount of an antigen in a specimen solution.

In addition, in nephrometry, an amount of insoluble presipitates resulted from an antigen-antibody reaction in a gel or a solution is measured. Also when an amount of an antigen in a specimen solution is small and a small amount of presipitates are obtained, laser nephrometry using laser scattering is used.

Upon application of individual immunological measuring methods to the present measuring method, setting of special conditions and operations are not required. A system for measuring present protein or a salt thereof may be constructed by addition of technical consideration by those skilled in the art to the conventional conditions and operation procedures in respective methods. For details of these general technical means, a reference can be made to reviews and books [for example, see "Radioimmunoassay" edited by Hiroshi Irie (Kodansha, published in 1974), "Radioimmunoassay, Second Series" edited by Hiroshi Irie (Kodansha, published in 1979), "Enzyme Immunoassay" edited by Eiji Ishikawa et al. (Igakushoin, published in 1978), "Enzyme Immunoassay" edited by Eiji Ishikawa et al. (2nd edition) (Igakushoin, published in 1982), "Enzyme Immunoassay" edited by Eiji Ishikawa et al. (3rd edition) (Igakushoin, published in 1987), "Methods in ENZYMOLOGY" Vol. 70 (Immunochemical Techniques (Part A)), Ibid. Vol. 73 (Immunochemical Techniques (Part B)), Ibid. Vol. 74 (Immunochemical Techniques (Part C)), Ibid. Vol. 84 (Immunochemical Techniques (Part D: Selected Immunoassay)), Ibid. Vol. 92 (Immunochemical Techniques (Part E:Monoclonal Antibodies and Generals Immunoassay Methods)), Ibid. Vol. 121 (Immunochemical Techniques (Part I: Hybridoma Technology and Monoclonal Antibodies)) (All published by Academic Press).

As described above, by using an antibody of the present invention, present protein or a salt thereof can be quantitated with the high sensitivity. Further, various diseases can be diagnosed by quantitating present protein or a salt thereof using an antibody of the present invention.

In addition, an antibody of the present invention can be used to detect present protein or the like present in a specimen such as a body liquid or a tissue. In addition, it can be used for manufacturing an antibody column used for purifying present protein or the like, detecting present protein or the like in each fraction upon purification, and analyzing the behavior of present protein or the like in a specimen cell.

(7) Preparation of non- human animal having a DNA encoding a G protein conjugated type protein of the present invention

A transgenic non-human animal expressing present protein or the like can be made using a DNA of the present invention. As a non-human animal, there is a mammal (for example, rat, mouse, rabbit, sheep, pig, cow, cat, dog, monkey and the like) (hereinafter, abbreviated as animal), although mouse and rabbit are particularly suitable.

Upon transference of a DNA of the present invention to a subject animal, it is generally advantageous to use as a gene construct bound downstream of promoter which can express the DNA in an animal. For example, when a DNA of the present invention derived from a rabbit is transferred, a DNA-transferred animal which produces present protein or the like at a large amount can be produced by introducing a gene construct in which a DNA of the present invention, derived from an animal, having high homology with the above DNA is bound downstream of various promoters which can express a DNA of the present invention in an animal cell, into rabbit fertilized ovum by microinjection. As this promoter, for example, ubiquous expression promoters such as virus-derived promoter, metallothionein promoter and the like may be also used, although NGF gene promoter and enolase gene promoter which are specifically expressed in brain are preferably used.

Transference of a DNA of the present invention at fertilized ovum cell stage is maintained such that the DNA is present in all of an embryo cell and a somatic cell of a subject animal. The presence of present protein or the like in an embryo cell in a produced animal after DNA transference means that all progenies of produced animal have present protein or the like in their all embryo cells and somatic cells. A progeny of such the animal which inheritated a gene has present protein or the like in all its embryo cells and somatic cells.

A DNA transferred animal of the present invention can be passage-bred as an animal harboring the DNA under the normal breeding circumstances by assuring that a gene is stably retained by mating. Further, a homozygote animal having an introduced gene in both of homologous chromosomes can be obtained by mating male and female animals retaining a DNA of interest and breeding passage may be performed such that all progenies have the DNA, by mating the male and female.

Since an animal, in which a DNA of the present invention is introduced, expresses highly present protein or the like, it is useful as an animal for screening an agonist or an antagonist for present protein or the like.

A DNA-transferred animal of the present invention may be used as a cell source for tissue culturing. For example, present protein or the like can be analyzed by analyzing directly a DNA or a RNA in a tissue of a DNA-transferred mouse of the present invention, or by analyzing a tissue in which a protein of the present invention expressed by a gene is present. A cell of a tissue having present protein or the like is cultured by standard tissue culturing technique, which can be used to study the function of a cell from a tissue which is generally difficult to culture, such as derived from brain or peripheral tissue. In addition, by using the cell, for example, a drug which enhances the function of various tissues can be selected. In addition, when there is a high expressing cell strain, present protein or the like can be also isolated and purified therefrom.

When a nucleotide, an amino acid and the like are expressed as an abbreviation in the present invention and drawings, the abbreviation is based on abbreviation by IUPAC-IUB Commission on Biochemical Nomenclature or the conventional abbreviation in the art. Examples thereof are shown below. When there are optical isomers regarding an amino acid, L amino acid is shown unless indicated.

DNA: deoxyribonucleic acid
cDNA: complementary deoxyribonucleic acid
A: adenine
T: thymine
G: guanine
C: cytosine
RNA: ribonucleic acid
mRNA: messenger ribonucleic acid
dATP: deoxyadenosine triphosphate
dTTP: deoxythymidine triphosphate

dGTP: deoxyguanosine triphosphate
dCTP: deoxycytidine triphosphate
ATP: adenosine triphosphate
EDTA: ethylenediamine tetraacetic acid
SDS: sodium dodecylsulfate
Gly: glycine
Ala: alanine
Val: valine
Leu: leucine
Ile: isoleucine
Ser: serine
Thr: threonie
Cys: cysteine
Met: methione
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine
pGlu: pyroglutamic acid
Me: methyl group
Et: ethyl group
Bu: butyl group
Ph: phenyl group
TC: thiazolidine-4(R)-carboxamide group

In addition, substituents, protecting groups and reagents which are frequently used are expressed by the following symbols:

Tos: p-toluenesulfonyl
CHO: formyl
Bzl: benzyl
$Cl_2Bzl$: 2,6-dichlorobenzyl
Bom: benzyloxymethyl
Z: benzyloxycarbonyl
Cl-Z: 2-chlorobenzyloxycarbonyl
Br-Z: 2-bromobenzyloxycarbonyl
Boc: t-butoxycarbonyl
DNP: dinitrophenol
Trt: trityl
Bum: t-butoxymethyl
Fmoc: N-9-fluorenylmethoxycarbonyl
HOBt: 1-hydroxybenztriazole
HOOBt: 3,4-dihydro-3-hydroxy-4-oxo-1,2,3-benzotriazine
HONB: 1-hydroxy- 5 norbornene-2,3-dicarboyximide
DCC: N,N'-dicyclohexylcarbodiimide

Sequence numbers of SEQ ID Tables in the present specification show the following sequences.

[SEQ ID No:1]

An amino acid sequence of a human brain-derived protein of the present invention is shown.

[SEQ ID No:2]

A nucleotide sequence of a DNA encoding a human brain-derived protein of the present invention having an amino acid represented by SEQ ID No:1.

[SEQ ID No:3]

An amino acid sequence of a human brain-derived protein of the present invention is shown.

[SEQ ID No:4]

A nucleotide sequence of a DNA encoding a human brain-derived protein of the present invention having an amino acid represented by SEQ ID No:3.

[SEQ ID No:5]

An amino acid sequence of a human brain-derived protein of the present invention is shown.

[SEQ ID No:6]

A nucleotide sequence of a DNA encoding a human brain-derived protein of the present invention having an amino acid represented by SEQ ID No:5.

Transformant Escherichia coli JM109/pHK05006 obtained in Example 1 below has been deposited at Trade and Commerce Ministry Industrial Technology Institute Life Technology Industrial Technology Laboratory (NIBH) under accession number of FERM BP-6433 since July 21, 1998 and at Foundation Fermentation Laboratory under accession number of IFO 16189 since July 8, 1998.

Transformant Escherichia coli JM109/pHK05490 obtained in Example 2 below has been deposited at Trade and Commerce Ministry Industrial Technology Institute Life Technology Industrial Technology Laboratory (NIBH) under accession number of FERM BP-6456 since August 7, 1998.

Transformant Escherichia coli JM109/pHH02631 obtained in Example 3 below has been deposited at Trade and Commerce Ministry Industrial Technology Institute Life Technology Industrial Technology Laboratory (NIBH) under accession number of FERM BP-6540 since October 6, 1998.

Examples

The following Examples illustrate the present invention but do not limit the scope thereof. Gene manipulations using Escherichia coli were according to a method described in Molecular cloning.

Example 1

Search of a human brain-derived G protein-coupled receptor protein and determination of a nucleotide sequence (1)

Homology search was made to amino acid sequences encoded by cDNAs obtained by a method described in DNA RESEARCH, vol.4, pp.53-59 (1997) or a similar method using sequences of the known receptors; in particular, sequences of human ORF receptors as a template, and an amino acid sequence represented by SEQ ID No:1 (amino acid sequence in Fig. 1 and Fig. 2) showed homology.

Then, cDNA corresponding to amino acid sequence in SEQ ID No:1 (amino acid sequence in Fig. 1 and Fig. 2) was sequence-analyzed and SEQ ID No:2 (nucleotide sequence in Fig. 1 and Fig. 2) was obtained. Hydrophobicity plot shown in Fig. 3 was obtained and it was found that seven times transmembrane type (G protein conjugated type) receptor is encoded.

Further, plasmid pHK05006 harboring a DNA represented by SEQ ID No:2 was introduced into E. coli JM109 to obtain E. coli JM109/pHK05006.

Example 2

Search of a human brain-derived G protein-coupled receptor protein and determination of a nucleotide sequence (2)

Homology search was made to amino acid sequences encoded by cDNAs [SEQ ID No:4 (DNA sequence in Fig.7-Fig. 15)] obtained by a method described in DNA RESEARCH, vol.4, pp.53-59 (1997) or a similar method using sequences of the known receptors; in particular, sequences of human ORF receptors as a template, and an amino acid sequence represented by SEQ ID No:3 (amino acid sequence represented by HKO05490 in Fig. 4 and Fig. 5; amino acid sequence in Fig. 7-Fig.15) showed homology.

Then, hydrophobicity plot was analyzed and this is shown in Fig. 6, and it was found that seven times transmembrane type (G protein conjugated type) receptor is encoded. In addition, it was confirmed that a protein represented by an amino acid sequence represented by SEQ ID No:3 shows high homology with a protein represented by an amino acid sequence represented by SEQ ID No:1 described in Example 1 (Fig.4).

Further, plasmid pHK05490 harboring a DNA represented by SEQ ID No:4 was introduced into E. coli JM109 to obtain E. coli JM109/pHK05490.

Example 3

Search of a human brain-derived G protein-coupled receptor protein and determination of a nucleotide sequence (1)

Homology search was made to amino acid sequences encoded by cDNAs obtained by a method described in DNA RESEARCH, vol.4, pp.53-59 (1997) or a similar method using sequences of the known receptors; in particular, sequences of human ORF receptors as a template, and an amino acid sequence represented by SEQ ID No:5 showed homology.

Then, cDNA corresponding to amino acid sequence in SEQ ID No:5 was sequence-analyzed and SEQ ID No:6 was obtained. Hydrophobicity plot shown in Fig. 25 was obtained and it was found that seven times transmembrane type (G protein conjugated type) receptor is encoded.

Further, plasmid pHH02631 harboring a DNA represented by SEQ ID No:6 was introduced into E. coli JM109 to obtain E. coli JM109/pHH02631.

Industrial Applicability

A protein of the present invention, a partial peptide thereof or a salt thereof, and a DNA encoding it can be used in or as (1) determination of a ligand (agonist), (2) obtaining of an antibody and antiserum, (3) construction of the expression system for a recombinant protein, (4) development of the receptor binding assay using the same expression system and screening of drug candidates, (5) implementation of drug design based on comparison with a ligand receptor having the structural similarity, (6) reagents for preparing a probe or a PCR primer in gene therapy, (7) production of a transgenic animal, or (8) a drug for gene prophylaxis or therapy.

## Claims

1. A protein comprises the same or substantially the same amino acid sequence represented by SEQ ID No:1, SEQ ID No:3 or SEQ ID No:5, or a salt thereof.

2. A partial peptide of the protein according to claim 1, or a salt thereof.

3. A DNA comprises a DNA having a nucleotide sequence encoding the protein according to claim 1.

4. The DNA according to claim 3, which comprising a nucleotide sequence represented by SEQ ID No:2, SEQ ID No:4 or SEQ ID No:6.

5. A recombinant vector comprising the DNA according to claim 3.

6. A transformant transformed with the recombinant vector according to claim 5.

7. A process for producing the protein or a salt thereof according to claim 1, which comprises culturing the transformant according to claim 6, and producing and accumulating the protein according to claim 1.

8. An antibody to the protein according to claim 1, the partial peptide according to claim 2 or a salt thereof.

9. A method for determining a ligand for the protein according to claim 1 or a salt thereof, which comprises using the protein according to claim 1, the partial peptide according to claim 2 or a salt thereof.

**10.** A method for screening a compound or a salt thereof which alters property of a ligand binding with the protein according to claim 1 or a salt thereof, which comprises using the protein according to claim 1, the partial peptide according to claim 2 or a salt thereof.

**11.** A kit for screening a compound or a salt thereof which alters property of a ligand binding with the protein according to claim 1 or a salt thereof, which comprises the protein according to claim 1, the partial peptide according to claim 2 or a salt thereof.

**12.** A compound or a salt thereof which alters property of a ligand binding with the protein according to claim 1 or a salt thereof, which is obtainable by the method for screening according to claim 10 or the kit for screening according to claim 11.

**13.** A pharmaceutical which comprises a compound or a salt thereof which alters property of a ligand binding with the protein according to claim 1 or a salt thereof, which is obtainable by the method for screening according to claim 10 or the kit for screening according to claim 11.

**14.** A DNA which hybridizes with the DNA according to claim 3 under stringent conditions.

**15.** A nucleotide containing a nucleotide sequence encoding the protein according to claim 1.

**16.** A nucleotide containing a part of a nucleotide sequence which is complementary to a nucleotide sequence encoding the protein according to claim 1.

# Figure 1

1 GCTGAACAGACAAGAAATCACTTGAATGCTGGGGACATCACCTACTCTGTCCGGGCCATG 60
1 AlaGluGlnThrArgAsnHisLeuAsnAlaGlyAspIleThrTyrSerValArgAlaMet 20

61 GACCAGCTGGTAGGCCTCCTAGATGTACAGCTTCGGAACTTGACCCCAGGTGGAAAAGAT 120
21 AspGlnLeuValGlyLeuLeuAspValGlnLeuArgAsnLeuThrProGlyGlyLysAsp 40

121 AGTGCTGCCCGGAGTTTGAACAAGGCAATGGTCGAGACAGTTAACAACCTCCTTCAGCCA 180
41 SerAlaAlaArgSerLeuAsnLysAlaMetValGluThrValAsnAsnLeuLeuGlnPro 60

181 CAAGCTTTGAATGCATGGAGAGACCTGACTACGAGTGATCAGCTGCGTGCGGCCACCATG 240
61 GlnAlaLeuAsnAlaTrpArgAspLeuThrThrSerAspGlnLeuArgAlaAlaThrMet 80

241 TTGCTTCATACTGTGGAGGAAAGTGCTTTTGTGCTGGCTGATAACCTTTTGAAGACTGAC 300
81 LeuLeuHisThrValGluGluSerAlaPheValLeuAlaAspAsnLeuLeuLysThrAsp 100

301 ATTGTCAGGGAGAATACAGACAATATTAAATTGGAAGTTGCAAGACTGAGCACAGAAGGA 360
101 IleValArgGluAsnThrAspAsnIleLysLeuGluValAlaArgLeuSerThrGluGly 120

361 AACTTAGAAGACCTAAAATTTCCAGAAAACATGGGCCATGGAAGCACTATCCAGCTGTCT 420
121 AsnLeuGluAspLeuLysPheProGluAsnMetGlyHisGlySerThrIleGlnLeuSer 140

421 GCAAATACCTTAAAGCAAAATGGCCGAAATGGAGAGATCAGAGTGGCCTTTGTCCTGTAT 480
141 AlaAsnThrLeuLysGlnAsnGlyArgAsnGlyGluIleArgValAlaPheValLeuTyr 160

481 AACAACTTGGGTCCTTATTTATCCACGGAGAATGCCAGTATGAAGTTGGGAACGGAAGCT 540
161 AsnAsnLeuGlyProTyrLeuSerThrGluAsnAlaSerMetLysLeuGlyThrGluAla 180

541 TTGTCCACAAATCATTCTGTTATTGTCAATTCCCCTGTTATTACGGCAGCAATAAACAAA 600
181 LeuSerThrAsnHisSerValIleValAsnSerProValIleThrAlaAlaIleAsnLys 200

601 GAGTTCAGTAACAAGGTTTATTTGGCTGATCCTGTGGTATTTACTGTTAAACATATCAAG 660
201 GluPheSerAsnLysValTyrLeuAlaAspProValValPheThrValLysHisIleLys 220

661 CAGTCAGAGGAAAATTTCAACCCTAACTGTTCATTTTGGAGCTACTCCAAGCGTACAATG 720
221 GlnSerGluGluAsnPheAsnProAsnCysSerPheTrpSerTyrSerLysArgThrMet 240

721 ACAGGTTATTGGTCAACACAAGGCTGTCGGCTCCTGACAACAAATAAGACACATACTACA 780
241 ThrGlyTyrTrpSerThrGlnGlyCysArgLeuLeuThrThrAsnLysThrHisThrThr 260

781 TGCTCTTGTAACCACCTAACAAATTTTGCAGTACTGATGGCACATGTGGAAGTTAAGCAC 840
261 CysSerCysAsnHisLeuThrAsnPheAlaValLeuMetAlaHisValGluValLysHis 280

841 AGTGATGCGGTCCATGACCTCCTTCTGGATGTGATCACGTGGGTTGGAATTTTGCTGTCC 900
281 SerAspAlaValHisAspLeuLeuLeuAspValIleThrTrpValGlyIleLeuLeuSer 300

901 CTTGTTTGTCTCCTGATTTGCATCTTCACATTTTGCTTTTTCCGCGGGCTCCAGAGTGAC 960
301 LeuValCysLeuLeuIleCysIlePheThrPheCysPhePheArgGlyLeuGlnSerAsp 320

961 CGTAACACCATCCACAAGAACCTCTGCATCAGTCTCTTTGTAGCAGAGCTGCTCTTCCTG 1020
321 ArgAsnThrIleHisLysAsnLeuCysIleSerLeuPheValAlaGluLeuLeuPheLeu 340

1021 ATTGGGATCAACCGAACTGACCAACCAATTGCCTGTGCTGTTTTCGCTGCCCTGTTACAT 1080
341 IleGlyIleAsnArgThrAspGlnProIleAlaCysAlaValPheAlaAlaLeuLeuHis 360

1081 TTCTTCTTCTTGGCTGCCTTCACCTGGATGTTCCTGGAGGGGGTGCAGCTTTATATCATG 1140
361 PhePhePheLeuAlaAlaPheThrTrpMetPheLeuGluGlyValGlnLeuTyrIleMet 380

1141 CTGGTGGAGGTTTTTGAGAGTGAACATTCACGTAGGAAATACTTTTATCTGGTCGGCTAT 1200
381 LeuValGluValPheGluSerGluHisSerArgArgLysTyrPheTyrLeuValGlyTyr 400

1201 GGGATGCCTGCACTCATTGTGGCTGTGTCAGCTGCAGTAGACTACAGGAGTTATGGAACA 1260
401 GlyMetProAlaLeuIleValAlaValSerAlaAlaValAspTyrArgSerTyrGlyThr 420

1261 GATAAAGTATGTTGGCTCCGACTTGACACCTACTTCATTTGGAGTTTTATAGGACCAGCA 1320
421 AspLysValCysTrpLeuArgLeuAspThrTyrPheIleTrpSerPheIleGlyProAla 440

1321 ACTTTGATAATTATGCTTAATGTAATCTTCCTTGGGATTGCTTTATATAAAATGTTTCAT 1380
441 ThrLeuIleIleMetLeuAsnValIlePheLeuGlyIleAlaLeuTyrLysMetPheHis 460

1381 CATACTGCTATACTGAAACCTGAATCAGGCTGTCTTGATAACATCAAGTCATGGGTTATA 1440
461 HisThrAlaIleLeuLysProGluSerGlyCysLeuAspAsnIleLysSerTrpValIle 480

1441 GGTGCAATAGCTCTTCTCTGCCTATTAGGATTGACCTGGGCCTTTGGACTCATGTATATT 1500
481 GlyAlaIleAlaLeuLeuCysLeuLeuGlyLeuThrTrpAlaPheGlyLeuMetTyrIle 500

# Figure 2

```
1501 AATGAAAGCACAGTCATCATGGCCTATCTCTTCACCATTTTCAATTCTCTACAGGGAATG 1560
 501 AsnGluSerThrValIleMetAlaTyrLeuPheThrIlePheAsnSerLeuGlnGlyMet 520

1561 TTTATATTTATTTTCCATTGTGTCCTACAGAAGAAGGTACGAAAAGAGTATGGAAATGC 1620
 521 PheIlePheIlePheHisCysValLeuGlnLysLysValArgLysGluTyrGlyLysCys 540

1621 CTGCGAACACATTGCTGTAGTGGCAAAAGTACAGAGAGTTCCATTGGTTCAGGGAAAACA 1680
 541 LeuArgThrHisCysCysSerGlyLysSerThrGluSerSerIleGlySerGlyLysThr 560

1681 TCTGGTTCTCGAACTCCTGGACGCTACTCCACAGGCTCACAGAGCCGAATCCGTAGAATG 1740
 561 SerGlySerArgThrProGlyArgTyrSerThrGlySerGlnSerArgIleArgArgMet 580

1741 TGGAATGACACGGTTCGAAAGCAGTCAGAGTCTTCCTTTATTACTGGAGACATAAACAGT 1800
 581 TrpAsnAspThrValArgLysGlnSerGluSerSerPheIleThrGlyAspIleAsnSer 600

1801 TCAGCGTCACTCAACAGAGAGGGGCTTCTGAACAATGCCAGGGATACAAGTGTCATGGAT 1860
 601 SerAlaSerLeuAsnArgGluGlyLeuLeuAsnAsnAlaArgAspThrSerValMetAsp 620

1861 ACTCTACCACTGAATGGTAACCATGGCAATAGTTACAGCATTGCCAGCGGCGAATACCTG 1920
 621 ThrLeuProLeuAsnGlyAsnHisGlyAsnSerTyrSerIleAlaSerGlyGluTyrLeu 640

1921 AGCAACTGTGTGCAAATCATAGACCGTGGCTATAACCATAACGAGACCGCCCTAGAGAAA 1980
 641 SerAsnCysValGlnIleIleAspArgGlyTyrAsnHisAsnGluThrAlaLeuGluLys 660

1981 AAGATTCTGAAGGAACTCACTTCCAACTATATCCCTTCTTACCTGAACAACCATGAGCGC 2040
 661 LysIleLeuLysGluLeuThrSerAsnTyrIleProSerTyrLeuAsnAsnHisGluArg 680

2041 TCCAGTGAACAGAACAGGAATCTGATGAACAAGCTGGTGAATAACCTTGGCAGTGGAAGG 2100
 681 SerSerGluGlnAsnArgAsnLeuMetAsnLysLeuValAsnAsnLeuGlySerGlyArg 700

2101 GAAGATGATGCCATTGTCCTGGATGATGCCACCTCGTTTAACCACGAGGAGAGTTTGGGC 2160
 701 GluAspAspAlaIleValLeuAspAspAlaThrSerPheAsnHisGluGluSerLeuGly 720

2161 CTGGAACTCATTCATGAGGAATCTGATGCTCCTTTGCTGCCCCCAAGAGTATACTCCACC 2220
 721 LeuGluLeuIleHisGluGluSerAspAlaProLeuLeuProProArgValTyrSerThr 740

2221 GAGAACCACCAGCCACACCATTATACCAGAAGGCGGATCCCCCAAGACCACAGTGAGAGC 2280
 741 GluAsnHisGlnProHisHisTyrThrArgArgArgIleProGlnAspHisSerGluSer 760

2281 TTTTTCCCTTTGCTAACCAACGAGCACACAGAAGATCTCCAGTCACCCCATAGAGACTCT 2340
 761 PhePheProLeuLeuThrAsnGluHisThrGluAspLeuGlnSerProHisArgAspSer 780

2341 CTCTATACCAGCATGCCGACACTGGCTGGTGTGGCCGCCACAGAGAGTGTTACCACCAGC 2400
 781 LeuTyrThrSerMetProThrLeuAlaGlyValAlaAlaThrGluSerValThrThrSer 800

2401 ACCCAGACCGAACCCCCACCGGCCAAATGTGGTGATGCCGAAGATGTTTACTACAAAAGC 2460
 801 ThrGlnThrGluProProProAlaLysCysGlyAspAlaGluAspValTyrTyrLysSer 820

2461 ATGCCAAACCTAGGCTCCAGAAACCACGTCCATCAGCTGCATACTTACTACCAGCTAGGT 2520
 821 MetProAsnLeuGlySerArgAsnHisValHisGlnLeuHisThrTyrTyrGlnLeuGly 840

2521 CGCGGCAGCAGTGATGGATTTATAGTTCCTCCAAACAAAGATGGGACCCCTCCCGAGGGA 2580
 841 ArgGlySerSerAspGlyPheIleValProProAsnLysAspGlyThrProProGluGly 860

2581 AGTTCAAAAGGACCGGCTCATTTGGTCACTAGTCTATAGAAGATGACACAGAAATTGGAA 2640
 861 SerSerLysGlyProAlaHisLeuValThrSerLeu*** 873

2641 CCAACAAAACTGCTAACACCTTGTTGACTGTTCTGAGTTGATATAAGCAGTGGTAATAAT 2700
 873 873

2701 GTGTGTACTCCTAAATCTTTATGCTGTCCTCTAAAGACAAACACAAACTCTCAGACTTTT 2760
 873 873

2761 TTTTTTTTTAATGGGATTTTTAGGTCAGCCCAGGGGAGAAAGATAACTGCTAAAATTCCC 2820
 873 873

2821 CTGTACCCCATCCTTTCTTGTCCTTTCCCCTTCAGATGGAGACTTCATTATGTTAATGAA 2880
 873 873

2881 CAAGATATGAAGAAAATGGCACTCATTGTGGCCTTGTTGAATTATCTTGTGTATGTTTTA 2940
 873 873

2941 ACATCTCTGATGCTGTGTTACTAAAATTACAAGGACCTGCTTTTTAAAAGGCCAGAACAA 3000
 873 873
```

Figure 3

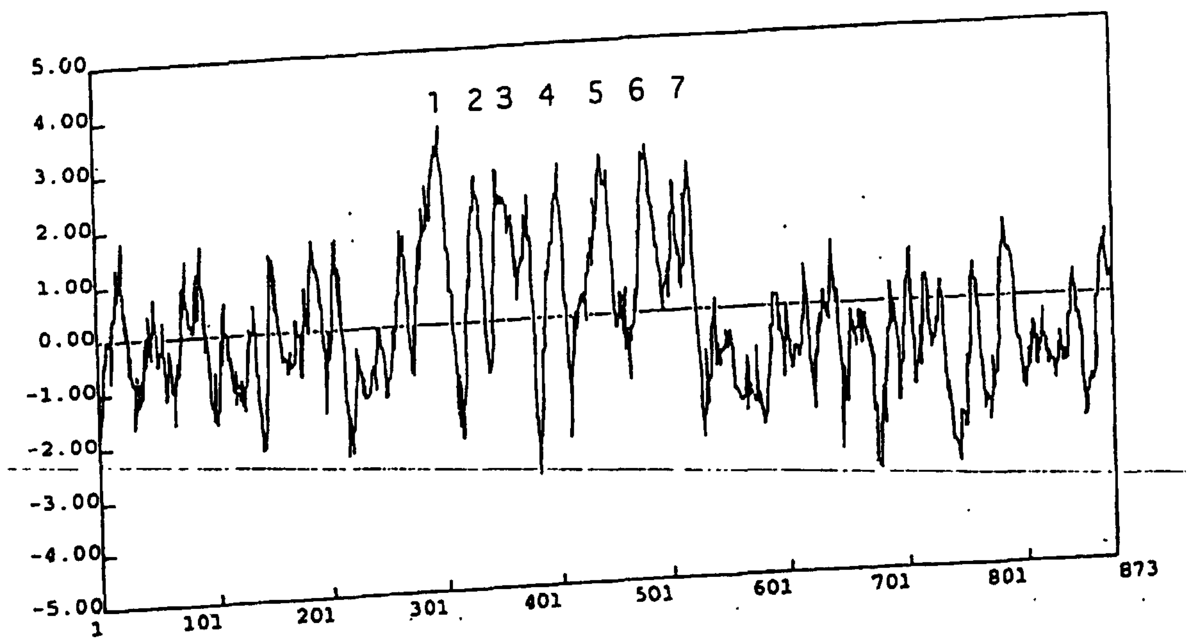
5.00
4.00
3.00
2.00
1.00
0.00
-1.00
-2.00
-3.00
-4.00
-5.00
1 2 3 4 5 6 7
1
101
201
301
401
501
601
701
801
873

Figure 4

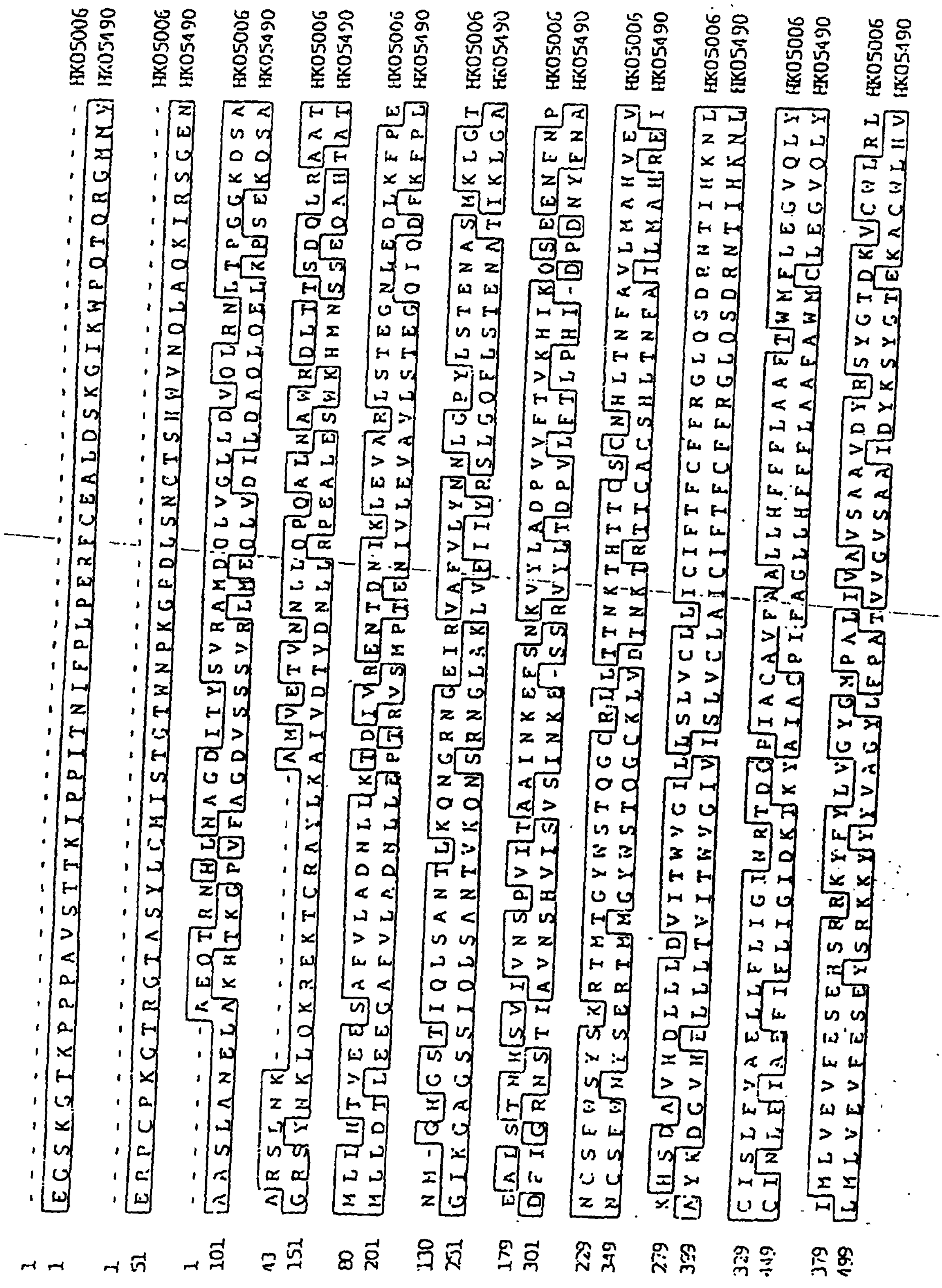

Figure 5

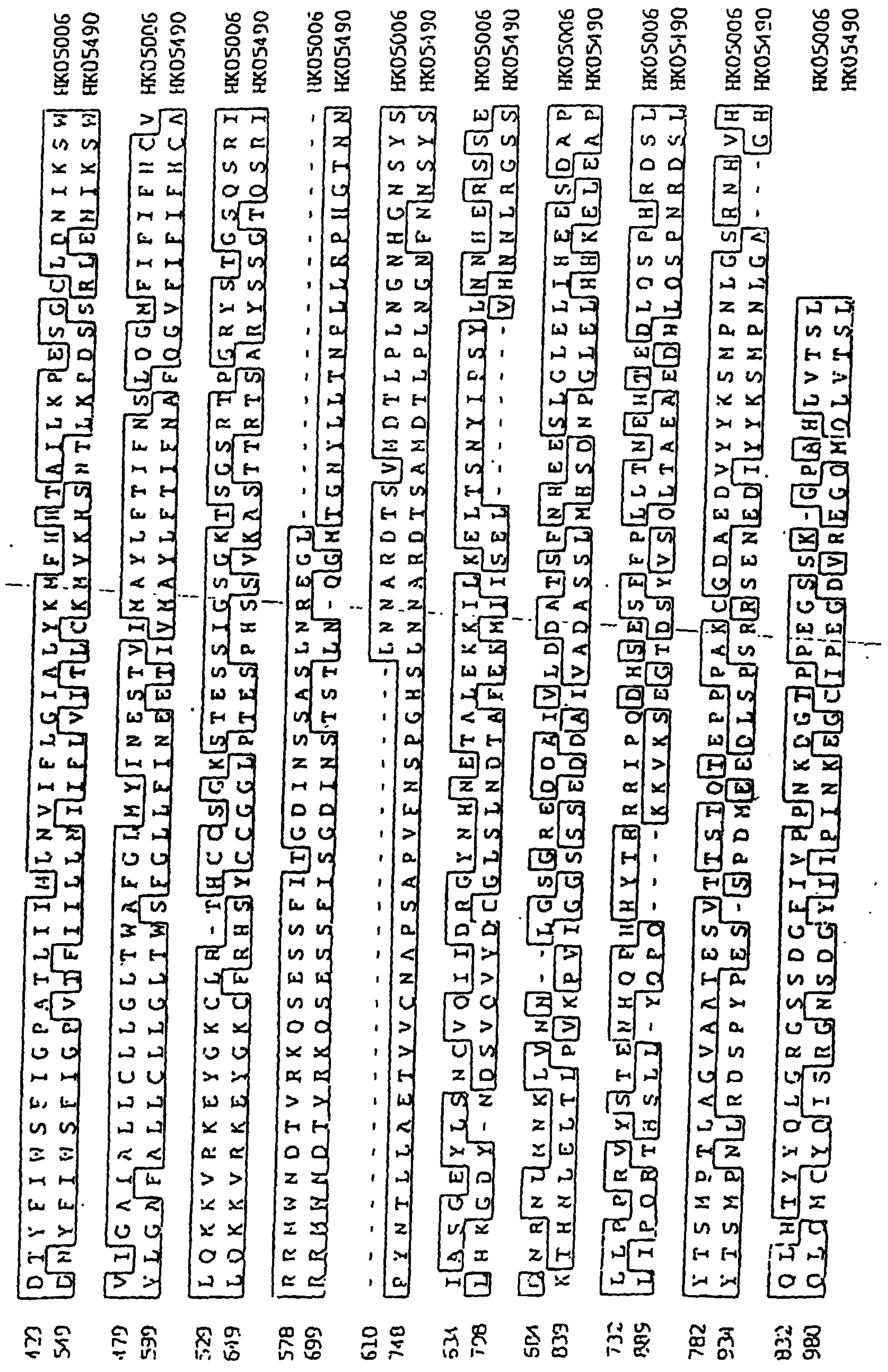

Figure 6

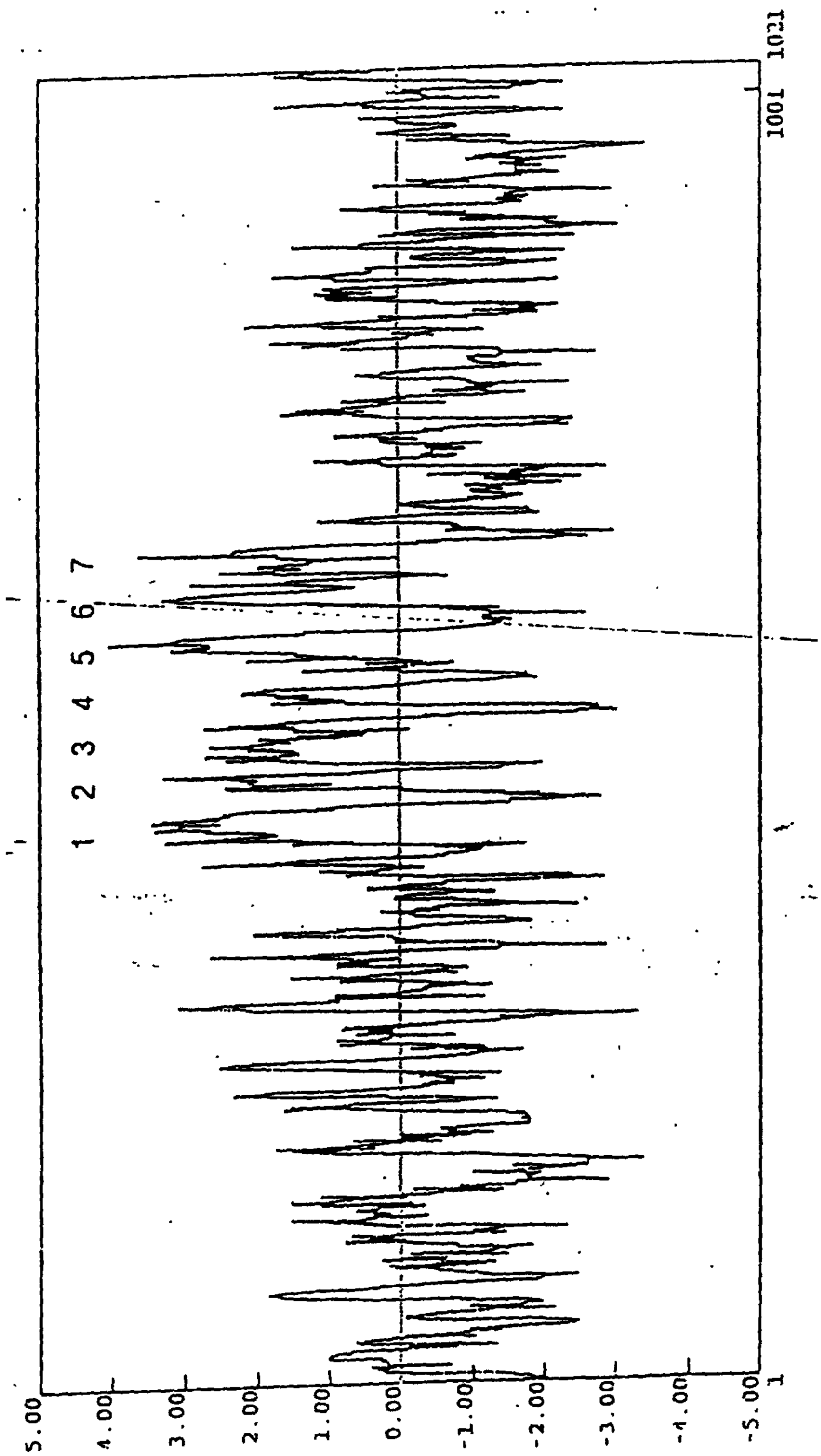

Figure 7

```
1   GAAGGAAGCAAAGGGACAAAACCACCTCCAGCAGTTTCTACAACCAAAATTCCACCTATA   60
1   GluGlySerLysGlyThrLysProProProAlaValSerThrThrLysIleProProIle   20

61  ACAAATATTTTTCCCCTGCCAGAGAGATTCTGTGAAGCATTAGACTCCAAGGGGATAAAG   120
21  ThrAsnIlePheProLeuProGluArgPheCysGluAlaLeuAspSerLysGlyIleLys   40

121 TGGCCTCAGACACAAAGGGGAATGATGGTTGAACGACCATGCCCTAAGGGAACAAGAGGA   180
41  TrpProGlnThrGlnArgGlyMetMetValGluArgProCysProLysGlyThrArgGly   60

181 ACTGCCTCATATCTCTGCATGATTTCCACTGGAACATGGAACCCTAAGGGCCCCGATCTT   240
61  ThrAlaSerTyrLeuCysMetIleSerThrGlyThrTrpAsnProLysGlyProAspLeu   80

241 AGCAACTGTACCTCACACTGGGTGAATCAGCTGGCTCAGAAGATCAGAAGCGGAGAAAAT   300
81  SerAsnCysThrSerHisTrpValAsnGlnLeuAlaGlnLysIleArgSerGlyGluAsn   100

301 GCTGCTAGTCTTGCCAATGAACTGGCTAAACATACCAAAGGGCCAGTGTTTGCTGGGGAT   360
101 AlaAlaSerLeuAlaAsnGluLeuAlaLysHisThrLysGlyProValPheAlaGlyAsp   120

361 GTAAGTTCTTCAGTGAGATTGATGGAGCAGTTGGTGGACATCCTTGATGCACAGCTGCAG   420
121 ValSerSerSerValArgLeuMetGluGlnLeuValAspIleLeuAspAlaGlnLeuGln   140

421 GAACTGAAACCTAGTGAAAAAGATTCAGCTGGACGGAGTTATAACAAGCTCCAAAAACGA   480
141 GluLeuLysProSerGluLysAspSerAlaGlyArgSerTyrAsnLysLeuGlnLysArg   160
```

Figure 8

```
481 GAGAAGACATGCAGGGCTTACCTTAAGGCAATTGTTGACACAGTGGACAACCTTCTGAGA  540
161 GluLysThrCysArgAlaTyrLeuLysAlaIleValAspThrValAspAsnLeuLeuArg  180

541 CCTGAAGCTTTGGAATCATGGAAACATATGAATTCTTCTGAACAAGCACATACTGCAACA  600
181 ProGluAlaLeuGluSerTrpLysHisMetAsnSerSerGluGlnAlaHisThrAlaThr  200

601 ATGTTACTCGATACATTGGAAGAAGGAGCTTTTGTCCTAGCTGACAATCTTTTAGAACCA  660
201 MetLeuLeuAspThrLeuGluGluGlyAlaPheValLeuAlaAspAsnLeuLeuGluPro  220

661 ACAAGGGTCTCAATGCCCACAGAAAATATTGTCCTGGAAGTTGCCGTACTCAGTACAGAA  720
221 ThrArgValSerMetProThrGluAsnIleValLeuGluValAlaValLeuSerThrGlu  240

721 GGACAGATCCAAGACTTTAAATTTCCTCTGGGCATCAAAGGAGCAGGCAGCTCAATCCAA  780
241 GlyGlnIleGlnAspPheLysPheProLeuGlyIleLysGlyAlaGlySerSerIleGln  260

781 CTGTCCGCAAATACCGTCAAACAGAACAGCAGGAATGGGCTTGCAAAGTTGGTGTTCATC  840
261 LeuSerAlaAsnThrValLysGlnAsnSerArgAsnGlyLeuAlaLysLeuValPheIle  280

841 ATTTACCGGAGCCTGGGACAGTTCCTTAGTACAGAAAATGCAACCATTAAACTGGGTGCT  900
281 IleTyrArgSerLeuGlyGlnPheLeuSerThrGluAsnAlaThrIleLysLeuGlyAla  300

901 GATTTTATTGGTCGTAATAGCACCATTGCAGTGAACTCTCACGTCATTTCAGTTTCAATC  960
301 AspPheIleGlyArgAsnSerThrIleAlaValAsnSerHisValIleSerValSerIle  320

961 AATAAAGAGTCCAGCCGAGTATACCTGACTGATCCTGTGCTTTTTACCCTGCCACACATT  1020
```

Figure 9

```
 321 AsnLysGluSerSerArgValTyrLeuThrAspProValLeuPheThrLeuProHisIle    340

1021 GATCCTGACAATTATTTCAATGCAAACTGCTCCTTCTGGAACTACTCAGAGAGAACTATG  1080
 341 AspProAspAsnTyrPheAsnAlaAsnCysSerPheTrpAsnTyrSerGluArgThrMet    360

1081 ATGGGATATTGGTCTACCCAGGGCTGCAAGCTGGTTGACACTAATAAAACTCGAACAACG  1140
 361 MetGlyTyrTrpSerThrGlnGlyCysLysLeuValAspThrAsnLysThrArgThrThr    380

1141 TGTGCATGCAGCCACCTAACCAATTTTGCAATTCTCATGGCCCACAGGGAAATTGCATAT  1200
 381 CysAlaCysSerHisLeuThrAsnPheAlaIleLeuMetAlaHisArgGluIleAlaTyr    400

1201 AAAGATGGCGTTCATGAATTACTTCTTACAGTCATCACCTGGGTGGGAATTGTCATTTCC  1260
 401 LysAspGlyValHisGluLeuLeuLeuThrValIleThrTrpValGlyIleValIleSer    420

1261 CTTGTTTGCCTGGCTATCTGCATCTTCACCTTCTGCTTTTTCCGTGGCCTACAGAGTGAC  1320
 421 LeuValCysLeuAlaIleCysIlePheThrPheCysPhePheArgGlyLeuGlnSerAsp    440

1321 CGAAATACTATTCACAAGAACCTTTGTATCAACCTTTTCATTGCTGAATTTATTTTCCTA  1380
 441 ArgAsnThrIleHisLysAsnLeuCysIleAsnLeuPheIleAlaGluPheIlePheLeu    460

1381 ATAGGCATTGATAAGACAAAATATGCGATTGCATGCCCAATATTTGCAGGACTTCTACAC  1440
 461 IleGlyIleAspLysThrLysTyrAlaIleAlaCysProIlePheAlaGlyLeuLeuHis    480
```

Figure 10

1441 TTTTTCTTTTTGGCAGCTTTTGCTTGGATGTGCCTAGAAGGTGTGCAGCTCTACCTAATG 1500
481 PhePhePheLeuAlaAlaPheAlaTrpMetCysLeuGluGlyValGlnLeuTyrLeuMet 500

1501 TTAGTTGAAGTTTTTGAAAGTGAATATTCAAGGAAAAAATATTACTATGTTGCTGGTTAC 1560
501 LeuValGluValPheGluSerGluTyrSerArgLysLysTyrTyrTyrValAlaGlyTyr 520

1561 TTGTTTCCTGCCACAGTGGTTGGAGTTTCAGCTGCTATTGACTATAAGAGCTATGGAACA 1620
521 LeuPheProAlaThrValValGlyValSerAlaAlaIleAspTyrLysSerTyrGlyThr 540

1621 GAAAAAGCTTGCTGGCTTCATGTTGATAACTACTTTATATGGAGCTTCATTGGACCTGTT 1680
541 GluLysAlaCysTrpLeuHisValAspAsnTyrPheIleTrpSerPheIleGlyProVal 560

1681 ACCTTCATTATTCTGCTAAATATTATCTTCTTGGTGATCACATTGTGCAAAATGGTGAAG 1740
561 ThrPheIleIleLeuLeuAsnIleIlePheLeuValIleThrLeuCysLysMetValLys 580

1741 CATTCAAACACTTTGAAACCAGATTCTAGCAGGTTGGAAAACATTAAGTCTTGGGTGCTT 1800
581 HisSerAsnThrLeuLysProAspSerSerArgLeuGluAsnIleLysSerTrpValLeu 600

1801 GGCGCTTTCGCTCTTCTGTGTCTTCTTGGCCTCACCTGGTCCTTTGGGTTGCTTTTTATT 1860
601 GlyAlaPheAlaLeuLeuCysLeuLeuGlyLeuThrTrpSerPheGlyLeuLeuPheIle 620

1861 AATGAGGAGACTATTGTGATGGCATATCTCTTCACTATATTTAATGCTTTCCAGGGAGTG 1920
621 AsnGluGluThrIleValMetAlaTyrLeuPheThrIlePheAsnAlaPheGlnGlyVal 640

1921 TTCATTTTCATCTTTCACTGTGCTCTCCAAAAGAAAGTACGAAAAGAATATGGCAAGTGC 1980
641 PheIlePheIlePheHisCysAlaLeuGlnLysLysValArgLysGluTyrGlyLysCys 660

Figure 11

```
1981 TTCAGACACTCATACTGCTGTGGAGGCCTCCCAACTGAGAGTCCCCACAGTTCAGTGAAG  2040
 661 PheArgHisSerTyrCysCysGlyGlyLeuProThrGluSerProHisSerSerValLys  680

2041 GCATCAACCACCAGAACCAGTGCTCGCTATTCCTCTGGCACACAGAGTCGTATAAGAAGA  2100
 681 AlaSerThrThrArgThrSerAlaArgTyrSerSerGlyThrGlnSerArgIleArgArg  700

2101 ATGTGGAATGATACTGTGAGAAAACAATCAGAATCTTCTTTTATCTCAGGTGACATCAAT  2160
 701 MetTrpAsnAspThrValArgLysGlnSerGluSerSerPheIleSerGlyAspIleAsn  720

2161 AGCACTTCAACACTTAATCAAGGAATGACTGGCAATTACCTACTAACAAACCCTCTTCTT  2220
 721 SerThrSerThrLeuAsnGlnGlyMetThrGlyAsnTyrLeuLeuThrAsnProLeuLeu  740

2221 CGACCCCACGGCACTAACAACCCCTATAACACATTGCTCGCTGAAACAGTTGTATGTAAT  2280
 741 ArgProHisGlyThrAsnAsnProTyrAsnThrLeuLeuAlaGluThrValValCysAsn  760

2281 GCCCCTTCAGCTCCTGTATTTAACTCACCAGGACATTCACTGAACAATGCCAGGGATACA  2340
 761 AlaProSerAlaProValPheAsnSerProGlyHisSerLeuAsnAsnAlaArgAspThr  780

2341 AGTGCCATGGATACTCTACCGCTAAATGGTAATTTTAACAACAGCTACTCGCTGCACAAG  2400
 781 SerAlaMetAspThrLeuProLeuAsnGlyAsnPheAsnAsnSerTyrSerLeuHisLys  800

2401 GGTGACTATAATGACAGCGTGCAAGTTGTGGACTGTGGACTAAGTCTGAATGATACTGCT  2460
 801 GlyAspTyrAsnAspSerValGlnValValAspCysGlyLeuSerLeuAsnAspThrAla  820
```

Figure 12

```
2461 TTTGAGAAAATGATCATTTCAGAATTAGTGCACAACAACTTACGGGGCAGCAGCAAGACT  2520
 821 PheGluLysMetIleIleSerGluLeuValHisAsnAsnLeuArgGlySerSerLysThr  840

2521 CACAACCTCGAGCTCACGCTACCAGTCAAACCTGTGATTGGAGGTAGCAGCAGTGAAGAT  2580
 841 HisAsnLeuGluLeuThrLeuProValLysProValIleGlyGlySerSerSerGluAsp  860

2581 GATGCTATTGTGGCAGATGCTTCATCTTTAATGCACAGCGACAACCCAGGGCTGGAGCTC  2640
 861 AspAlaIleValAlaAspAlaSerSerLeuMetHisSerAspAsnProGlyLeuGluLeu  880

2641 CATCACAAAGAACTCGAGGCACCACTTATTCCTCAGCGGACTCACTCCCTTCTGTACCAA  2700
 881 HisHisLysGluLeuGluAlaProLeuIleProGlnArgThrHisSerLeuLeuTyrGln  900

2701 CCCCAGAAGAAAGTGAAGTCCGAGGGAACTGACAGCTATGTCTCCCAACTGACAGCAGAG  2760
 901 ProGlnLysLysValLysSerGluGlyThrAspSerTyrValSerGlnLeuThrAlaGlu  920

2761 GCTGAAGATCACCTACAGTCCCCCAACAGAGACTCTCTTTATACAAGCATGCCCAATCTT  2820
 921 AlaGluAspHisLeuGlnSerProAsnArgAspSerLeuTyrThrSerMetProAsnLeu  940

2821 AGAGACTCTCCCTATCCGGAGAGCAGCCCTGACATGGAAGAAGACCTCTCTCCCTCCAGG  2880
 941 ArgAspSerProTyrProGluSerSerProAspMetGluGluAspLeuSerProSerArg  960

2881 AGGAGTGAGAATGAGGACATTTACTATAAAAGCATGCCAAATCTTGGAGCTGGCCATCAG  2940
 961 ArgSerGluAsnGluAspIleTyrTyrLysSerMetProAsnLeuGlyAlaGlyHisGln  980
```

Figure 13

```
2941 CTTCAGATGTGCTACCAGATCAGCAGGGGCAATAGTGATGGTTATATAATCCCCATTAAC  3000
 981 LeuGlnMetCysTyrGlnIleSerArgGlyAsnSerAspGlyTyrIleIleProIleAsn  1000

3001 AAAGAAGGGTGTATTCCAGAAGGAGATGTTAGAGAAGGACAAATGCAGCTGGTTACAAGT  3060
1001 LysGluGlyCysIleProGluGlyAspValArgGluGlyGlnMetGlnLeuValThrSer  1020

3061 CTTTAATCATACAGCTAAGGAATTCCAAGGGCCACATGCGAGTATTAATAAATAAAGACA  3120
1021 Leu***                                                          1022

3121 CCATTGGCCTGACGCAGCTCCCTCAAACTCTGCTTGAAGAGATGACTCTTGACCTGTGGT  3180
1022                                                                 1022

3181 TCTCTGGTGTAAAAAAGATGACTGAACCTTGCAGTTCTGTGAATTTTTATAAAACATACA  3240
1022                                                                 1022

3241 AAAACTTTGTATATACACAGAGTATACTAAAGTGAATTATTTGTTACAAAGAAAAGAGAT  3300
1022                                                                 1022

3301 GCCAGCCAGGTATTTTAAGATTCTGCTGCTGTTTAGAGAAATTGTGAAACAAGCAAAACA  3360
1022                                                                 1022

3361 AAACTTTCCAGCCATTTTACTGCAGCAGTCTGTGAACTAAATTTGTAAATATGGCTGCAC  3420
1022                                                                 1022
```

Figure 14

3421 CATTTTTGTAGGCCTGCATTGTATTATATACAAGACGTAGGCTTTAAAATCCTGTGGGAC 3480
1022 1022

3481 AAATTTACTGTACCTTACTATTCCTGACAAGACTTGGAAAAGCAGGAGAGATATTCTGCA 3540
1022 1022

3541 TCAGTTTGCAGTTCACTGCAAATCTTTTACATTAAGGCAAAGATTGAAAACATGCTTAAC 3600
1022 1022

3601 CACTAGCAATCAAGCCACAGGCCTTATTTCATATGTTTCCTCAACTGTACAATGAACTAT 3660
1022 1022

3661 TCTCATGAAAAATGGCTAAAGAAATTATATTTTGTTCTATTGCTAGGGTAAAATAAATAC 3720
1022 1022

3721 ATTTGTGTCCAACTGAAATATAATTGTCATTAAAATAATTTTAAAGAGTGAAGAAAATAT 3780
1022 1022

3781 TGTGAAAAGCTCTTGGTTGCACATGTTATGAAATGTTTTTTCTTACACTTTGTCATGGTA 3840
1022 1022

3841 AGTTCTACTCATTTTCACTTCTTTTCCACTGTATACAGTGTTCTGCTTTGACAAAGTTAG 3900
1022 1022

3901 TCTTTATTACTTACATTTAAATTTCTTATTGCCAAAAGAACGTGTTTTATGGGAGAAAC 3960

Figure 15

1022 1022

3961 AAACTCTTTGAAGCCAGTTATGTCATGCCTTGCACAAAAGTGATGAAATCTAGAAAAGAT 4020
1022 1022

4021 TGTGTGTCACCCCTGTTTATTCTTGAACAGAGGGCAAAGAGGGCACTGGCACTTCTCAC 4080
1022 1022

4081 AAACTTTCTAGTGAACAAAAGGTGCCTATTCTTTTTTAAAAAAATAAAATAAAACATAAA 4140
1022 1022

4141 TATTACTCTTCCATATTCCTTCTGCCTATATTTAGTAATTAATTTATTTTATGATAAAGT 4200
1022 1022

4201 TCTAATGAAATGTAAATTGTTTCAGCAAAATTCTGCTTTTTTTTCATCCCTTTGTGTAAA 4260
1022 1022

4261 CCTGTTAATAATGAGCCCATCACTAATATCCAGTGTAAAGTTTAACACGGTTTGACAGTA 4320
1022 1022

4321 AATAAATGTGAATTTTTTTCAAGT 4343
1022 1022

# Figure 16

| | 1 | | | | 50 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | MARLAAVLWN | LCVTAVLVTS | ATQGLSRAGL | PFGLMRRELA | CEGYPIELRC |

| | 51 | | | | 100 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | PGSDVIMVEN | ANYGRTDDKI | CDADPFQMEN | VQCYLPDAFK | IMSQRCNNRT |

| | 101 | | | | 150 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | QCVVVAGSDA | FPDPCPGTYK | YLEVQYDCVP | YKVEQKVFVC | PGTLQKVLEP |

| | 151 | | | | 200 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | TSTHESEHQS | GAWCKDPLQA | GDRIYVMPWI | PYRTDTLTEY | ASWEDYVAAR |

| | 201 | | | | 250 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | HTTTYRLPNR | VDGTGFVVYD | GAVFYNKERT | RNIVKYDLRT | RIKSGETVIN |

| | 251 | | | | 300 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | TANYHDTSPY | RWGGKTDIDL | AVDENGLWVI | YATEGNNGRL | VVSQLNPYTL |

| | 301 | | | | 350 |
|---|---|---|---|---|---|
| HK05006 | ---------- | ---------- | ---------- | ---------- | ---------- |
| HK05490 | ---------- | ---------- | ---------- | ---------- | ---------- |
| HH02631 | RFEGTWETGY | DKRSASNAFM | VCGVLYVLRS | VYVDDDSEAA | GNRVDYAFNT |

# Figure 17

```
          351                                                    400
HK05006   __________ __________ __________ __________ __________
HK05490   __________ __________ __________ __________ __________
HH02631   NANREEPVSL TFPNPYQFIS SVDYNPRDNQ LYVWNNYFVV RYSLEFGPPD

          401                                                    450
HK05006   __________ __________ __________ __________ __________
HK05490   __________ __________ __________ __________ _________E
HH02631   PSAGPATSPP LSTTTTARPT PLTSTASPAA TTPLRRAPLT THPVGAINQL

          451                                                    500
HK05006   __________ __________ __________ __________ __________
HK05490   GSKGTKPPPA VSTTKIPPIT NIFPLPERFC EALDSKGIKW PQTQRGMMVE
HH02631   GPDLPPATAP VPSTRRPPAP NLHVSPELFC EPREVRRVQW PATQQGMLVE

          501                                                    550
HK05006   __________ __________ __________ __________ __________
HK05490   RPCPKGTRGT ASYLCMISTG TWNPKGPDLS NCTSHWVNQL AQKIRSGENA
HH02631   RPCPKGTRGI ASFQCLPALG LWNPRGPDLS NCTSPWVNQV AQKIKSGENA

          551                                                    600
HK05006   _______AEQ TRNHLNAGDI TYSVRAMDQL VGLLDVQLRN LTPGGKDSAA
HK05490   ASLANELAKH TKGPVFAGDV SSSVRLMEQL VDILDAQLQE LKPSEKDSAG
HH02631   ANIASELARH TRGSIYAGDV SSSVKLMEQL LDILDAQLQA LRPIERESAG

          601                                                    650
HK05006   RSLN...... .......KAM VETVNNLLQP QALNAWRDLT TSDQLRAATM
HK05490   RSYNKLQKRE KTCRAYLKAI VDTVDNLLRP EALESWKHMN SSEQAHTATM
HH02631   KNYNKMHKRE RTCKDYIKAV VETVDNLLRP EALESWKDMN ATEQVHTATM

          651                                                    700
HK05006   LLHTVEESAF VLADNLLKTD IVRENTDNIK LEVARLSTEG NLEDLKFP.E
HK05490   LLDTLEEGAF VLADNLLEPT RVSMPTENIV LEVAVLSTEG QIQDFKFPLG
HH02631   LLDVLEEGAF LLADNVREPA RFLAAKENVV LEVTVLNTEG QVQELVFPQE
```

Figure 18

```
         701                                                     750
HK05006  NMGHGSTIQL SANTLKQNGR NGEIRVAFVL YNNLGPYLST ENASMKLGTE
HK05490  IKGAGSSIQL SANTVKQNSR NGLAKLVFII YRSLGQFLST ENATIKLGAD
HH02631  EYPRKNSIQL SAKTIKQNSR NGVVKVVFIL YNNLGLFLST ENATVKLAGE

         751                                                     800
HK05006  A...LSTNHS VIVNSPVITA AINKEFSNKV YLADPVVFTV KHIKQSEENF
HK05490  F...IGRNST IAVNSHVISV SINKE.SSRV YLTDPVLFTL PHI.DPDNYF
HH02631  AGPGGPGGAS LVVNSQVIAA SINKE.SSRV FLMDPVIFTV AHL.EDKNHF

         801                                                     850
HK05006  NPNCSFWSYS KRTMTGYWST QGCRLLTTNK THTTCSCNHL TNFAVLMAHV
HK05490  NANCSFWNYS ERTMMGYWST QGCKLVDTNK TRTTCACSHL TNFAILMAHR
HH02631  NANCSFWNYS ERSMLGYWST QGCRLVESNK THTTCACSHL TNFAVLMAHR

         851                                                     900
HK05006  EVKHSDAVHD LLLDVITWVG ILLSLVCLLI CIFTFCFFRG LQSDRNTIHK
HK05490  EIAYKDGVHE LLLTVITWVG IVISLVCLAI CIFTFCFFRG LQSDRNTIHK
HH02631  EI.YQGRINE LLLSVITWVG IVISLVCLAI CISTFCFLRG LQTDRNTIHK

         901                                                     950
HK05006  NLCISLFVAE LLFLIGINRT DQPIACAVFA ALLHFFFLAA FTWMFLEGVQ
HK05490  NLCINLFIAE FIFLIGIDKT KYAIACPIFA GLLHFFFLAA FAWMCLEGVQ
HH02631  NLCINLFLAE LLFLVGIDKT QYEIACPIFA GLLHYFFLAA FSWLCLEGVH

         951                                                     1000
HK05006  LYIMLVEVFE SEHSRRKYFY LVGYGMPALI VAVSAAVDYR SYGTDKVCWL
HK05490  LYLMLVEVFE SEYSRKKYYY VAGYLFPATV VGVSAAIDYK SYGTEKACWL
HH02631  LYLLLVEVFE SEYSRTKYYY LGGYCFPALV VGIAAAIDYR SYGTEKACWL

         1001                                                    1050
HK05006  RLDTYFIWSF IGPATLIIML NVIFLGIALY KMFHHTAILK PESGCLDNIK
HK05490  HVDNYFIWSF IGPVTFIILL NIIFLVITLC KMVKHSNTLK PDSSRLENIK
HH02631  RVDNYFIWSF IGPVSFVIVV NLVFLMVTLH KMIRSSSVLK PDSSRLDNIK
```

# Figure 19

```
         1051                                               1100
HK05006  SWVIGAIALL CLLGLTWAFG LMYINESTVI MAYLFTIFNS LQGMFIFIFH
HK05490  SWVLGAFALL CLLGLTWSFG LLFINEETIV MAYLFTIFNA FQGVFIFIFH
HH02631  SWALGAIALL FLLGLTWAFG LLFINKESVV MAYLFTTFNA FQGVFIFVFH

         1101                                               1150
HK05006  CVLQKKVRKE YGKCLR.THC CSGKSTESSI GSGKTSGSRT PGRYSTGSQS
HK05490  CALQKKVRKE YGKCFRHSYC CGGLPTESPH SSVKASTTRT SARYSSGTQS
HH02631  CALQKKVHKE YSKCLRHSYC CIRSPPGGTH GSLKTSAMRS NTRYYTGTQS

         1151                                               1200
HK05006  RIRRMWNDTV RKQSESSFIT GDINSSASLN REGLLN.... ..........
HK05490  RIRRMWNDTV RKQSESSFIS GDINSTSTLN QGMTGNYLLT NPLLRPHGTN
HH02631  RIRRMWNDTV RKQTESSFMA GDINSTPTLN RGTMGNHLLT NPVLQPRGGT

         1201                                               1250
HK05006  .......... .......... .......... .....NARDTS VMDTLPLNGN
HK05490  NPYNTLLAET VVCNAPSAPV FNSPGHSLN. ....NARDTS AMDTLPLNGN
HH02631  SPYNTLIAES VGFNPSSPPV FNSPGSYREP KHPLGGREAC GMDTLPLNGN

         1251                                               1300
HK05006  HGNSYSIASG EYLSN.CVQI IDRGYNHNE. TALEKKILKE LTSNYIPSYL
HK05490  FNNSYSLHKG DY..NDSVQV VDCGLSLND. TAFEKMIISE LVHN....NL
HH02631  FNNSYSLRSG DFPPGDGGPE PPRGRNLADA AAFEKMIISE LVHN....NL

         1301                                               1350
HK05006  NNHERSSEQN RNLMNKLVNN LGSGREDDAI VLDDATSFNH EESLGLELIH
HK05490  RGSSKTHN.. LELTLPVKPV IGGSSSEDDA IVADASSLMH SDNPGLELHH
HH02631  RGSSSAAKGP PPPEPPVPPV PGGGGEE... ...EAGGPGG ADRAEIELLY

         1351                                               1400
HK05006  EESDAPLLPP RVYSTENHQP HHYTRRRIPQ DHSESFFPLL TNEHTEDLQS
HK05490  KELEAPLIPQ RTHSL..... LYQPQKKVKS EGTDSYVSQL TAEAEDHLQS
HH02631  KALEEPLLLP RAQSV..... LYQSD....L DESESCTAED GATSRPLSSP
```

Figure 20

```
          1401                                                  1450
HK05006   PHRDSLYTSM PTLAGVAATE SVTTSTQTE. ..PPPAKCGD AEDVYYKSM.
HK05490   PNRDSLYTSM PNLRDSP.YP ESSPDMEEDL ...SPSRRSE NEDIYYKSM.
HH02631   PGRDSLYASG ANLRDSPSYP DSSPEGPSEA LPPPPPAPPG PPEIYYTSRP

          1451                                                  1500
HK05006   PNLGSRNHVH QLHTYYQLGR GSSDGFIVPP NKDGTPPEGS .SKGPAHLVT
HK05490   PNLGAG...H QLQMCYQISR GNSDGYIIPI NKEGCIPEGD VREGQMQLVT
HH02631   PALVAR...N PLQGYYQVRR PSHEGYLAAP GLEGPGPDGD ...GQMQLVT

          1501
HK05006   SL
HK05490   SL
HH02631   SL
```

Figure 21

TTTTTTTTTTTTTTTTTTCCTAATTTTTGGTCGGCGGCGGTGCTGGGCCAG 50
GGGAAGGAAGGGACACGGAGGCCGCCCTCGTCCCGCCACCTCCTACCCGC 100
TTCCCCCCAGCCCCGGCTCCGGGAGATGTGCCGGGCGGGGGGCCCGGGTT 150
CGCCGAGCCGCAGGAGAGACACGCTGGGCCGACCCCAGAGAGGCGCTGGA 200
CAGGCTGGTGGTCCAGGCCGTGGTGCCTGCCAGGTGATGTGGGGCAAAGC 250
CCCCCGCACAGGCCACTGAGAGCTCCGGACACGCACCCGGCTGCCACCAT 300
GGCCCGCCTAGCCGCAGTGCTCTGGAATCTGTGTGTCACCGCCGTCCTGG 350
TCACCTCGGCCACCCAAGGCCTGAGCCGGGCCGGGCTCCCGTTCGGGCTG 400
ATGCGCCGGGAGCTGGCGTGTGAAGGCTACCCCATCGAGCTGCGGTGCCC 450
CGGCAGCGACGTCATCATGGTGGAGAATGCCAACTACGGGCGCACGGACG 500
ACAAGATTTGCGATGCTGACCCTTTCCAGATGGAGAATGTGCAGTGCTAC 550
CTGCCGGACGCCTTCAAGATCATGTCACAGAGGTGTAACAACCGCACCCA 600
GTGCGTGGTGGTCGCCGGCTCGGATGCCTTTCCTGACCCCTGTCCTGGGA 650
CCTACAAGTACCTGGAGGTGCAGTACGACTGTGTCCCCTACAAAGTGGAG 700
CAGAAAGTCTTCGTGTGCCCAGGGACCCTGCAGAAGGTGCTGGAGCCCAC 750
CTCGACACACGAGTCAGAGCACCAGTCTGGCGCATGGTGCAAGGACCCGC 800
TGCAGGCGGGTGACCGCATCTACGTGATGCCCTGGATCCCCTACCGCACG 850
GACACACTGACTGAGTATGCCTCGTGGGAGGACTACGTGGCCGCCCGCCA 900
CACCACCACCTACCGCCTGCCCAACCGCGTGGATGGCACAGGCTTTGTGG 950
TCTACGATGGTGCCGTCTTCTACAACAAGGAGCGCACGCGCAACATCGTC 1000
AAGTATGACCTACGGACGCGCATCAAGAGCGGGGAGACGGTCATCAATAC 1050
CGCCAACTACCATGACACCTCGCCCTACCGCTGGGGCGGAAAGACCGACA 1100
TTGACCTGGCGGTGGACGAGAACGGGCTGTGGGTCATCTACGCCACTGAG 1150
GGCAACAACGGGCGGCTGGTGGTGAGCCAGCTGAACCCCTACACACTGCG 1200
CTTTGAGGGCACGTGGGAGACGGGTTACGACAAGCGCTCGGCATCCAACG 1250
CCTTCATGGTGTGTGGGGTCCTGTACGTCCTGCGCTCCGTGTACGTGGAT 1300
GATGACAGCGAGGCGGCTGGCAACCGCGTGGACTATGCCTTCAACACCAA 1350
TGCCAACCGCGAGGAGCCTGTCAGCCTCACCTTCCCCAACCCCTACCAGT 1400
TCATCTCCTCCGTTGACTACAACCCTCGCGACAACCAGCTGTACGTCTGG 1450
AACAACTATTTCGTGGTGCGCTACAGCCTGGAGTTCGGGCCGCCCGACCC 1500
CAGTGCTGGCCCAGCCACTTCCCCACCCCTCAGCACGACCACCACAGCCA 1550
GGCCCACGCCCCTCACCAGCACAGCCTCGCCCGCAGCCACCACCCCGCTC 1600
CGCCGGGCACCCCTCACCACGCACCCAGTGGGTGCCATCAACCAGCTGGG 1650
ACCTGATCTGCCTCCAGCCACAGCCCCAGTCCCCAGCACCCGGCGGCCCC 1700
CAGCCCCGAATCTACACGTGTCCCCTGAGCTCTTCTGCGAGCCCCGAGAG 1750

Figure 22

GTACGGCGGGTCCAGTGGCCGGCCACCCAGCAGGGCATGCTGGTGGAGAG 1800
GCCCTGCCCCAAGGGGACTCGAGGAATTGCCTCCTTCCAGTGTCTACCAG 1850
CCTTGGGGCTCTGGAACCCCCGGGGCCCTGACCTCAGCAACTGCACCTCC 1900
CCCTGGGTCAACCAGGTGGCCCAGAAGATCAAGAGTGGGGAGAACGCGGC 1950
CAACATCGCCAGCGAGCTGGCCCGACACACCCGGGGCTCCATCTACGCGG 2000
GGGACGTCTCCTCCTCTGTGAAGCTGATGGAGCAGCTGCTGGACATCCTG 2050
GATGCCCAGCTGCAGGCCCTGCGGCCCATCGAGCGCGAGTCAGCCGGCAA 2100
GAACTACAACAAGATGCACAAGCGAGAGAGAACTTGTAAGGATTATATCA 2150
AGGCCGTGGTGGAGACAGTGGACAATCTGCTCCGGCCAGAAGCTCTGGAG 2200
TCCTGGAAGGACATGAATGCCACGGAGCAGGTGCACACGGCCACCATGCT 2250
CCTCGACGTCCTGGAGGAGGGCGCCTTCCTGCTGGCCGACAATGTCAGGG 2300
AGCCTGCCCGCTTCCTGGCTGCCAAGGAGAACGTGGTCCTGGAGGTCACA 2350
GTCCTGAACACAGAGGGCCAGGTGCAGGAGCTGGTGTTCCCCCAGGAGGA 2400
GTACCCGAGAAAGAACTCCATCCAGCTGTCTGCCAAAACCATCAAGCAGA 2450
ACAGCCGCAATGGGGTGGTCAAAGTTGTCTTCATCCTCTACAACAACCTG 2500
GGCCTCTTCCTGTCCACGGAGAATGCCACAGTGAAGCTGGCCGGCGAAGC 2550
AGGCCCGGGTGGCCCTGGGGGCGCCTCTCTAGTGGTGAACTCACAGGTCA 2600
TCGCAGCATCCATCAACAAGGAGTCCAGCCGCGTCTTCCTCATGGACCCT 2650
GTCATCTTCACCGTGGCCCACCTGGAGGACAAGAACCACTTCAATGCTAA 2700
CTGCTCCTTCTGGAACTACTCGGAGCGTTCCATGCTGGGCTATTGGTCGA 2750
CCCAAGGCTGCCGCCTGGTGGAGTCCAACAAGACCCATACCACGTGTGCC 2800
TGCAGCCACCTCACCAACTTCGCTGTGCTCATGGCTCACCGTGAGATCTA 2850
CCAGGGCCGCATCAACGAGCTGCTGCTGTCGGTCATCACCTGGGTGGGCA 2900
TTGTGATCTCCCTGGTCTGCTTGGCCATCTGCATCTCCACCTTCTGCTTC 2950
CTGCGGGGGCTGCAGACCGACCGCAACACCATCCACAAGAACCTGTGCAT 3000
CAACCTCTTCCTGGCTGAGCTGCTCTTCCTGGTCGGGATCGACAAGACTC 3050
AGTATGAGATTGCCTGCCCCATCTTCGCCGGCCTGCTGCACTATTTCTTC 3100
CTGGCTGCCTTCTCCTGGCTGTGCCTGGAGGGCGTGCACCTCTACCTGCT 3150
ACTAGTGGAGGTGTTTGAGAGCGAGTATTCCCGCACCAAGTACTACTACC 3200
TGGGTGGCTACTGCTTCCCGGCCCTGGTGGTGGGCATCGCGGCTGCCATT 3250
GACTACCGCAGCTACGGCACCGAGAAGGCCTGCTGGCTCCGAGTGGACAA 3300
TTACTTCATCTGGAGTTTCATCGGGCCAGTCTCCTTCGTTATCGTGGTCA 3350
ACCTGGTGTTCCTCATGGTGACCCTGCACAAGATGATCCGAAGCTCATCT 3400
GTGCTCAAGCCCGACTCCAGCCGCCTGGACAACATTAAATCCTGGGCGCT 3450
GGGGGCCATCGCGCTGCTGTTCCTGCTGGGCCTCACCTGGGCTTTCGGCC 3500

Figure 23

TCCTCTTCATCAACAAGGAGTCGGTGGTCATGGCCTATCTCTTCACCACC 3550
TTCAACGCCTTCCAGGGGGTCTTCATCTTCGTCTTTCACTGCGCCTTACA 3600
GAAGAAGGTGCACAAGGAGTACAGCAAGTGCCTGCGTCACTCCTACTGCT 3650
GCATCCGCTCCCCACCCGGGGGCACTCACGGATCCCTCAAGACCTCAGCC 3700
ATGCGAAGCAACACCCGCTACTACACAGGGACCCAGAGCCGAATTCGGAG 3750
GATGTGGAATGACACTGTGAGGAAACAGACGGAGTCCTCCTTCATGGCGG 3800
GTGACATCAACAGCACCCCCACCCTGAACCGAGGTACCATGGGGAACCAC 3850
CTGCTGACCAACCCCGTGCTGCAGCCCCGTGGGGGCACCAGTCCCTACAA 3900
CACCCTCATCGCCGAGTCAGTGGGCTTCAATCCCTCCTCGCCCCCTGTCT 3950
TCAACTCCCCAGGGAGCTACCGGGAACCCAAGCACCCCTTGGGAGGCCGG 4000
GAAGCCTGTGGCATGGACACCCTGCCCCTGAACGGCAACTTCAATAACAG 4050
TTACTCCTTGCGAAGTGGGGATTTCCCTCCCGGGGATGGGGGCCCTGAGC 4100
CGCCCCGAGGCCGGAACCTAGCCGATGCGGCGGCCTTTGAGAAGATGATC 4150
ATCTCAGAGCTGGTGCACAACAACCTGCGGGGGAGCAGCAGCGCGGCCAA 4200
GGGCCCTCCACCGCCTGAGCCCCCTGTGCCACCTGTGCCAGGGGGCGGGG 4250
GCGAGGAAGAGGCGGGCGGGCCCGGGGGTGCTGACCGGGCCGAGATTGAA 4300
CTTCTCTATAAGGCCCTGGAGGAGCCTCTGCTGCTGCCCCGGGCCCAGTC 4350
GGTGCTGTACCAGAGCGATCTGGACGAGTCGGAGAGCTGCACGGCCGAGG 4400
ACGGCGCCACCAGCCGGCCCCTCTCCTCCCCTCCTGGCCGGGACTCCCTC 4450
TATGCCAGCGGGGCCAACCTGCGGGACTCACCCTCCTACCCGGACAGCAG 4500
CCCTGAGGGGCCCAGTGAGGCCCTGCCCCCACCCCCTCCCGCACCCCCCG 4550
GCCCCCCCGAAATCTACTACACCTCGCGCCCGCCAGCCCTGGTGGCCCGG 4600
AATCCCCTGCAGGGCTACTACCAGGTGCGGCGTCCTAGCCACGAGGGCTA 4650
CCTGGCAGCCCCAGGCCTTGAGGGGCCAGGGCCCGATGGGGACGGGCAGA 4700
TGCAGCTGGTCACCAGTCTCTGAGGGCACCTCATGGACCAGGGGCTGGTG 4750
GCCCAGGCCAGGGAGGGAACCCTGGGCAGGGCTCTGGTGGGAGAGGGAGA 4800
CAGATGGAGGCAGTGGCTGGTGGGCCACTCTCTCCAGGTGCCCCTCAGCC 4850
ATGGGCCCTACAGTCCCCTCAGGGGACTCTAACCTGGGGGCCTGAGGTGC 4900
CAGGGTTCACAGACAGGGTTTCCCACCAGCCACACGCACCAGCTCTATTT 4950
GGGGGAAGTGTAGTGAGGAGCAGCCCAGAGGACCCCAGGGGAGTGAGGAG 5000
GGAGAACTTGGAAGGGTGCAGCCCACTTCCAGACTCTCCCCTCTCCCACC 5050
CTTCTACCCTGTGAAGGGAAATGAGGCTTTAGTTTCCTGGGCAGGGAGG 5100
GGCAGCTTCTGAGGTTGCCAAAGGCCCCCACTGGATGGAACCTGTTAGCT 5150
GCTCCTCTCCGCAGCCAGAAATGCTGCCGGCTGCACCCAGAGGGAGCAGT 5200
GAGGCAGGACAGATGGACAGGTTCCTCCTGCGCTGTAATTCCCTGCTCCC 5250

Figure 24

TGGAGACTGGGAAAAGGCCGCAGGGCAGGGGGACTGGGCGGTGGTGGCTG 5300
GTGGTTTAAAGGTTGAACTTTCTCTGAAGCTCCTTTCCCCTTGCTCTTGG 5350
TCCCTGCCCCGCAAGCAAACCTGCCCCCTCTGCCTCCCAGTGCACCCAAT 5400
GACCCCCTCCCTTGGGGCGACTCCTGATGAAGCACAACTCCCCGCAGGGC 5450
CCCCAGCCCACAGGGGTGGCCATATTTGGGCAGTTCCCAGTCCTGTGGGC 5500
TCGGCTATCTGGGGAGCAGATTTTGGGTCTGGATCTCCCTGGGGAGTGGG 5550
TCCTGGGCTTGGATCTTTCCCTAGGGGGCCCTCTTACTCCTTCCTCTCTC 5600
CTCCTCCTTCCCCATTGCTGTAAATATTTCAACGAAATGGAAAAGAAAAA 5650
AAAAAAGAC 5659

Figure 25

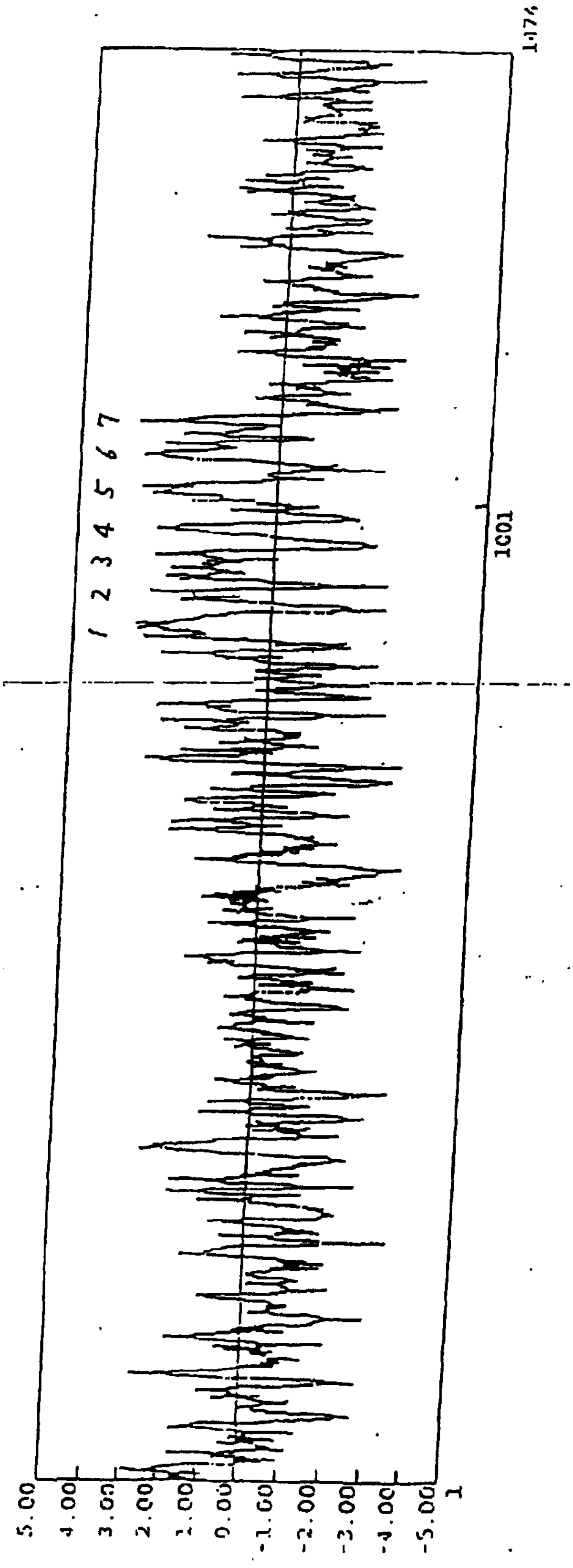

## INTERNATIONAL SEARCH REPORT

International application No.
PCT/JP99/03909

A. CLASSIFICATION OF SUBJECT MATTER
Int.Cl[6] C07K14/705, C12N15/12, C12P21/02, C07K16/28, G01N33/50, A61K38/17, C12Q1/68

According to International Patent Classification (IPC) or to both national classification and IPC

B. FIELDS SEARCHED
Minimum documentation searched (classification system followed by classification symbols)
Int.Cl[6] C07K14/705, C12N15/12, C12P21/02, C07K16/28, G01N33/50, A61K38/17, C12Q1/68

Documentation searched other than minimum documentation to the extent that such documents are included in the fields searched

Electronic data base consulted during the international search (name of data base and, where practicable, search terms used)
GenBank/EMBL/DDBJ/GeneSeq
SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| X<br>A | Genomics Vol.26 No.2 (1995) Baud V.et al."EMR1, an unusual member in the family of hormone receptors with seven transmembrane segments"p.334-344 | 15,16<br>1-14 |
| X | Neuron Vol.18 (1997) Krasnoperov V.G.et al."α-Latroxin stimulates exocytosis by the interaction with a neuronal G-protein-coupled receptor"p.925-937 | 1-16 |
| X | The Journal of Biological Chemistry Vol.272 No.34 (1997) Lelianova V.G.et al. "α-Latroxin receptor, latrophilin, is a novel member of the secretin family of G-protein-coupled receptors" p.21504-21508 | 1-16 |
| P,X | WO,98/39440,A2 (Univ.New York State) 11 September, 1998 (11.09.98) & AU,9866853,A | 1-16 |
| P,X | DNA Research Vol.5 (1998 Oct.) Nagase T.et al." Prediction of the coding sequences of unidentified human genes. XI.The complete sequences of 100 new cDNA clones from brain which code for large proteins in vitro" p.277-286 | 1-16 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 November, 1999 (05.11.99) | 16 November, 1999 (16.11.99) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/03909

C (Continuation). DOCUMENTS CONSIDERED TO BE RELEVANT

| Category* | Citation of document, with indication, where appropriate, of the relevant passages | Relevant to claim No. |
|---|---|---|
| P,X | FEBS Letters Vol.443 (1999 Jan.) Matsushita H.et al."The latrophilin family: multiply spliced G protein-coupled receptors with differential tissue distribution "p.348-352 | 1-16 |
| P,X | The Journal of Biological Chemistry Vol.274 No.9 (1999 Feb.) Ichtchenko K.et al."A novel ubiquitously expressed α-latrotoxin receptor is a member of the CIRL family of G-protein-coupled receptors"p.5491-5498 | 1-16 |

Form PCT/ISA/210 (continuation of second sheet) (July 1992)